# EUROPEAN PATENT APPLICATION

(11) **EP 3 498 839 A1**
(43) Date of publication of application: **19.06.2019**
(21) Application number: 17841449.6
(22) Date of filing: 10.08.2017
(51) Int. Cl.: C12N 15/09, A61K 39/395, A61P 31/00, A61P 35/00, C07K 16/18, C07K 16/28, C07K 16/46, C12N 1/15, C12N 1/19, C12N 1/21, C12N 5/10, C12P 21/08

(54) **ANTI-PD-1 ANTIBODY**

(30) Priority: 15.08.2016 JP 2016159090; 19.05.2017 JP 2017099615
(71) Applicant: National University Corporation Hokkaido University, Sapporo-shi, Hokkaido 060-0808 (JP); Fuso Pharmaceutical Industries, Ltd., Osaka-shi, Osaka 541-0045 (JP)
(72) Inventor: KONNAI, Satoru, Sapporo-shi Hokkaido 060-0808 (JP); OHASHI, Kazuhiko, Sapporo-shi Hokkaido 060-0808 (JP); MURATA, Shiro, Sapporo-shi Hokkaido 060-0808 (JP); OKAGAWA, Tomohiro, Sapporo-shi Hokkaido 060-0808 (JP); NISHIMORI, Asami, Sapporo-shi Hokkaido 060-0808 (JP); MAEKAWA, Naoya, Sapporo-shi Hokkaido 060-0808 (JP); SUZUKI, Yasuhiko, Sapporo-shi Hokkaido 060-0808 (JP); NAKAJIMA, Chie, Sapporo-shi Hokkaido 060-0808 (JP)
(74) Representative: J A Kemp
(86) International application number: PCT/JP2017/029056
(87) International publication number: WO 2018/034226

(57) **Abstract**

The present invention provides an anti-PD-1 antibody capable of repeated administration even to animals other than rat. An anti-PD-1 antibody comprising (a) a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat; and (b) a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat. A pharmaceutical composition comprising the above-described anti-PD-1 antibody as an active ingredient. A method for preparing the anti-PD-1 antibody is also provided.

SEQUENCE LISTING

<110>  NATIONAL UNIVERSITY CORPORATION HOKKAISO UNIVERSITY

<120>  Anti-PD-1 antibodies

<130>  FP-222PCT

<150>  JP P2016-159090
<151>  2016-08-15

<150>  JP P2017-099615
<151>  2017-05-19

<160>  86

<170>  PatentIn version 3.5

<210>  1
<211>  131
<212>  PRT
<213>  Rattus norvegicus

<400>  1

Met Lys Val Pro Gly Arg Leu Leu Val Leu Leu Phe Trp Ile Pro Ala
1               5                   10                  15

Ser Arg Ser Asp Val Val Leu Thr Gln Thr Pro Val Ser Leu Ser Val
            20                  25                  30

Thr Leu Gly Asp Gln Ala Ser Ile Ser Cys Arg Ser Ser Gln Ser Leu
        35                  40                  45

Glu Tyr Ser Asp Gly Tyr Thr Tyr Leu Glu Trp Tyr Leu Gln Lys Pro
    50                  55                  60

Gly Gln Ser Pro Gln Leu Leu Ile Tyr Gly Val Ser Asn Arg Phe Ser
65                  70                  75                  80

Gly Val Pro Asp Arg Phe Ile Gly Ser Gly Ser Gly Thr Asp Phe Thr
                85                  90                  95

Leu Lys Ile Ser Arg Val Glu Pro Glu Asp Leu Gly Val Tyr Tyr Cys
            100                 105                 110

Phe Gln Ala Thr His Asp Pro Asp Thr Phe Gly Ala Gly Thr Lys Leu
        115                 120                 125

Glu Leu Lys
    130

<210>  2
<211>  138

<212>  PRT
<213>  Rattus norvegicus

<400>  2

Met Ala Ile Leu Val Leu Leu Leu Cys Leu Val Thr Ile Pro His Ser
1               5                   10                  15

Val Leu Ser Gln Val Gln Leu Lys Glu Thr Gly Pro Gly Leu Val Gln
            20                  25                  30

Pro Thr Gln Thr Leu Ser Ile Thr Cys Thr Val Ser Gly Phe Ser Leu
        35                  40                  45

Thr Ser Tyr Tyr Ile Gln Trp Val Arg Gln Thr Pro Gly Lys Gly Leu
    50                  55                  60

Glu Trp Met Gly Phe Ile Arg Ser Gly Gly Ser Thr Glu Tyr Asn Ser
65                  70                  75                  80

Glu Phe Lys Ser Arg Leu Ser Ile Asn Arg Asp Thr Ser Lys Asn Gln
                85                  90                  95

Val Phe Leu Lys Met Asn Ser Leu Lys Thr Glu Asp Thr Gly Val Tyr
            100                 105                 110

Tyr Cys Ala Arg Thr Ser Ser Gly Tyr Glu Gly Gly Phe Asp Tyr Trp
        115                 120                 125

Gly Gln Gly Val Met Val Thr Val Ser Ser
    130                 135


<210>  3
<211>  105
<212>  PRT
<213>  Bos taurus

<400>  3

Gln Pro Lys Ser Pro Pro Ser Val Thr Leu Phe Pro Pro Ser Thr Glu
1               5                   10                  15

Glu Leu Asn Gly Asn Lys Ala Thr Leu Val Cys Leu Ile Ser Asp Phe
            20                  25                  30

Tyr Pro Gly Ser Val Thr Val Val Trp Lys Ala Asp Gly Ser Thr Ile
        35                  40                  45

Thr Arg Asn Val Glu Thr Thr Arg Ala Ser Lys Gln Ser Asn Ser Lys
    50                  55                  60

Tyr Ala Ala Ser Ser Tyr Leu Ser Leu Thr Ser Ser Asp Trp Lys Ser
65                  70                  75                  80

Lys Gly Ser Tyr Ser Cys Glu Val Thr His Glu Gly Ser Thr Val Thr
                85                  90                  95

Lys Thr Val Lys Pro Ser Glu Cys Ser
            100                 105

<210>  4
<211>  328
<212>  PRT
<213>  Bos taurus

<400>  4

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe
65                  70                  75                  80

Thr Cys Asn Val Ala His Pro Ala Ser Ser Thr Lys Val Asp Lys Ala
                85                  90                  95

Val Asp Pro Thr Cys Lys Pro Ser Pro Cys Asp Cys Cys Pro Pro Pro
            100                 105                 110

Pro Val Ala Gly Pro Ser Val Phe Ile Phe Pro Pro Lys Pro Lys Asp
        115                 120                 125

Thr Leu Thr Ile Ser Gly Thr Pro Glu Val Thr Cys Val Val Val Asp
    130                 135                 140

Val Gly His Asp Asp Pro Glu Val Lys Phe Ser Trp Phe Val Asp Asp
145                 150                 155                 160

Val Glu Val Asn Thr Ala Thr Thr Lys Pro Arg Glu Glu Gln Phe Asn
                165                 170                 175

Ser Thr Tyr Arg Val Val Ser Ala Leu Arg Ile Gln His Gln Asp Trp
            180                 185                 190

Thr Gly Gly Lys Glu Phe Lys Cys Lys Val His Asn Glu Gly Leu Pro
        195                 200                 205

Ser Ser Ile Val Arg Thr Ile Ser Arg Thr Lys Gly Pro Ala Arg Glu
    210                 215                 220

Pro Gln Val Tyr Val Leu Ala Pro Pro Gln Glu Glu Leu Ser Lys Ser
225                 230                 235                 240

Thr Val Ser Leu Thr Cys Met Val Thr Ser Phe Tyr Pro Asp Tyr Ile
                245                 250                 255

Ala Val Glu Trp Gln Arg Asn Gly Gln Pro Glu Ser Glu Asp Lys Tyr
            260                 265                 270

Gly Thr Thr Pro Pro Gln Leu Asp Ala Asp Ser Ser Tyr Phe Leu Tyr
        275                 280                 285

Ser Lys Leu Arg Val Asp Arg Asn Ser Trp Gln Glu Gly Asp Thr Tyr
    290                 295                 300

Thr Cys Val Val Met His Glu Ala Leu His Asn His Tyr Thr Gln Lys
305                 310                 315                 320

Ser Thr Ser Lys Ser Ala Gly Lys
                325

<210>  5
<211>  393
<212>  DNA
<213>  Rattus norvegicus

<400>  5
atgaaagtgc ctggtaggct gctggtgctg ttgttttgga ttccagcttc caggagtgat      60

gttgtgttga cacaaactcc agtttccctg tctgtcacac ttggagatca agcttctata     120

tcttgcaggt ctagtcagag cctggaatat agtgatggat acacttattt ggaatggtac     180

ctacagaagc caggccagtc tccacagctc ctcatctatg gagtttccaa ccgattttct     240

ggggtcccag acaggttcat tggcagtggg tcagggacag atttcaccct caagatcagc     300

agagtagagc ctgaggactt gggagtttat tactgcttcc aagctacaca tgatccggac     360

acgtttggag ctgggaccaa gctggaactg aaa                                  393

<210>  6

<211>  414
<212>  DNA
<213>  Rattus norvegicus

<400>  6
atggctatcc tggtgctgct tctctgcctg gtgaccattc cacactctgt cttgtcccag      60

gtgcagctga aggagacagg acctggcctg gtgcaaccaa cacagaccct gtccatcaca     120

tgtactgttt ctgggttctc attaaccagc tattatatac agtgggttcg ccagactcca     180

ggaaagggac tagaatggat gggatttata cggagtggtg gaagcacaga gtataattca     240

gagttcaaat cccgacttag catcaacagg gacacctcca agaaccaagt tttcttaaaa     300

atgaacagtc tgaaaacaga ggacacaggc gtgtactact gtgccagaac ctcttcgggg     360

tacgaagggg gttttgatta ctggggccaa ggagtcatgg tcacagtctc ctca           414


<210>  7
<211>  318
<212>  DNA
<213>  Bos taurus

<400>  7
cagcccaagt ccccaccctc ggtcaccctg ttcccgccct ccacggagga gctcaacggc      60

aacaaggcca ccctggtgtg tctcatcagc gacttctacc cgggtagcgt gaccgtggtc     120

tggaaggcag acggcagcac catcacccgc aacgtggaga ccacccgggc ctccaaacag     180

agcaacagca agtacgcggc cagcagctac ctgagcctga cgagcagcga ctggaaatcg     240

aaaggcagtt acagctgcga ggtcacgcac gaggggagca ccgtgacgaa gacagtgaag     300

ccctcagagt gttcttag                                                   318


<210>  8
<211>  987
<212>  DNA
<213>  Artificial Sequence

<220>
<223>  codon-optimized sequence

<400>  8
gctagcacca cagcacctaa agtttaccct ctgtcttcct gctgcggcga caagtcttca      60

tcaactgtta ctcttggatg cctggtctca agttacatgc ccgagcccgt gacagtgacc     120

tggaactcag gcgctctgaa gtctggagtg cacacatttc cagctgtgct tcagtctagc     180

ggcctgtatt ccctcagctc tatggttact gtacctggta gcaccagcgg acagactttc     240

acctgtaatg ttgcccatcc cgcatcttct accaaggtcg ataaagccgt tgaccccact     300

tgcaaaccat ccccttgtga ttgttgtcca ccccctccag tggctggccc ttccgtcttc     360

attttccctc ctaaacctaa ggatactctg accatctcag ggacacccga ggtcacctgt     420

gtcgtcgtgg acgtgggaca tgacgaccca gaagtcaagt tctcatggtt cgtggacgat     480

gtggaggtga acacagcaac aacaaagccc agagaagaac agtttaacag cacatatcgg 540

gtggtcagcg ccttgcgtat tcagcaccag gactggactg gtggcaagga gtttaagtgc 600

aaggtgcata acgaaggtct gccctcttct atagtgagaa ctatctcccg aactaagggc 660

cccgctcggg agccccaggt ttacgtcctt gctccccctc aggaggaact gagtaaatca 720

accgtgagtc tcacctgtat ggttacctca ttttacccag actacatcgc cgtagagtgg 780

cagaggaatg gacagccaga gtctgaggac aaatacggca ctactcctcc ccaactggat 840

gccgactctt cctacttcct ctactccaaa ttgcgagttg accggaactc atggcaggag 900

ggggacacat acacatgcgt cgttatgcac gaggccctgc acaaccatta cacccagaag 960

tccacatcta aaagtgcagg taagtaa 987

<210> 9
<211> 236
<212> PRT
<213> Artificial Sequence

<220>
<223> chimeric L chain

<400> 9

Met Lys Val Pro Gly Arg Leu Leu Val Leu Leu Phe Trp Ile Pro Ala
1               5                   10                  15

Ser Arg Ser Asp Val Val Leu Thr Gln Thr Pro Val Ser Leu Ser Val
            20                  25                  30

Thr Leu Gly Asp Gln Ala Ser Ile Ser Cys Arg Ser Ser Gln Ser Leu
        35                  40                  45

Glu Tyr Ser Asp Gly Tyr Thr Tyr Leu Glu Trp Tyr Leu Gln Lys Pro
    50                  55                  60

Gly Gln Ser Pro Gln Leu Leu Ile Tyr Gly Val Ser Asn Arg Phe Ser
65                  70                  75                  80

Gly Val Pro Asp Arg Phe Ile Gly Ser Gly Ser Gly Thr Asp Phe Thr
                85                  90                  95

Leu Lys Ile Ser Arg Val Glu Pro Glu Asp Leu Gly Val Tyr Tyr Cys
            100                 105                 110

Phe Gln Ala Thr His Asp Pro Asp Thr Phe Gly Ala Gly Thr Lys Leu
        115                 120                 125

Glu Leu Lys Gln Pro Lys Ser Pro Pro Ser Val Thr Leu Phe Pro Pro

130                 135                 140

Ser Thr Glu Glu Leu Asn Gly Asn Lys Ala Thr Leu Val Cys Leu Ile
145                 150                 155                 160

Ser Asp Phe Tyr Pro Gly Ser Val Thr Val Val Trp Lys Ala Asp Gly
                165                 170                 175

Ser Thr Ile Thr Arg Asn Val Glu Thr Thr Arg Ala Ser Lys Gln Ser
            180                 185                 190

Asn Ser Lys Tyr Ala Ala Ser Ser Tyr Leu Ser Leu Thr Ser Ser Asp
        195                 200                 205

Trp Lys Ser Lys Gly Ser Tyr Ser Cys Glu Val Thr His Glu Gly Ser
    210                 215                 220

Thr Val Thr Lys Thr Val Lys Pro Ser Glu Cys Ser
225                 230                 235

<210>  10
<211>  466
<212>  PRT
<213>  Artificial Sequence

<220>
<223>  chimeric H chain

<400>  10

Met Ala Ile Leu Val Leu Leu Leu Cys Leu Val Thr Ile Pro His Ser
1               5                   10                  15

Val Leu Ser Gln Val Gln Leu Lys Glu Thr Gly Pro Gly Leu Val Gln
            20                  25                  30

Pro Thr Gln Thr Leu Ser Ile Thr Cys Thr Val Ser Gly Phe Ser Leu
        35                  40                  45

Thr Ser Tyr Tyr Ile Gln Trp Val Arg Gln Thr Pro Gly Lys Gly Leu
    50                  55                  60

Glu Trp Met Gly Phe Ile Arg Ser Gly Gly Ser Thr Glu Tyr Asn Ser
65                  70                  75                  80

Glu Phe Lys Ser Arg Leu Ser Ile Asn Arg Asp Thr Ser Lys Asn Gln
                85                  90                  95

Val Phe Leu Lys Met Asn Ser Leu Lys Thr Glu Asp Thr Gly Val Tyr
            100                 105                 110

Tyr Cys Ala Arg Thr Ser Ser Gly Tyr Glu Gly Gly Phe Asp Tyr Trp
        115                 120                 125

Gly Gln Gly Val Met Val Thr Val Ser Ser Ala Ser Thr Thr Ala Pro
    130                 135                 140

Lys Val Tyr Pro Leu Ser Ser Cys Cys Gly Asp Lys Ser Ser Ser Thr
145                 150                 155                 160

Val Thr Leu Gly Cys Leu Val Ser Ser Tyr Met Pro Glu Pro Val Thr
                165                 170                 175

Val Thr Trp Asn Ser Gly Ala Leu Lys Ser Gly Val His Thr Phe Pro
            180                 185                 190

Ala Val Leu Gln Ser Ser Gly Leu Tyr Ser Leu Ser Ser Met Val Thr
        195                 200                 205

Val Pro Gly Ser Thr Ser Gly Gln Thr Phe Thr Cys Asn Val Ala His
    210                 215                 220

Pro Ala Ser Ser Thr Lys Val Asp Lys Ala Val Asp Pro Thr Cys Lys
225                 230                 235                 240

Pro Ser Pro Cys Asp Cys Cys Pro Pro Pro Pro Val Ala Gly Pro Ser
                245                 250                 255

Val Phe Ile Phe Pro Pro Lys Pro Lys Asp Thr Leu Thr Ile Ser Gly
            260                 265                 270

Thr Pro Glu Val Thr Cys Val Val Val Asp Val Gly His Asp Asp Pro
        275                 280                 285

Glu Val Lys Phe Ser Trp Phe Val Asp Asp Val Glu Val Asn Thr Ala
    290                 295                 300

Thr Thr Lys Pro Arg Glu Glu Gln Phe Asn Ser Thr Tyr Arg Val Val
305                 310                 315                 320

Ser Ala Leu Arg Ile Gln His Gln Asp Trp Thr Gly Gly Lys Glu Phe
                325                 330                 335

Lys Cys Lys Val His Asn Glu Gly Leu Pro Ser Ser Ile Val Arg Thr
            340                 345                 350

Ile Ser Arg Thr Lys Gly Pro Ala Arg Glu Pro Gln Val Tyr Val Leu

355                 360                 365

Ala Pro Pro Gln Glu Glu Leu Ser Lys Ser Thr Val Ser Leu Thr Cys
    370                 375                 380

Met Val Thr Ser Phe Tyr Pro Asp Tyr Ile Ala Val Glu Trp Gln Arg
385                 390                 395                 400

Asn Gly Gln Pro Glu Ser Glu Asp Lys Tyr Gly Thr Thr Pro Pro Gln
                405                 410                 415

Leu Asp Ala Asp Ser Ser Tyr Phe Leu Tyr Ser Lys Leu Arg Val Asp
            420                 425                 430

Arg Asn Ser Trp Gln Glu Gly Asp Thr Tyr Thr Cys Val Val Met His
        435                 440                 445

Glu Ala Leu His Asn His Tyr Thr Gln Lys Ser Thr Ser Lys Ser Ala
    450                 455                 460

Gly Lys
465

<210>  11
<211>  393
<212>  DNA
<213>  Artificial Sequence

<220>
<223>  codon-optimzed sequence

<400>  11
atgaaggtcc ctggtaggct gctggttctc ttgttctgga tccctgcttc cagaagtgac      60

gtggtgctga ctcaaacacc agtgagtctc agtgtgaccc tcggcgacca ggcctccatt     120

tcttgccgta gcagccagtc cttggaatac tctgatggtt atacttatct ggagtggtac     180

ctccagaagc ccgggcagtc accccagctt cttatctacg gtgtgagcaa cagattttct     240

ggggttcctg atcggtttat tggatctgga tccggtaccg acttcacatt gaaaatttca     300

cgcgtcgaac ccgaggatct gggggtctac tattgcttcc aagccaccca cgatcccgac     360

accttcggcg ctggcactaa gctggagctg aaa                                  393

<210>  12
<211>  414
<212>  DNA
<213>  Artificial Sequence

<220>
<223>  codon-optimized sequence

<400>  12
atggcaatcc tcgtgttgct tctgtgcttg gtgaccattc cacactctgt gctttcccag      60

gtgcagctca aggaaacagg gccaggactc gtccaaccta cacaaaccct gtcaatcacc     120

tgtaccgtat ccggttttag cctcaccagc tattatatac aatgggtgag gcagaccccc     180

gggaaaggac tggaatggat gggcttcatt cgcagcggtg ggagtaccga gtacaatagc     240

gagtttaaaa gtcgcttgag tatcaataga gatacttcca agaatcaggt gttcttgaag     300

atgaactccc tcaagaccga agatacaggg gtctattact gcgccaggac ctccagtgga     360

tatgaaggag gctttgatta ttgggggcag ggcgtcatgg taactgtgag ctca           414


<210>  13
<211>  318
<212>  DNA
<213>  Artificial Sequence

<220>
<223>  codon-optimized sequence

<400>  13
caacccaagt caccaccatc cgtgactctg tttcctccat ctacagagga gctgaacgga      60

aacaaagcta ccttggtgtg tctcatctct gacttttacc ccggatctgt cactgtggta     120

tggaaagcag atggcagcac aataaccagg aatgttgaaa ccacacgagc ctccaagcag     180

tccaatagta agtatgccgc atcttcatat ctgtccctta caagctcaga ctggaaatcc     240

aaaggcagct acagttgcga ggtcacacat gaaggcagca ccgtgacaaa gaccgtaaag     300

ccatctgagt gtagctag                                                   318


<210>  14
<211>  711
<212>  DNA
<213>  Artificial Sequence

<220>
<223>  codon-optimized sequence

<400>  14
atgaaggtcc ctggtaggct gctggttctc ttgttctgga tccctgcttc cagaagtgac      60

gtggtgctga ctcaaacacc agtgagtctc agtgtgaccc tcggcgacca ggcctccatt     120

tcttgccgta gcagccagtc cttggaatac tctgatggtt atacttatct ggagtggtac     180

ctccagaagc ccgggcagtc accccagctt cttatctacg gtgtgagcaa cagattttct     240

ggggttcctg atcggtttat tggatctgga tccggtaccg acttcacatt gaaaatttca     300

cgcgtcgaac ccgaggatct gggggtctac tattgcttcc aagccaccca cgatcccgac     360

accttcggcg ctggcactaa gctggagctg aaacaaccca agtcaccacc atccgtgact     420

ctgtttcctc catctacaga ggagctgaac ggaaacaaag ctaccttggt gtgtctcatc     480

tctgactttt accccggatc tgtcactgtg gtatggaaag cagatggcag cacaataacc 540

aggaatgttg aaaccacacg agcctccaag cagtccaata gtaagtatgc cgcatcttca 600

tatctgtccc ttacaagctc agactggaaa tccaaaggca gctacagttg cgaggtcaca 660

catgaaggca gcaccgtgac aaagaccgta aagccatctg agtgtagcta g 711

<210> 15
<211> 1401
<212> DNA
<213> Artificial Sequence

<220>
<223> codon-optimized sequence

<400> 15
atggcaatcc tcgtgttgct tctgtgcttg gtgaccattc cacactctgt gctttcccag 60

gtgcagctca aggaaacagg gccaggactc gtccaaccta cacaaaccct gtcaatcacc 120

tgtaccgtat ccggttttag cctcaccagc tattatatac aatgggtgag gcagaccccc 180

gggaaaggac tggaatggat gggcttcatt cgcagcggtg ggagtaccga gtacaatagc 240

gagtttaaaa gtcgcttgag tatcaataga gatacttcca agaatcaggt gttcttgaag 300

atgaactccc tcaagaccga agatacaggg gtctattact gcgccaggac ctccagtgga 360

tatgaaggag gctttgatta ttgggggcag ggcgtcatgg taactgtgag ctcagctagc 420

accacagcac ctaaagttta ccctctgtct tcctgctgcg gcgacaagtc ttcatcaact 480

gttactcttg gatgcctggt ctcaagttac atgcccgagc ccgtgacagt gacctggaac 540

tcaggcgctc tgaagtctgg agtgcacaca tttccagctg tgcttcagtc tagcggcctg 600

tattccctca gctctatggt tactgtacct ggtagcacca gcggacagac tttcacctgt 660

aatgttgccc atcccgcatc ttctaccaag gtcgataaag ccgttgaccc cacttgcaaa 720

ccatcccctt gtgattgttg tccaccccct ccagtggctg gcccttccgt cttcattttc 780

cctcctaaac ctaaggatac tctgaccatc tcagggacac ccgaggtcac ctgtgtcgtc 840

gtggacgtgg gacatgacga cccagaagtc aagttctcat ggttcgtgga cgatgtggag 900

gtgaacacag caacaacaaa gcccagagaa gaacagttta acagcacata tcgggtggtc 960

agcgccttgc gtattcagca ccaggactgg actggtggca aggagtttaa gtgcaaggtg 1020

cataacgaag gtctgccctc ttctatagtg agaactatct cccgaactaa gggccccgct 1080

cgggagcccc aggtttacgt ccttgctccc cctcaggagg aactgagtaa atcaaccgtg 1140

agtctcacct gtatggttac ctcattttac ccagactaca tcgccgtaga gtggcagagg 1200

aatggacagc cagagtctga ggacaaatac ggcactactc ctccccaact ggatgccgac 1260

tcttcctact tcctctactc caaattgcga gttgaccgga actcatggca ggagggggac 1320

acatacacat gcgtcgttat gcacgaggcc ctgcacaacc attacaccca gaagtccaca 1380

tctaaaagtg caggtaagta a                                                1401

<210>  16
<211>  11
<212>  PRT
<213>  Rattus norvegicus

<400>  16

Gln Ser Leu Glu Tyr Ser Asp Gly Tyr Thr Tyr
1               5                   10

<210>  17
<211>  9
<212>  PRT
<213>  Rattus norvegicus

<400>  17

Phe Gln Ala Thr His Asp Pro Asp Thr
1               5

<210>  18
<211>  8
<212>  PRT
<213>  Rattus norvegicus

<400>  18

Gly Phe Ser Leu Thr Ser Tyr Tyr
1               5

<210>  19
<211>  7
<212>  PRT
<213>  Rattus norvegicus

<400>  19

Ile Arg Ser Gly Gly Ser Thr
1               5

<210>  20
<211>  13
<212>  PRT
<213>  Rattus norvegicus

<400>  20

Ala Arg Thr Ser Ser Gly Tyr Glu Gly Gly Phe Asp Tyr
1               5                   10

<210>  21
<211>  329
<212>  PRT
<213>  Bos taurus

<400> 21

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe
65                  70                  75                  80

Thr Cys Asn Val Ala His Pro Ala Ser Ser Thr Lys Val Asp Lys Ala
                85                  90                  95

Val Asp Pro Thr Cys Lys Pro Ser Pro Cys Asp Cys Cys Pro Pro Pro
            100                 105                 110

Glu Leu Pro Gly Gly Pro Ser Val Phe Ile Phe Pro Pro Lys Pro Lys
        115                 120                 125

Asp Thr Leu Thr Ile Ser Gly Thr Pro Glu Val Thr Cys Val Val Val
    130                 135                 140

Asp Val Gly His Asp Asp Pro Glu Val Lys Phe Ser Trp Phe Val Asp
145                 150                 155                 160

Asp Val Glu Val Asn Thr Ala Thr Thr Lys Pro Arg Glu Glu Gln Phe
                165                 170                 175

Asn Ser Thr Tyr Arg Val Val Ser Ala Leu Arg Ile Gln His Gln Asp
            180                 185                 190

Trp Thr Gly Gly Lys Glu Phe Lys Cys Lys Val His Asn Glu Gly Leu
        195                 200                 205

Pro Ala Pro Ile Val Arg Thr Ile Ser Arg Thr Lys Gly Pro Ala Arg
    210                 215                 220

Glu Pro Gln Val Tyr Val Leu Ala Pro Pro Gln Glu Glu Leu Ser Lys
225                 230                 235                 240

Ser Thr Val Ser Leu Thr Cys Met Val Thr Ser Phe Tyr Pro Asp Tyr
                245                 250                 255

Ile Ala Val Glu Trp Gln Arg Asn Gly Gln Pro Glu Ser Glu Asp Lys
            260                 265                 270

Tyr Gly Thr Thr Pro Pro Gln Leu Asp Ala Asp Ser Ser Tyr Phe Leu
        275                 280                 285

Tyr Ser Lys Leu Arg Val Asp Arg Asn Ser Trp Gln Glu Gly Asp Thr
    290                 295                 300

Tyr Thr Cys Val Val Met His Glu Ala Leu His Asn His Tyr Thr Gln
305                 310                 315                 320

Lys Ser Thr Ser Lys Ser Ala Gly Lys
                325

<210>  22
<211>  329
<212>  PRT
<213>  Bos taurus

<400>  22

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe
65                  70                  75                  80

Thr Cys Asn Val Ala His Pro Ala Ser Ser Thr Lys Val Asp Lys Ala
                85                  90                  95

Val Asp Pro Thr Cys Lys Pro Ser Pro Cys Asp Cys Cys Pro Pro Pro
            100                 105                 110

Glu Leu Pro Gly Gly Pro Ser Val Phe Ile Phe Pro Pro Lys Pro Lys
        115                 120                 125

Asp Thr Leu Thr Ile Ser Gly Thr Pro Glu Val Thr Cys Val Val Val
    130                 135                 140

Asp Val Gly His Asp Asp Pro Glu Val Lys Phe Ser Trp Phe Val Asp
145                 150                 155                 160

Asp Val Glu Val Asn Thr Ala Thr Thr Lys Pro Arg Glu Glu Gln Phe
                165                 170                 175

Asn Ser Thr Tyr Arg Val Val Ser Ala Leu Arg Ile Gln His Gln Asp
            180                 185                 190

Trp Thr Gly Gly Lys Glu Phe Lys Cys Lys Val His Asn Glu Gly Leu
        195                 200                 205

Pro Ala Pro Ile Val Arg Thr Ile Ser Arg Thr Lys Gly Pro Ala Arg
    210                 215                 220

Glu Pro Gln Val Tyr Val Leu Ala Pro Pro Gln Glu Glu Leu Ser Lys
225                 230                 235                 240

Ser Thr Val Ser Leu Thr Cys Met Val Thr Ser Phe Tyr Pro Asp Tyr
                245                 250                 255

Ile Ala Val Glu Trp Gln Arg Asn Gly Gln Pro Glu Ser Glu Asp Lys
            260                 265                 270

Tyr Gly Thr Thr Pro Pro Gln Leu Asp Ala Asp Ser Ser Tyr Phe Leu
        275                 280                 285

Tyr Ser Lys Leu Arg Val Asp Arg Asn Ser Trp Gln Glu Gly Asp Thr
    290                 295                 300

Tyr Thr Cys Val Val Met His Glu Ala Leu His Asn His Tyr Thr Gln
305                 310                 315                 320

Lys Ser Thr Ser Lys Ser Ala Gly Lys
                325

<210>  23
<211>  329
<212>  PRT
<213>  Bos taurus

<400>  23

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Thr Gln Thr
65                  70                  75                  80

Phe Thr Cys Asn Val Ala His Pro Ala Ser Ser Thr Lys Val Asp Lys
                85                  90                  95

Ala Val Asp Pro Arg Cys Lys Thr Thr Cys Asp Cys Cys Pro Pro Pro
            100                 105                 110

Glu Leu Pro Gly Gly Pro Ser Val Phe Ile Phe Pro Pro Lys Pro Lys
        115                 120                 125

Asp Thr Leu Thr Ile Ser Gly Thr Pro Glu Val Thr Cys Val Val Val
    130                 135                 140

Asp Val Gly His Asp Asp Pro Glu Val Lys Phe Ser Trp Phe Val Asp
145                 150                 155                 160

Asp Val Glu Val Asn Thr Ala Thr Thr Lys Pro Arg Glu Glu Gln Phe
                165                 170                 175

Asn Ser Thr Tyr Arg Val Val Ser Ala Leu Arg Ile Gln His Gln Asp
            180                 185                 190

Trp Thr Gly Gly Lys Glu Phe Lys Cys Lys Val His Asn Glu Gly Leu
        195                 200                 205

Pro Ala Pro Ile Val Arg Thr Ile Ser Arg Thr Lys Gly Pro Ala Arg
    210                 215                 220

Glu Pro Gln Val Tyr Val Leu Ala Pro Pro Gln Glu Glu Leu Ser Lys
225                 230                 235                 240

Ser Thr Val Ser Leu Thr Cys Met Val Thr Ser Phe Tyr Pro Asp Tyr
                245                 250                 255

Ile Ala Val Glu Trp Gln Arg Asn Gly Gln Pro Glu Ser Glu Asp Lys
            260                 265                 270

Tyr Gly Thr Thr Pro Pro Gln Leu Asp Ala Asp Gly Ser Tyr Phe Leu
        275                 280                 285

Tyr Ser Arg Leu Arg Val Asp Arg Asn Ser Trp Gln Glu Gly Asp Thr
    290                 295                 300

Tyr Thr Cys Val Val Met His Glu Ala Leu His Asn His Tyr Thr Gln
305                 310                 315                 320

Lys Ser Thr Ser Lys Ser Ala Gly Lys
                325

<210>  24
<211>  326
<212>  PRT
<213>  Bos taurus

<400>  24

Ala Ser Thr Thr Ala Pro Lys Val Tyr Pro Leu Ala Ser Ser Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Ala Ser Ser Ser Gly Gln Thr Phe
65                  70                  75                  80

Thr Cys Asn Val Ala His Pro Ala Ser Ser Thr Lys Val Asp Lys Ala
                85                  90                  95

Val Gly Val Ser Ile Asp Cys Ser Lys Cys His Asn Gln Pro Cys Val
            100                 105                 110

Arg Glu Pro Ser Val Phe Ile Phe Pro Pro Lys Pro Lys Asp Thr Leu
        115                 120                 125

Met Ile Thr Gly Thr Pro Glu Val Thr Cys Val Val Val Asn Val Gly
    130                 135                 140

His Asp Asn Pro Glu Val Gln Phe Ser Trp Phe Val Asp Asp Val Glu
145                 150                 155                 160

Val His Thr Ala Arg Ser Lys Pro Arg Glu Glu Gln Phe Asn Ser Thr
                165                 170                 175

Tyr Arg Val Val Ser Ala Leu Pro Ile Gln His Gln Asp Trp Thr Gly
            180                 185                 190

Gly Lys Glu Phe Lys Cys Lys Val Asn Asn Lys Gly Leu Ser Ala Pro
        195                 200                 205

Ile Val Arg Ile Ile Ser Arg Ser Lys Gly Pro Ala Arg Glu Pro Gln
    210                 215                 220

Val Tyr Val Leu Asp Pro Pro Lys Glu Glu Leu Ser Lys Ser Thr Leu
225                 230                 235                 240

Ser Val Thr Cys Met Val Thr Gly Phe Tyr Pro Glu Asp Val Ala Val
                245                 250                 255

Glu Trp Gln Arg Asn Arg Gln Thr Glu Ser Glu Asp Lys Tyr Arg Thr
            260                 265                 270

Thr Pro Pro Gln Leu Asp Thr Asp Arg Ser Tyr Phe Leu Tyr Ser Lys
        275                 280                 285

Leu Arg Val Asp Arg Asn Ser Trp Gln Glu Gly Asp Ala Tyr Thr Cys
    290                 295                 300

Val Val Met His Glu Ala Leu His Asn His Tyr Met Gln Lys Ser Thr
305                 310                 315                 320

Ser Lys Ser Ala Gly Lys
                325

<210>  25
<211>  326
<212>  PRT
<213>  Bos taurus

<400>  25

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe
65                  70                  75                  80

Thr Cys Asn Val Ala His Pro Ala Ser Ser Thr Lys Val Asp Lys Ala
                85                  90                  95

Val Gly Val Ser Ser Asp Cys Ser Lys Pro Asn Asn Gln His Cys Val
            100                 105                 110

Arg Glu Pro Ser Val Phe Ile Phe Pro Pro Lys Pro Lys Asp Thr Leu
        115                 120                 125

Met Ile Thr Gly Thr Pro Glu Val Thr Cys Val Val Val Asn Val Gly
    130                 135                 140

His Asp Asn Pro Glu Val Gln Phe Ser Trp Phe Val Asp Asp Val Glu
145                 150                 155                 160

Val His Thr Ala Arg Thr Lys Pro Arg Glu Glu Gln Phe Asn Ser Thr
                165                 170                 175

Tyr Arg Val Val Ser Ala Leu Pro Ile Gln His Gln Asp Trp Thr Gly
            180                 185                 190

Gly Lys Glu Phe Lys Cys Lys Val Asn Ile Lys Gly Leu Ser Ala Ser
        195                 200                 205

Ile Val Arg Ile Ile Ser Arg Ser Lys Gly Pro Ala Arg Glu Pro Gln
    210                 215                 220

Val Tyr Val Leu Asp Pro Pro Lys Glu Glu Leu Ser Lys Ser Thr Val
225                 230                 235                 240

Ser Val Thr Cys Met Val Ile Gly Phe Tyr Pro Glu Asp Val Asp Val
                245                 250                 255

Glu Trp Gln Arg Asp Arg Gln Thr Glu Ser Glu Asp Lys Tyr Arg Thr
            260                 265                 270

Thr Pro Pro Gln Leu Asp Ala Asp Arg Ser Tyr Phe Leu Tyr Ser Lys
        275                 280                 285

Leu Arg Val Asp Arg Asn Ser Trp Gln Arg Gly Asp Thr Tyr Thr Cys
    290                 295                 300

Val Val Met His Glu Ala Leu His Asn His Tyr Met Gln Lys Ser Thr
305                 310                 315                 320

Ser Lys Ser Ala Gly Lys
                325

<210>  26
<211>  327
<212>  PRT
<213>  Bos taurus

<400>  26

Ala Ser Thr Thr Ala Pro Lys Val Tyr Pro Leu Ser Ser Cys Cys Gly
1               5                   10                  15

Asp Lys Ser Ser Ser Gly Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Ala Ser Ser Ser Gly Thr Gln Thr
65                  70                  75                  80

Phe Thr Cys Asn Val Ala His Pro Ala Ser Ser Thr Lys Val Asp Lys
                85                  90                  95

Ala Val Gly Val Ser Ser Asp Cys Ser Lys Pro Asn Asn Gln His Cys
            100                 105                 110

Val Arg Glu Pro Ser Val Phe Ile Phe Pro Pro Lys Pro Lys Asp Thr
        115                 120                 125

Leu Met Ile Thr Gly Thr Pro Glu Val Thr Cys Val Val Val Asn Val
    130                 135                 140

Gly His Asp Asn Pro Glu Val Gln Phe Ser Trp Phe Val Asp Asp Val
145                 150                 155                 160

Glu Val His Thr Ala Arg Thr Lys Pro Arg Glu Glu Gln Phe Asn Ser
                165                 170                 175

Thr Tyr Arg Val Val Ser Ala Leu Pro Ile Gln His Gln Asp Trp Thr
            180                 185                 190

Gly Gly Lys Glu Phe Lys Cys Lys Val Asn Ile Lys Gly Leu Ser Ala
        195                 200                 205

Ser Ile Val Arg Ile Ile Ser Arg Ser Lys Gly Pro Ala Arg Glu Pro
    210                 215                 220

Gln Val Tyr Val Leu Asp Pro Pro Lys Glu Glu Leu Ser Lys Ser Thr
225                 230                 235                 240

Val Ser Leu Thr Cys Met Val Ile Gly Phe Tyr Pro Glu Asp Val Asp
                245                 250                 255

Val Glu Trp Gln Arg Asp Arg Gln Thr Glu Ser Glu Asp Lys Tyr Arg
            260                 265                 270

Thr Thr Pro Pro Gln Leu Asp Ala Asp Arg Ser Tyr Phe Leu Tyr Ser
        275                 280                 285

Lys Leu Arg Val Asp Arg Asn Ser Trp Gln Arg Gly Asp Thr Tyr Thr
    290                 295                 300

Cys Val Val Met His Glu Ala Leu His Asn His Tyr Met Gln Lys Ser
305                 310                 315                 320

Thr Ser Lys Ser Ala Gly Lys
                325

<210>  27
<211>  352
<212>  PRT
<213>  Bos taurus

<400>  27

Ala Ser Thr Thr Ala Pro Lys Val Tyr Pro Leu Ala Ser Ser Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Arg Gln Ser Ser Gly Leu Tyr Ser
    50                  55                  60

Leu Ser Ser Met Val Thr Val Pro Ala Ser Ser Ser Glu Thr Gln Thr
65                  70                  75                  80

Phe Thr Cys Asn Val Ala His Pro Ala Ser Ser Thr Lys Val Asp Lys
                85                  90                  95

Ala Val Thr Ala Arg Arg Pro Val Pro Thr Thr Pro Lys Thr Thr Ile
            100                 105                 110

Pro Pro Gly Lys Pro Thr Thr Pro Lys Ser Glu Val Glu Lys Thr Pro
        115                 120                 125

Cys Gln Cys Ser Lys Cys Pro Glu Pro Leu Gly Gly Leu Ser Val Phe
    130                 135                 140

Ile Phe Pro Pro Lys Pro Lys Asp Thr Leu Thr Ile Ser Gly Thr Pro
145                 150                 155                 160

Glu Val Thr Cys Val Val Val Asp Val Gly Gln Asp Asp Pro Glu Val
                165                 170                 175

Gln Phe Ser Trp Phe Val Asp Asp Val Glu Val His Thr Ala Arg Thr
            180                 185                 190

Lys Pro Arg Glu Glu Gln Phe Asn Ser Thr Tyr Arg Val Val Ser Ala
        195                 200                 205

Leu Arg Ile Gln His Gln Asp Trp Leu Gln Gly Lys Glu Phe Lys Cys
    210                 215                 220

Lys Val Asn Asn Lys Gly Leu Pro Ala Pro Ile Val Arg Thr Ile Ser
225                 230                 235                 240

Arg Thr Lys Gly Gln Ala Arg Glu Pro Gln Val Tyr Val Leu Ala Pro
                245                 250                 255

Pro Arg Glu Glu Leu Ser Lys Ser Thr Leu Ser Leu Thr Cys Leu Ile
            260                 265                 270

Thr Gly Phe Tyr Pro Glu Glu Ile Asp Val Glu Trp Gln Arg Asn Gly
        275                 280                 285

Gln Pro Glu Ser Glu Asp Lys Tyr His Thr Thr Ala Pro Gln Leu Asp
    290                 295                 300

Ala Asp Gly Ser Tyr Phe Leu Tyr Ser Lys Leu Arg Val Asn Lys Ser
305                 310                 315                 320

Ser Trp Gln Glu Gly Asp His Tyr Thr Cys Ala Val Met His Glu Ala

                325                 330                 335

Leu Arg Asn His Tyr Lys Glu Lys Ser Ile Ser Arg Ser Pro Gly Lys
            340                 345                 350

<210> 28
<211> 352
<212> PRT
<213> Bos taurus

<400> 28

Ala Ser Thr Thr Ala Pro Lys Val Tyr Pro Leu Ala Ser Arg Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Ala Ser Thr Ser Glu Thr Gln Thr
65                  70                  75                  80

Phe Thr Cys Asn Val Ala His Pro Ala Ser Ser Thr Lys Val Asp Lys
                85                  90                  95

Ala Val Thr Ala Arg Arg Pro Val Pro Thr Thr Pro Lys Thr Thr Ile
            100                 105                 110

Pro Pro Gly Lys Pro Thr Thr Gln Glu Ser Glu Val Glu Lys Thr Pro
        115                 120                 125

Cys Gln Cys Ser Lys Cys Pro Glu Pro Leu Gly Gly Leu Ser Val Phe
    130                 135                 140

Ile Phe Pro Pro Lys Pro Lys Asp Thr Leu Thr Ile Ser Gly Thr Pro
145                 150                 155                 160

Glu Val Thr Cys Val Val Val Asp Val Gly Gln Asp Asp Pro Glu Val
                165                 170                 175

Gln Phe Ser Trp Phe Val Asp Asp Val Glu Val His Thr Ala Arg Thr
            180                 185                 190

Lys Pro Arg Glu Glu Gln Phe Asn Ser Thr Tyr Arg Val Val Ser Ala

195                 200                 205

Leu Arg Ile Gln His Gln Asp Trp Leu Gln Gly Lys Glu Phe Lys Cys
    210                 215                 220

Lys Val Asn Asn Lys Gly Leu Pro Ala Pro Ile Val Arg Thr Ile Ser
225                 230                 235                 240

Arg Thr Lys Gly Gln Ala Arg Glu Pro Gln Val Tyr Val Leu Ala Pro
                245                 250                 255

Pro Arg Glu Glu Leu Ser Lys Ser Thr Leu Ser Leu Thr Cys Leu Ile
            260                 265                 270

Thr Gly Phe Tyr Pro Glu Glu Ile Asp Val Glu Trp Gln Arg Asn Gly
        275                 280                 285

Gln Pro Glu Ser Glu Asp Lys Tyr His Thr Thr Ala Pro Gln Leu Asp
    290                 295                 300

Ala Asp Gly Ser Tyr Phe Leu Tyr Ser Arg Leu Arg Val Asn Lys Ser
305                 310                 315                 320

Ser Trp Gln Glu Gly Asp His Tyr Thr Cys Ala Val Met His Glu Ala
                325                 330                 335

Leu Arg Asn His Tyr Lys Glu Lys Ser Ile Ser Arg Ser Pro Gly Lys
            340                 345                 350

<210>  29
<211>  990
<212>  DNA
<213>  Bos taurus

<400>  29
gcctccacca cagccccgaa agtctaccct ctgagttctt gctgcgggga caagtccagc      60

tccaccgtga ccctgggctg cctggtctcc agctacatgc ccgagccggt gaccgtgacc     120

tggaactcgg gtgccctgaa gagcggcgtg cacaccttcc cggctgtcct tcagtcctcc     180

gggctgtact ctctcagcag catggtgacc gtgcccggca gcacctcagg acagaccttc     240

acctgcaacg tagcccaccc ggccagcagc accaaggtgg acaaggctgt tgatcccaca     300

tgcaaaccat caccctgtga ctgttgccca ccccctgagc tccccggagg accctctgtc     360

ttcatcttcc caccgaaacc caaggacacc ctcacaatct cgggaacgcc cgaggtcacg     420

tgtgtggtgg tggacgtggg ccacgatgac cccgaggtga agttctcctg gttcgtggac     480

gacgtggagg taaacacagc cacgacgaag ccgagagagg agcagttcaa cagcacctac     540

cgcgtggtca gcgccctgcg catccagcac caggactgga ctggaggaaa ggagttcaag 600

tgcaaggtcc acaacgaagg cctcccggcc cccatcgtga ggaccatctc caggaccaaa 660

gggccggccc gggagccgca ggtgtatgtc ctggccccac cccaggaaga gctcagcaaa 720

agcacggtca gcctcacctg catggtcacc agcttctacc cagactacat cgccgtggag 780

tggcagagaa acgggcagcc tgagtcggag gacaagtacg gcacgacccc gccccagctg 840

gacgccgaca gctcctactt cctgtacagc aagctcaggg tggacaggaa cagctggcag 900

gaaggagaca cctacacgtg tgtggtgatg cacgaggccc tgcacaatca ctacacgcag 960

aagtccacct ctaagtctgc gggtaaatga 990

<210> 30
<211> 990
<212> DNA
<213> Bos taurus

<400> 30
gcctccacca cagccccgaa agtctaccct ctgagttctt gctgcgggga caagtccagc 60

tccaccgtga ccctgggctg cctggtctcc agctacatgc ccgagccggt gaccgtgacc 120

tggaactcgg gtgccctgaa gagcggcgtg cacaccttcc cggccgtcct tcagtcctcc 180

gggctgtact ctctcagcag catggtgacc gtgcccggca gcacctcagg acagaccttc 240

acctgcaacg tagcccaccc ggccagcagc accaaggtgg acaaggctgt tgatcccaca 300

tgcaaaccat caccctgtga ctgttgccca ccccctgagc tccccggagg accctctgtc 360

ttcatcttcc caccgaaacc caaggacacc ctcacaatct cgggaacgcc cgaggtcacg 420

tgtgtggtgg tggacgtggg ccacgatgac cccgaggtga agttctcctg gttcgtggac 480

gacgtggagg taaacacagc cacgacgaag ccgagagagg agcagttcaa cagcacctac 540

cgcgtggtca gcgccctgcg catccagcac caggactgga ctggaggaaa ggagttcaag 600

tgcaaggtcc acaacgaagg cctcccggcc cccatcgtga ggaccatctc caggaccaaa 660

gggccggccc gggagccgca ggtgtatgtc ctggccccac cccaggaaga gctcagcaaa 720

agcacggtca gcctcacctg catggtcacc agcttctacc cagactacat cgccgtggag 780

tggcagagaa acgggcagcc tgagtcggag gacaagtacg gcacgacccc gccccagctg 840

gacgccgaca gctcctactt cctgtacagc aagctcaggg tggacaggaa cagctggcag 900

gaaggagaca cctacacgtg tgtggtgatg cacgaggccc tgcacaatca ctacacgcag 960

aagtccacct ctaagtctgc gggtaaatga 990

<210> 31
<211> 990
<212> DNA
<213> Bos taurus

<400>  31
gcctccacca cagccccgaa agtctaccct ctgagttctt gctgcgggga caagtccagc      60

tccaccgtga ccctgggctg cctggtctcc agctacatgc ccgagccggt gaccgtgacc     120

tggaactcgg gtgccctgaa gagcggcgtg cacaccttcc cggccgtcct tcagtcctcc     180

gggctctact ctctcagcag catggtgacc gtgcccggca gcacctcagg aacccagacc     240

ttcacctgca acgtagccca cccggccagc agcaccaagg tggacaaggc tgttgatccc     300

agatgcaaaa caacctgtga ctgttgccca ccgcctgagc tccctggagg accctctgtc     360

ttcatcttcc caccgaaacc caaggacacc ctcacaatct cgggaacgcc cgaggtcacg     420

tgtgtggtgg tggacgtggg ccacgatgac cccgaggtga agttctcctg gttcgtggac     480

gacgtggagg taaacacagc cacgacgaag ccgagagagg agcagttcaa cagcacctac     540

cgcgtggtca gcgccctgcg catccagcac caggactgga ctggaggaaa ggagttcaag     600

tgcaaggtcc acaacgaagg cctcccagcc cccatcgtga ggaccatctc caggaccaaa     660

gggccggccc gggagccgca ggtgtatgtc ctggccccac cccaggaaga gctcagcaaa     720

agcacggtca gcctcacctg catggtcacc agcttctacc cagactacat cgccgtggag     780

tggcagagaa atgggcagcc tgagtcagag gacaagtacg gcacgacccc tccccagctg     840

gacgccgacg gctcctactt cctgtacagc aggctcaggg tggacaggaa cagctggcag     900

gaaggagaca cctacacgtg tgtggtgatg cacgaggccc tgcacaatca ctacacgcag     960

aagtccacct ctaagtctgc gggtaaatga                                      990

<210>  32
<211>  981
<212>  DNA
<213>  Bos taurus

<400>  32
gcctccacca cagccccgaa agtctaccct ctggcatcca gctgcggaga cacatccagc      60

tccaccgtga ccctgggctg cctggtgtcc agctacatgc ccgagccggt gaccgtgacc     120

tggaactcgg gtgccctgaa gagcggcgtg cacaccttcc cggctgtcct tcagtcctcc     180

gggctctact ctctcagcag catggtgacc gtgcccgcca gcagctcagg acagaccttc     240

acctgcaacg tagcccaccc ggccagcagc accaaggtgg acaaggctgt tggggtctcc     300

attgactgct ccaagtgtca taaccagcct tgcgtgaggg aaccatctgt cttcatcttc     360

ccaccgaaac ccaaagacac cctgatgatc acaggaacgc ccgaggtcac gtgtgtggtg     420

gtgaacgtgg gccacgataa ccccgaggtg cagttctcct ggttcgtgga tgacgtggag     480

gtgcacacgg ccaggtcgaa gccaagagag gagcagttca acagcacgta ccgcgtggtc     540

agcgccctgc ccatccagca ccaggactgg actggaggaa aggagttcaa gtgcaaggtc     600

aacaacaaag gcctctcggc ccccatcgtg aggatcatct ccaggagcaa agggccggcc     660

cgggagccgc aggtgtatgt cctggaccca cccaaggaag agctcagcaa aagcacgctc 720

agcgtcacct gcatggtcac cggcttctac ccagaagatg tagccgtgga gtggcagaga 780

aaccggcaga ctgagtcgga ggacaagtac cgcacgaccc cgccccagct ggacaccgac 840

cgctcctact tcctgtacag caagctcagg gtggacagga acagctggca ggaaggagac 900

gcctacacgt gtgtggtgat gcacgaggcc ctgcacaatc actacatgca gaagtccacc 960

tctaagtctg cgggtaaatg a 981

<210> 33
<211> 981
<212> DNA
<213> Bos taurus

<400> 33
gcctccacca cagccccgaa agtctaccct ctgagttctt gctgcgggga caagtccagc 60

tccaccgtga ccctgggctg cctggtgtcc agctacatgc ccgagccggt gaccgtgacc 120

tggaactcgg gtgccctgaa gagcggcgtg cacaccttcc cggccgtcct tcagtcctcc 180

gggctctact ctctcagcag catggtgacc gtgcccggca gcacctcagg acagaccttc 240

acctgcaacg tagcccaccc ggccagcagc accaaggtgg acaaggctgt tggggtctcc 300

agtgactgct ccaagcctaa taaccagcat tgcgtgaggg aaccatctgt cttcatcttc 360

ccaccgaaac ccaaagacac cctgatgatc acaggaacgc ccgaggtcac gtgtgtggtg 420

gtgaacgtgg gccacgataa ccccgaggtg cagttctcct ggttcgtgga cgacgtggag 480

gtgcacacgg ccaggacgaa gccgagagag gagcagttca acagcacgta ccgcgtggtc 540

agcgccctgc ccatccagca ccaggactgg actggaggaa aggagttcaa gtgcaaggtc 600

aacatcaaag gcctctcggc ctccatcgtg aggatcatct ccaggagcaa agggccggcc 660

cgggagccgc aggtgtatgt cctggaccca cccaaggaag agctcagcaa aagcacggtc 720

agcgtcacct gcatggtcat cggcttctac ccagaagatg tagacgtgga gtggcagaga 780

gaccggcaga ctgagtcgga ggacaagtac cgcacgaccc cgccccagct ggacgccgac 840

cgctcctact tcctgtacag caagctcagg gtggacagga acagctggca gagaggagac 900

acctacacgt gtgtggtgat gcacgaggcc ctgcacaatc actacatgca gaagtccacc 960

tctaagtctg cgggtaaatg a 981

<210> 34
<211> 984
<212> DNA
<213> Bos taurus

<400> 34
gcctccacca cagccccgaa agtctaccct ctgagttctt gctgcgggga caagtccagc 60

tcgggggtga ccctgggctg cctggtctcc agctacatgc ccgagccggt gaccgtgacc 120

tggaactcgg gtgccctgaa gagcggcgtg cacaccttcc cggccgtcct tcagtcctcc 180

gggctctact ctctcagcag catggtgacc gtgcccgcca gcagctcagg aacccagacc 240

ttcacctgca acgtagccca cccggccagc agcaccaagg tggacaaggc tgttggggtc 300

tccagtgact gctccaagcc taataaccag cattgcgtga gggaaccatc tgtcttcatc 360

ttcccaccga aacccaaaga caccctgatg atcacaggaa cgcccgaggt cacgtgtgtg 420

gtggtgaacg tgggccacga taaccccgag gtgcagttct cctggttcgt ggacgacgtg 480

gaggtgcaca cggccaggac gaagccgaga gaggagcagt tcaacagcac gtaccgcgtg 540

gtcagcgccc tgcccatcca gcaccaggac tggactggag gaaaggagtt caagtgcaag 600

gtcaacatca aaggcctctc ggcctccatc gtgaggatca tctccaggag caaagggccg 660

gcccgggagc cgcaggtgta tgtcctggac ccacccaagg aagagctcag caaaagcacg 720

gtcagcctca cctgcatggt catcggcttc tacccagaag atgtagacgt ggagtggcag 780

agagaccggc agactgagtc ggaggacaag taccgcacga ccccgcccca gctggacgcc 840

gaccgctcct acttcctgta cagcaagctc agggtggaca ggaacagctg gcagagagga 900

gacacctaca cgtgtgtggt gatgcacgag gccctgcaca atcactacat gcagaagtcc 960

acctctaagt ctgcgggtaa atga 984

<210> 35
<211> 1059
<212> DNA
<213> Bos taurus

<400> 35
gcctccacca cagccccgaa agtctaccct ctggcatcca gctgcggaga cacatccagc 60

tccaccgtga ccctgggctg cctggtctcc agctacatgc ccgagccggt gaccgtgacc 120

tggaactcgg gtgccctgaa gagcggcgtg cacaccttcc cggccgtccg gcagtcctct 180

gggctgtact ctctcagcag catggtgact gtgcccgcca gcagctcaga aacccagacc 240

ttcacctgca acgtagccca cccggccagc agcaccaagg tggacaaggc tgtcactgca 300

aggcgtccag tcccgacgac gccaaagaca actatccctc ctggaaaacc cacaacccca 360

aagtctgaag ttgaaaagac accctgccag tgttccaaat gcccagaacc tctgggagga 420

ctgtctgtct tcatcttccc accgaaaccc aaggacaccc tcacaatctc gggaacgccc 480

gaggtcacgt gtgtggtggt ggacgtgggc caggatgacc ccgaggtgca gttctcctgg 540

ttcgtggacg acgtggaggt gcacacggcc aggacgaagc cgagagagga gcagttcaac 600

agcacctacc gcgtggtcag cgccctgcgc atccagcacc aggactggct gcagggaaag 660

gagttcaagt gcaaggtcaa caacaaaggc ctcccggccc ccattgtgag gaccatctcc 720

aggaccaaag ggcaggcccg ggagccgcag gtgtatgtcc tggccccacc ccgggaagag 780

ctcagcaaaa gcacgctcag cctcacctgc ctgatcaccg gtttctaccc agaagagata 840

gacgtggagt ggcagagaaa tgggcagcct gagtcggagg acaagtacca cacgaccgca 900

ccccagctgg atgctgacgg ctcctacttc ctgtacagca agctcagggt gaacaagagc 960

agctggcagg aaggagacca ctacacgtgt gcagtgatgc acgaagcttt acggaatcac 1020

tacaaagaga agtccatctc gaggtctccg ggtaaatga 1059

<210> 36
<211> 1059
<212> DNA
<213> Bos taurus

<400> 36
gcctccacca cagccccgaa agtctaccct ctggcatccc gctgcggaga cacatccagc 60

tccaccgtga ccctgggctg cctggtctcc agctacatgc ccgagccggt gaccgtgacc 120

tggaactcgg gtgccctgaa gagtggcgtg cacaccttcc cggccgtcct tcagtcctcc 180

gggctgtact ctctcagcag catggtgacc gtgcccgcca gcacctcaga aacccagacc 240

ttcacctgca acgtagccca cccggccagc agcaccaagg tggacaaggc tgtcactgca 300

aggcgtccag tcccgacgac gccaaagaca accatccctc ctggaaaacc cacaacccag 360

gagtctgaag ttgaaaagac accctgccag tgttccaaat gcccagaacc tctgggagga 420

ctgtctgtct tcatcttccc accgaaaccc aaggacaccc tcacaatctc gggaacgccc 480

gaggtcacgt gtgtggtggt ggacgtgggc caggatgacc ccgaggtgca gttctcctgg 540

ttcgtggacg acgtggaggt gcacacggcc aggacgaagc cgagagagga gcagttcaac 600

agcacctacc gcgtggtcag cgccctgcgc atccagcacc aggactggct gcagggaaag 660

gagttcaagt gcaaggtcaa caacaaaggc ctcccggccc ccattgtgag gaccatctcc 720

aggaccaaag ggcaggcccg ggagccgcag gtgtatgtcc tggccccacc ccgggaagag 780

ctcagcaaaa gcacgctcag cctcacctgc ctgatcaccg gtttctaccc agaagagata 840

gacgtggagt ggcagagaaa tgggcagcct gagtcggagg acaagtacca cacgaccgca 900

ccccagctgg atgctgacgg ctcctacttc ctgtacagca ggctcagggt gaacaagagc 960

agctggcagg aaggagacca ctacacgtgt gcagtgatgc atgaagcttt acggaatcac 1020

tacaaagaga agtccatctc gaggtctccg ggtaaatga 1059

<210> 37
<211> 331
<212> PRT
<213> Ovis aries

<400> 37

Ala Ser Thr Thr Pro Pro Lys Val Tyr Pro Leu Thr Ser Cys Cys Gly
1               5                   10                  15

Asp Thr Ser Ser Ser Ile Val Thr Leu Gly Cys Leu Val Ser Ser Tyr
            20                  25                  30

Met Pro Glu Pro Val Thr Val Thr Trp Asn Ser Gly Ala Leu Thr Ser
        35                  40                  45

Gly Val His Thr Phe Pro Ala Ile Leu Gln Ser Ser Gly Leu Tyr Ser
    50                  55                  60

Leu Ser Ser Val Val Thr Val Pro Ala Ser Thr Ser Gly Ala Gln Thr
65                  70                  75                  80

Phe Ile Cys Asn Val Ala His Pro Ala Ser Ser Thr Lys Val Asp Lys
                85                  90                  95

Arg Val Glu Pro Gly Cys Pro Asp Pro Cys Lys His Cys Arg Cys Pro
            100                 105                 110

Pro Pro Glu Leu Pro Gly Gly Pro Ser Val Phe Ile Phe Pro Pro Lys
        115                 120                 125

Pro Lys Asp Thr Leu Thr Ile Ser Gly Thr Pro Glu Val Thr Cys Val
    130                 135                 140

Val Val Asp Val Gly Gln Asp Asp Pro Glu Val Gln Phe Ser Trp Phe
145                 150                 155                 160

Val Asp Asn Val Glu Val Arg Thr Ala Arg Thr Lys Pro Arg Glu Glu
                165                 170                 175

Gln Phe Asn Ser Thr Phe Arg Val Val Ser Ala Leu Pro Ile Gln His
            180                 185                 190

Gln Asp Trp Thr Gly Gly Lys Glu Phe Lys Cys Lys Val His Asn Glu
        195                 200                 205

Ala Leu Pro Ala Pro Ile Val Arg Thr Ile Ser Arg Thr Lys Gly Gln
    210                 215                 220

Ala Arg Glu Pro Gln Val Tyr Val Leu Ala Pro Pro Gln Glu Glu Leu
225                 230                 235                 240

Ser Lys Ser Thr Leu Ser Val Thr Cys Leu Val Thr Gly Phe Tyr Pro
                245                 250                 255

Asp Tyr Ile Ala Val Glu Trp Gln Lys Asn Gly Gln Pro Glu Ser Glu
            260                 265                 270

Asp Lys Tyr Gly Thr Thr Thr Ser Gln Leu Asp Ala Asp Gly Ser Tyr
        275                 280                 285

Phe Leu Tyr Ser Arg Leu Arg Val Asp Lys Asn Ser Trp Gln Glu Gly
    290                 295                 300

Asp Thr Tyr Ala Cys Val Val Met His Glu Ala Leu His Asn His Tyr
305                 310                 315                 320

Thr Gln Lys Ser Ile Ser Lys Pro Pro Gly Lys
                325                 330

<210>  38
<211>  996
<212>  DNA
<213>  Ovis aries

<400>  38
gcctcaacaa cacccccgaa agtctaccct ctgacttctt gctgcgggga cacgtccagc      60

tccatcgtga ccctgggctg cctggtctcc agctatatgc ccgagccggt gaccgtgacc     120

tggaactctg gtgccctgac cagcggcgtg cacaccttcc cggccatcct gcagtcctcc     180

gggctctact ctctcagcag cgtggtgacc gtgccggcca gcacctcagg agcccagacc     240

ttcatctgca acgtagccca cccggccagc agcaccaagg tggacaagcg tgttgagccc     300

ggatgcccgg acccatgcaa acattgccga tgcccacccc ctgagctccc cggaggaccg     360

tctgtcttca tcttcccacc gaaacccaag gacaccctta caatctctgg aacgcccgag     420

gtcacgtgtg tggtggtgga cgtgggccag gatgaccccg aggtgcagtt ctcctggttc     480

gtggacaacg tggaggtgcg cacggccagg acaaagccga gagaggagca gttcaacagc     540

accttccgcg tggtcagcgc cctgcccatc cagcaccaag actggactgg aggaaaggag     600

ttcaagtgca aggtccacaa cgaagccctc ccggccccca tcgtgaggac catctccagg     660

accaaagggc aggcccggga gccgcaggtg tacgtcctgg ccccacccca ggaagagctc     720

agcaaaagca cgctcagcgt cacctgcctg gtcaccggct tctacccaga ctacatcgcc     780

gtggagtggc agaaaaatgg gcagcctgag tcggaggaca agtacggcac gaccacatcc     840

cagctggacg ccgacggctc ctacttcctg tacagcaggc tcagggtgga caagaacagc     900

tggcaagaag gagacaccta cgcgtgtgtg gtgatgcacg aggctctgca caaccactac     960

acacagaagt cgatctctaa gcctccgggt aaatga                               996

<210>  39

<211>  329
<212>  PRT
<213>  Ovis aries

<400>  39

Ala Ser Thr Thr Ala Pro Lys Val Tyr Pro Leu Thr Ser Cys Cys Gly
1               5                   10                  15

Asp Thr Ser Ser Ser Ser Ser Ile Val Thr Leu Gly Cys Leu Val Ser
            20                  25                  30

Ser Tyr Met Pro Glu Pro Val Thr Val Thr Trp Asn Ser Gly Ala Leu
        35                  40                  45

Thr Ser Gly Val His Thr Phe Pro Ala Ile Leu Gln Ser Ser Gly Leu
    50                  55                  60

Tyr Ser Leu Ser Ser Val Val Thr Val Pro Ala Ser Thr Ser Gly Ala
65                  70                  75                  80

Gln Thr Phe Ile Cys Asn Val Ala His Pro Ala Ser Ser Ala Lys Val
                85                  90                  95

Asp Lys Arg Val Gly Ile Ser Ser Asp Tyr Ser Lys Cys Ser Lys Pro
            100                 105                 110

Pro Cys Val Ser Arg Pro Ser Val Phe Ile Phe Pro Pro Lys Pro Lys
        115                 120                 125

Asp Ser Leu Met Ile Thr Gly Thr Pro Glu Val Thr Cys Val Val Val
    130                 135                 140

Asp Val Gly Gln Gly Asp Pro Glu Val Gln Phe Ser Trp Phe Val Asp
145                 150                 155                 160

Asn Val Glu Val Arg Thr Ala Arg Thr Lys Pro Arg Glu Glu Gln Phe
                165                 170                 175

Asn Ser Thr Phe Arg Val Val Ser Ala Leu Pro Ile Gln His Asp His
            180                 185                 190

Trp Thr Gly Gly Lys Glu Phe Lys Cys Lys Val His Ser Lys Gly Leu
        195                 200                 205

Pro Ala Pro Ile Val Arg Thr Ile Ser Arg Ala Lys Gly Gln Ala Arg
    210                 215                 220

Glu Pro Gln Val Tyr Val Leu Ala Pro Pro Gln Glu Glu Leu Ser Lys

225                 230                 235                 240

Ser Thr Leu Ser Val Thr Cys Leu Val Thr Gly Phe Tyr Pro Asp Tyr
                245                 250                 255

Ile Ala Val Glu Trp Gln Arg Ala Arg Gln Pro Glu Ser Glu Asp Lys
            260                 265                 270

Tyr Gly Thr Thr Thr Ser Gln Leu Asp Ala Asp Gly Ser Tyr Phe Leu
        275                 280                 285

Tyr Ser Arg Leu Arg Val Asp Lys Ser Ser Trp Gln Arg Gly Asp Thr
    290                 295                 300

Tyr Ala Cys Val Val Met His Glu Ala Leu His Asn His Tyr Thr Gln
305                 310                 315                 320

Lys Ser Ile Ser Lys Pro Pro Gly Lys
                325

<210>  40
<211>  990
<212>  DNA
<213>  Ovis aries

<400>  40
gcctccacca cagccccgaa agtctaccct ctgacttctt gctgcgggga cacgtccagc      60

tccagctcca tcgtgaccct gggctgcctg gtctccagct atatgcccga gccggtgacc     120

gtgacctgga actctggtgc cctgaccagc ggcgtgcaca ccttcccggc catcctgcag     180

tcctccgggc tctactctct cagcagcgtg gtgaccgtgc cggccagcac ctcaggagcc     240

cagaccttca tctgcaacgt agcccacccg gccagcagcg ccaaggtgga caagcgtgtt     300

gggatctcca gtgactactc caagtgttct aaaccgcctt gcgtgagccg accgtctgtc     360

ttcatcttcc ccccgaaacc caaggacagc ctcatgatca caggaacgcc cgaggtcacg     420

tgtgtggtgg tggacgtggg ccagggtgac cccgaggtgc agttctcctg gttcgtggac     480

aacgtggagg tgcgcacggc caggacaaag ccgagagagg agcagttcaa cagcaccttc     540

cgcgtggtca gcgccctgcc catccagcac gaccactgga ctggaggaaa ggagttcaag     600

tgcaaggtcc acagcaaagg cctcccggcc cccatcgtga ggaccatctc cagggccaaa     660

gggcaggccc gggagccgca ggtgtacgtc ctggccccac cccaggaaga gctcagcaaa     720

agcacgctca gcgtcacctg cctggtcacc ggcttctacc cagactacat cgccgtggag     780

tggcagagag cgcggcagcc tgagtcggag gacaagtacg gcacgaccac atcccagctg     840

gacgccgacg gctcctactt cctgtacagc aggctcaggg tggacaagag cagctggcaa     900

agaggagaca cctacgcgtg tgtggtgatg cacgaggctc tgcacaacca ctacacacag 960

aagtcgatct ctaagcctcc gggtaaatga 990

<210> 41
<211> 102
<212> PRT
<213> Ovis aries

<400> 41

Pro Ser Val Phe Leu Phe Lys Pro Ser Glu Glu Gln Leu Arg Thr Gly
1               5                   10                  15

Thr Val Ser Val Val Cys Leu Val Asn Asp Phe Tyr Pro Lys Asp Ile
            20                  25                  30

Asn Val Lys Val Lys Val Asp Gly Val Thr Gln Asn Ser Asn Phe Gln
        35                  40                  45

Asn Ser Phe Thr Asp Gln Asp Ser Lys Lys Ser Thr Tyr Ser Leu Ser
    50                  55                  60

Ser Thr Leu Thr Leu Ser Ser Ser Glu Tyr Gln Ser His Asn Ala Tyr
65                  70                  75                  80

Ala Cys Glu Val Ser His Lys Ser Leu Pro Thr Ala Leu Val Lys Ser
                85                  90                  95

Phe Asn Lys Asn Glu Cys
            100

<210> 42
<211> 309
<212> DNA
<213> Ovis aries

<400> 42
ccatccgtct tcctcttcaa accatctgag gaacagctga ggaccggaac tgtctctgtc 60

gtgtgcttgg tgaatgattt ctaccccaaa gatatcaatg tcaaggtgaa agtggatggg 120

gttacccaga acagcaactt ccagaacagc ttcacagacc aggacagcaa gaaaagcacc 180

tacagcctca gcagcaccct gacactgtcc agctcagagt accagagcca taacgcctat 240

gcgtgtgagg tcagccacaa gagcctgccc accgccctcg tcaagagctt caataagaat 300

gaatgttag 309

<210> 43
<211> 106
<212> PRT
<213> Ovis aries

<400>  43

Gly Gln Pro Lys Ser Ala Pro Ser Val Thr Leu Phe Pro Pro Ser Thr
1               5                   10                  15

Glu Glu Leu Ser Thr Asn Lys Ala Thr Val Val Cys Leu Ile Asn Asp
            20                  25                  30

Phe Tyr Pro Gly Ser Val Asn Val Val Trp Lys Ala Asp Gly Ser Thr
        35                  40                  45

Ile Asn Gln Asn Val Lys Thr Thr Gln Ala Ser Lys Gln Ser Asn Ser
    50                  55                  60

Lys Tyr Ala Ala Ser Ser Tyr Leu Thr Leu Thr Gly Ser Glu Trp Lys
65                  70                  75                  80

Ser Lys Ser Ser Tyr Thr Cys Glu Val Thr His Glu Gly Ser Thr Val
                85                  90                  95

Thr Lys Thr Val Lys Pro Ser Glu Cys Ser
            100                 105

<210>  44
<211>  321
<212>  DNA
<213>  Ovis aries

<400>  44
ggtcagccca agtccgcacc ctcggtcacc ctgttcccgc cttccacgga ggagctcagt      60

accaacaagg ccaccgtggt gtgtctcatc aacgacttct acccgggtag cgtgaacgtg     120

gtctggaagg cagatggcag caccatcaat cagaacgtga agaccaccca ggcctccaaa     180

cagagcaaca gcaagtacgc ggccagcagc tacctgaccc tgacgggcag cgagtggaag     240

tctaagagca gttacacctg cgaggtcacg cacgagggga gcaccgtgac gaagacagtg     300

aagccctcag agtgttctta g                                               321

<210>  45
<211>  266
<212>  PRT
<213>  Bubalus bubalis

<400>  45

Ser Gly Val His Thr Phe Pro Ala Val Leu Gln Ser Ser Gly Leu Tyr
1               5                   10                  15

Ser Leu Ser Ser Thr Val Thr Ala Pro Ala Ser Ala Thr Lys Ser Gln
            20                  25                  30

Thr Phe Thr Cys Asn Val Ala His Pro Ala Ser Ser Thr Lys Val Asp
        35                  40                  45

Lys Ala Val Val Pro Pro Cys Arg Pro Lys Pro Cys Asp Cys Cys Pro
    50                  55                  60

Pro Pro Glu Leu Pro Gly Gly Pro Ser Val Phe Ile Phe Pro Pro Lys
65                  70                  75                  80

Pro Lys Asp Thr Leu Thr Ile Ser Gly Thr Pro Glu Val Thr Cys Val
                85                  90                  95

Val Val Asp Val Gly His Asp Asp Pro Glu Val Lys Phe Ser Trp Phe
            100                 105                 110

Val Asp Asp Val Glu Val Asn Thr Ala Arg Thr Lys Pro Arg Glu Glu
        115                 120                 125

Gln Phe Asn Ser Thr Tyr Arg Val Val Ser Ala Leu Pro Ile Gln His
    130                 135                 140

Asn Asp Trp Thr Gly Gly Lys Glu Phe Lys Cys Lys Val Tyr Asn Glu
145                 150                 155                 160

Gly Leu Pro Ala Pro Ile Val Arg Thr Ile Ser Arg Thr Lys Gly Gln
                165                 170                 175

Ala Arg Glu Pro Gln Val Tyr Val Leu Ala Pro Pro Gln Asp Glu Leu
            180                 185                 190

Ser Lys Ser Thr Val Ser Ile Thr Cys Met Val Thr Gly Phe Tyr Pro
        195                 200                 205

Asp Tyr Ile Ala Val Glu Trp Gln Lys Asp Gly Gln Pro Glu Ser Glu
    210                 215                 220

Asp Lys Tyr Gly Thr Thr Pro Pro Gln Leu Asp Ser Asp Gly Ser Tyr
225                 230                 235                 240

Phe Leu Tyr Ser Arg Leu Arg Val Asn Lys Asn Ser Trp Gln Glu Gly
                245                 250                 255

Gly Ala Tyr Thr Cys Val Val Met His Glu
            260                 265

<210>  46

<211>  801
<212>  DNA
<213>  Bubalus bubalis

<400>  46
gagcggcgtg cacaccttcc cggccgtcct tcagtcctcc gggctctact ctctcagcag      60

cacggtgacc gcgcccgcca gcgccacaaa aagccagacc ttcacctgca acgtagccca     120

cccggccagc agcaccaagg tggacaaggc tgttgttccc ccatgcagac cgaaaccctg     180

tgattgctgc ccaccccctg agctccccgg aggaccctct gtcttcatct tcccaccaaa     240

acccaaggac accctcacaa tctctggaac tcctgaggtc acgtgtgtgg tggtggacgt     300

gggccacgat gaccccgagg tgaagttctc ctggttcgtg gacgatgtgg aggtaaacac     360

agccaggacg aagccaagag aggagcagtt caacagcacc taccgcgtgg tcagcgccct     420

gcccatccag cacaacgact ggactggagg aaaggagttc aagtgcaagg tctacaatga     480

aggcctccca gcccccatcg tgaggaccat ctccaggacc aaagggcagg cccgggagcc     540

gcaggtgtac gtcctggccc caccccagga cgagctcagc aaaagcacgg tcagcatcac     600

ttgcatggtc actggcttct acccagacta catcgccgta gagtggcaga aagatgggca     660

gcctgagtca gaggacaaat atggcacgac cccgccccag ctggacagcg atggctccta     720

cttcctgtac agcaggctca gggtgaacaa gaacagctgg caagaaggag gcgcctacac     780

gtgtgtagtg atgcatgagg c                                               801

<210>  47
<211>  309
<212>  PRT
<213>  Bubalus bubalis

<400>  47

Ala Ser Ile Thr Ala Pro Lys Val Tyr Pro Leu Thr Ser Cys Arg Gly
1               5                   10                  15

Glu Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Thr Val Thr Ala Pro Ala Ser Ala Thr Lys Ser Gln Thr
65                  70                  75                  80

Phe Thr Cys Asn Val Ala His Pro Ala Ser Ser Thr Lys Val Asp Thr
                85                  90                  95

Ala Val Gly Phe Ser Ser Asp Cys Cys Lys Phe Pro Lys Pro Cys Val
            100                 105                 110

Arg Gly Pro Ser Val Phe Ile Phe Pro Pro Lys Pro Lys Asp Thr Leu
        115                 120                 125

Met Ile Thr Gly Asn Pro Glu Val Thr Cys Val Val Val Asp Val Gly
    130                 135                 140

Arg Asp Asn Pro Glu Val Gln Phe Ser Trp Phe Val Gly Asp Val Glu
145                 150                 155                 160

Val His Thr Gly Arg Ser Lys Pro Arg Glu Glu Gln Phe Asn Ser Thr
                165                 170                 175

Tyr Arg Val Val Ser Thr Leu Pro Ile Gln His Asn Asp Trp Thr Gly
            180                 185                 190

Gly Lys Glu Phe Lys Cys Lys Val Asn Asn Lys Gly Leu Pro Ala Pro
        195                 200                 205

Ile Val Arg Thr Ile Ser Arg Thr Lys Gly Gln Ala Arg Glu Pro Gln
    210                 215                 220

Val Tyr Val Leu Ala Pro Pro Gln Glu Glu Leu Ser Lys Ser Thr Val
225                 230                 235                 240

Ser Val Thr Cys Met Val Thr Gly Phe Tyr Pro Asp Tyr Ile Ala Val
                245                 250                 255

Glu Trp His Arg Asp Arg Gln Ala Glu Ser Glu Asp Lys Tyr Arg Thr
            260                 265                 270

Thr Pro Pro Gln Leu Asp Ser Asp Gly Ser Tyr Phe Leu Tyr Ser Arg
        275                 280                 285

Leu Lys Val Asn Lys Asn Ser Trp Gln Glu Gly Gly Ala Tyr Thr Cys
    290                 295                 300

Val Val Met His Glu
305

<210>  48
<211>  929
<212>  DNA
<213>  Bubalus bubalis

<400>  48
gcctccatca cagccccgaa agtctaccct ctgacttctt gccgcgggga aacgtccagc      60

tccaccgtga ccctgggctg cctggtctcc agctacatgc ccgagccggt gaccgtgacc     120

tggaactcgg gtgccctgaa gagcggcgtg cacaccttcc cggccgtcct tcagtcctct     180

gggctctact ctctcagcag cacggtgacc gcgcccgcca gcgccacaaa aagccagacc     240

ttcacctgca acgtagccca cccggccagc agcaccaagg tggacacggc tgttgggttc     300

tccagtgact gctgcaagtt tcctaagcct tgtgtgaggg gaccatctgt cttcatcttc     360

ccgccgaaac ccaaagacac cctgatgatc acaggaaatc ccgaggtcac atgtgtggtg     420

gtggacgtgg gccgggataa ccccgaggtg cagttctcct ggttcgtggg tgatgtggag     480

gtgcacacgg gcaggtcgaa gccgagagag gagcagttca acagcaccta ccgcgtggtc     540

agcaccctgc ccatccagca caatgactgg actggaggaa aggagttcaa gtgcaaggtc     600

aacaacaaag gcctcccagc ccccatcgtg aggaccatct ccaggaccaa agggcaggcc     660

cgggagccgc aggtgtacgt cctggcccca ccccaggaag agctcagcaa aagcacggtc     720

agcgtcactt gcatggtcac tggcttctac ccagactaca tcgccgtaga gtggcataga     780

gaccggcagg ctgagtcgga ggacaagtac cgcacgaccc cgccccagct ggacagcgat     840

ggctcctact tcctgtacag caggctcaag gtgaacaaga acagctggca agaaggaggc     900

gcctacacgt gtgtagtgat gcatgaggc                                       929

<210>  49
<211>  352
<212>  PRT
<213>  Bubalus bubalis

<400>  49

Ala Ser Thr Thr Ala Pro Lys Val Tyr Pro Leu Ala Ser Ser Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Asn
        35                  40                  45

Gly Val His Thr Phe Pro Ala Val Arg Gln Ser Ser Gly Leu Tyr Ser
    50                  55                  60

Leu Ser Ser Met Val Thr Met Pro Thr Ser Thr Ala Gly Thr Gln Thr
65                  70                  75                  80

Phe Thr Cys Asn Val Ala His Pro Ala Ser Ser Thr Lys Val Asp Thr
                85                  90                  95

Ala Val Thr Ala Arg His Pro Val Pro Lys Thr Pro Glu Thr Pro Ile
            100                 105                 110

His Pro Val Lys Pro Pro Thr Gln Glu Pro Arg Asp Glu Lys Thr Pro
        115                 120                 125

Cys Gln Cys Pro Lys Cys Pro Glu Pro Leu Gly Gly Leu Ser Val Phe
    130                 135                 140

Ile Phe Pro Pro Lys Pro Lys Asp Thr Leu Thr Ile Ser Gly Thr Pro
145                 150                 155                 160

Glu Val Thr Cys Val Val Val Asp Val Gly Gln Asp Asp Pro Glu Val
                165                 170                 175

Gln Phe Ser Trp Phe Val Asp Asp Val Glu Val His Thr Ala Arg Met
            180                 185                 190

Lys Pro Arg Glu Glu Gln Phe Asn Ser Thr Tyr Arg Val Val Ser Ala
        195                 200                 205

Leu Pro Ile Gln His Gln Asp Trp Leu Arg Glu Lys Glu Phe Lys Cys
    210                 215                 220

Lys Val Asn Asn Lys Gly Leu Pro Ala Pro Ile Val Arg Thr Ile Ser
225                 230                 235                 240

Arg Thr Lys Gly Gln Ala Arg Glu Pro Gln Val Tyr Val Leu Ala Pro
                245                 250                 255

Pro Arg Glu Glu Leu Ser Lys Ser Thr Leu Ser Leu Thr Cys Leu Ile
            260                 265                 270

Thr Gly Phe Tyr Pro Glu Glu Val Asp Val Glu Trp Gln Arg Asn Gly
        275                 280                 285

Gln Pro Glu Ser Glu Asp Lys Tyr His Thr Thr Pro Pro Gln Leu Asp
    290                 295                 300

Ala Asp Gly Ser Tyr Phe Leu Tyr Ser Arg Leu Arg Val Asn Arg Ser
305                 310                 315                 320

Ser Trp Gln Glu Gly Asp His Tyr Thr Cys Ala Val Met His Glu Ala
                325                 330                 335

Leu Arg Asn His Tyr Lys Glu Lys Pro Ile Ser Arg Ser Pro Gly Lys

340                 345                 350

<210>  50
<211>  1059
<212>  DNA
<213>  Bubalus bubalis

<400>  50
gcctccacca cagccccgaa agtctaccct ctggcatcca gctgcgggga cacgtccagc      60

tccaccgtga ccctgggctg cctggtctcc agctacatgc ccgagccggt gaccgtgacc     120

tggaactcgg gtgccctgaa gaacggcgtg cacaccttcc cggccgtccg gcagtcctcc     180

gggctctact ctctcagcag catggtgacc atgcccacca gcaccgcagg aacccagacc     240

ttcacctgca acgtagccca cccggccagc agcaccaagg tggacacggc tgtcactgca     300

aggcatccgg tcccgaagac accagagaca cctatccatc ctgtaaaacc cccaacccag     360

gagcccagag atgaaaagac accctgccag tgtcccaaat gcccagaacc tctgggagga     420

ctgtctgtct tcatcttccc accgaaaccc aaggacaccc tcacaatctc tggaacgccc     480

gaggtcacgt gtgtggtggt ggacgtgggc caggatgacc ccgaagtgca gttctcctgg     540

ttcgtggatg acgtggaggt gcacacagcc aggatgaagc caagagagga gcagttcaac     600

agcacctacc gcgtggtcag cgccctgccc atccagcacc aggactggct gcgggaaaag     660

gagttcaagt gcaaggtcaa caacaaaggc ctcccggccc ccatcgtgag gaccatctcc     720

aggaccaaag ggcaggcccg ggagccacag gtgtatgtcc tggccccacc ccgggaagag     780

ctcagcaaaa gcacgctcag cctcacctgc ctaatcaccg gcttctaccc agaagaggta     840

gacgtggagt ggcagagaaa tgggcagcct gagtcagagg acaagtacca cacgacccca     900

ccccagctgg acgctgacgg ctcctacttc ctgtacagca ggctcagggt gaacaggagc     960

agctggcagg aaggagacca ctacacgtgt gcagtgatgc atgaagcttt acggaatcac    1020

tacaaagaga agcccatctc gaggtctccg ggtaaatga                           1059

<210>  51
<211>  105
<212>  PRT
<213>  Bubalus bubalis

<400>  51

Gln Pro Lys Ser Ala Pro Ser Val Thr Leu Phe Pro Pro Ser Thr Glu
1               5                   10                  15

Glu Leu Ser Ala Asn Lys Ala Thr Leu Val Cys Leu Ile Ser Asp Phe
            20                  25                  30

Tyr Pro Gly Ser Met Thr Val Ala Arg Lys Ala Asp Gly Ser Thr Ile
        35                  40                  45

Thr Arg Asn Val Glu Thr Thr Arg Ala Ser Lys Gln Ser Asn Ser Lys
    50                  55                  60

Tyr Ala Ala Ser Ser Tyr Leu Ser Leu Thr Gly Ser Glu Trp Lys Ser
65                  70                  75                  80

Lys Gly Ser Tyr Ser Cys Glu Val Thr His Glu Gly Ser Thr Val Thr
                85                  90                  95

Lys Thr Val Lys Pro Ser Glu Cys Ser
            100                 105

<210>  52
<211>  318
<212>  DNA
<213>  Bubalus bubalis

<400>  52
cagcccaagt ccgcaccctc agtcaccctg ttcccaccct ccacggagga gctcagcgcc      60

aacaaggcca ccctggtgtg tctcatcagc gacttctacc cgggtagcat gaccgtggcc     120

aggaaggcag acggcagcac catcacccgg aacgtggaga ccacccgggc ctccaaacag     180

agcaacagca agtacgcggc cagcagctac ctgagcctga cgggcagcga gtggaaatcg     240

aaaggcagtt acagctgcga ggtcacgcac gaggggagca ccgtgacaaa gacagtgaag     300

ccctcagagt gttcttag                                                   318

<210>  53
<211>  229
<212>  PRT
<213>  Homo sapiens

<400>  53

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210>  54
<211>  690
<212>  DNA
<213>  Homo sapiens

<400>  54
gagtccaaat atggtccccc atgcccatca tgcccagcac ctgagttcct ggggggacca      60

tcagtcttcc tgttcccccc aaaacccaag gacactctca tgatctcccg gacccctgag     120

gtcacgtgcg tggtggtgga cgtgagccag gaagaccccg aggtccagtt caactggtac     180

gtggatggcg tggaggtgca taatgccaag acaaagccgc gggaggagca gttcaacagc     240

acgtaccgtg tggtcagcgt cctcaccgtc ctgcaccagg actggctgaa cggcaaggag     300

tacaagtgca aggtctccaa caaaggcctc ccgtcctcca tcgagaaaac catctccaaa     360

gccaaagggc agccccgaga gccacaggtg tacaccctgc ccccatccca ggaggagatg     420

accaagaacc aggtcagcct gacctgcctg gtcaaaggct tctaccccag cgacatcgcc     480

gtggagtggg agagcaatgg gcagccggag aacaactaca agaccacgcc tcccgtgctg 540

gactccgacg gctccttctt cctctacagc aggctaaccg tggacaagag caggtggcag 600

gaggggaatg tcttctcatg ctccgtgatg catgaggctc tgcacaacca ctacacacag 660

aagagcctct ccctgtctct gggtaaatga 690

<210> 55
<211> 229
<212> PRT
<213> Homo sapiens

<400> 55

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Val His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210>  56
<211>  690
<212>  DNA
<213>  Homo sapiens

<400>  56
gagtccaaat atggtccccc gtgcccatca tgcccagcac ctgagttcct ggggggacca      60

tcagtcttcc tgttcccccc aaaacccaag gacactctca tgatctcccg gacccctgag     120

gtcacgtgcg tggtggtgga cgtgagccag gaagaccccg aggtccagtt caactggtac     180

gtggatggcg tggaggtgca taatgccaag acaaagccgc gggaggagca gttcaacagc     240

acgtaccgtg tggtcagcgt cctcaccgtc gtgcaccagg actggctgaa cggcaaggag     300

tacaagtgca aggtctccaa caaaggcctc ccgtcctcca tcgagaaaac catctccaaa     360

gccaaagggc agccccgaga gccacaggtg tacaccctgc ccccatccca ggaggagatg     420

accaagaacc aggtcagcct gacctgcctg gtcaaaggct tctaccccag cgacatcgcc     480

gtggagtggg agagcaatgg gcagccggag aacaactaca agaccacgcc tcccgtgctg     540

gactccgacg gctccttctt cctctacagc aggctaaccg tggacaagag caggtggcag     600

gaggggaatg tcttctcatg ctccgtgatg catgaggctc tgcacaacca ctacacgcag     660

aagagcctct ccctgtctct gggtaaatga                                      690

<210>  57
<211>  217
<212>  PRT
<213>  Homo sapiens

<400>  57

Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Glu Gly Asn Val
            180                 185                 190

Phe Ser Cys Ser Val Met His Glu Ala Leu His Asn His Tyr Thr Gln
        195                 200                 205

Lys Ser Leu Ser Leu Ser Leu Gly Lys
    210                 215

<210>  58
<211>  654
<212>  DNA
<213>  Homo sapiens

<400>  58
gcacctgagt tcctgggggg accatcagtc ttcctgttcc ccccaaaacc caaggacact        60

ctcatgatct cccggacccc tgaggtcacg tgcgtggtgg tggacgtgag ccaggaagac       120

cccgaggtcc agttcaactg gtacgtggat ggcgtggagg tgcataatgc caagacaaag       180

ccgcgggagg agcagttcaa cagcacgtac cgtgtggtca gcgtcctcac cgtcctgcac       240

caggactggc tgaacggcaa ggagtacaag tgcaaggtct ccaacaaagg cctcccgtcc       300

tccatcgaga aaaccatctc caaagccaaa gggcagcccc gagagccaca ggtgtacacc       360

ctgcccccat cccaggagga gatgaccaag aaccaggtca gcctgacctg cctggtcaaa       420

ggcttctacc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcaggagggg aacgtcttct catgctccgt gatgcatgag 600

gctctgcaca accactacac gcagaagagc ctctccctgt ctctgggtaa atga 654

<210> 59
<211> 106
<212> PRT
<213> Homo sapiens

<400> 59

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210> 60
<211> 321
<212> DNA
<213> Homo sapiens

<400> 60
actgtggctg caccatctgt cttcatcttc ccgccatctg atgagcagtt gaaatctgga 60

actgcctctg ttgtgtgcct gctgaataac ttctatccca gagaggccaa agtacagtgg 120

aaggtggata acgccctcca atcgggtaac tcccaggaga gtgtcacaga gcaggacagc 180

aaggacagca cctacagcct cagcagcacc ctgacgctga gcaaagcaga ctacgagaaa 240

cacaaagtct acgcctgcga agtcacccat cagggcctga gctcgcccgt cacaaagagc 300

ttcaacaggg gagagtgtta g 321

<210>  61
<211>  32
<212>  DNA
<213>  Artificial Sequence

<220>
<223>  primer

<400>  61
atatgcggcc gcatggggac cccgcgggcg ct                                    32


<210>  62
<211>  30
<212>  DNA
<213>  Artificial Sequence

<220>
<223>  primer

<400>  62
gcgcaagctt tcagaggggc caggagcagt                                       30


<210>  63
<211>  35
<212>  DNA
<213>  Artificial Sequence

<220>
<223>  primer

<400>  63
ctagctagca ccatgaggat atatagtgtc ttaac                                 35


<210>  64
<211>  31
<212>  DNA
<213>  Artificial Sequence

<220>
<223>  primer

<400>  64
caatctcgag ttacagacag aagatgactg c                                     31


<210>  65
<211>  40
<212>  DNA
<213>  Artificial Sequence

<220>
<223>  primer

<400>  65
ataagaatgc ggccgccacc atggggaccc cgcgggcgct                            40


<210>  66
<211>  50

<212>  DNA
<213>  Artificial Sequence

<220>
<223>  primer

<400>  66
gccctcgagt taatggtgat ggtgatggtg gatgaccagg ctctgcatct          50


<210>  67
<211>  20
<212>  DNA
<213>  Artificial Sequence

<220>
<223>  primer

<400>  67
atggggaccc cgcgggcgcc                                            20


<210>  68
<211>  25
<212>  DNA
<213>  Artificial Sequence

<220>
<223>  primer

<400>  68
tcagaggggc caggagcagt gtcca                                      25


<210>  69
<211>  20
<212>  DNA
<213>  Artificial Sequence

<220>
<223>  primer

<400>  69
atggggaccc cgcgggcgct                                            20


<210>  70
<211>  20
<212>  DNA
<213>  Artificial Sequence

<220>
<223>  primer

<400>  70
gatgaccagg ctctgcatct                                            20


<210>  71
<211>  20
<212>  DNA
<213>  Artificial Sequence

<220>
<223>  primer

<400>  71
aatgacagcg gcgtctactt                                                    20


<210>  72
<211>  20
<212>  DNA
<213>  Artificial Sequence

<220>
<223>  primer

<400>  72
tcagaggggc caggagcagt                                                    20


<210>  73
<211>  29
<212>  DNA
<213>  Artificial Sequence

<220>
<223>  primer

<400>  73
gaagatctat ggggaccccg cgggcgccg                                          29


<210>  74
<211>  29
<212>  DNA
<213>  Artificial Sequence

<220>
<223>  primer

<400>  74
gacccgggga ggggccagga gcagtgtcc                                          29


<210>  75
<211>  1404
<212>  DNA
<213>  Artificial Sequence

<220>
<223>  DNA encoding chimeric H chain

<400>  75
atggcaatcc tcgtgttgct tctgtgcttg gtgaccattc cacactctgt gctttcccag        60

gtgcagctca aggaaacagg gccaggactc gtccaaccta cacaaaccct gtcaatcacc       120

tgtaccgtat ccggttttag cctcaccagc tattatatac aatgggtgag gcagaccccc       180

gggaaaggac tggaatggat gggcttcatt cgcagcggtg ggagtaccga gtacaatagc       240

gagtttaaaa gtcgcttgag tatcaataga gatacttcca agaatcaggt gttcttgaag       300

atgaactccc tcaagaccga agatacaggg gtctattact gcgccaggac ctccagtgga       360

tatgaaggag gctttgatta ttgggggcag ggcgtcatgg taactgtgag ctcagcctcc 420

accacagccc cgaaagtcta ccctctgagt tcttgctgcg gggacaagtc cagctccacc 480

gtgaccctgg gctgcctggt ctccagctac atgcccgagc cggtgaccgt gacctggaac 540

tcgggtgccc tgaagagcgg cgtgcacacc ttcccggctg tccttcagtc ctccgggctg 600

tactctctca gcagcatggt gaccgtgccc ggcagcacct caggacagac cttcacctgc 660

aacgtagccc acccggccag cagcaccaag gtggacaagg ctgttgatcc cacatgcaaa 720

ccatcaccct gtgactgttg cccaccccct gagctccccg gaggaccctc tgtcttcatc 780

ttcccaccga aacccaagga caccctcaca atctcgggaa cgcccgaggt cacgtgtgtg 840

gtggtggacg tgggccacga tgaccccgag gtgaagttct cctggttcgt ggacgacgtg 900

gaggtaaaca cagccacgac gaagccgaga gaggagcagt tcaacagcac ctaccgcgtg 960

gtcagcgccc tgcgcatcca gcaccaggac tggactggag gaaaggagtt caagtgcaag 1020

gtccacaacg aaggcctccc ggcccccatc gtgaggacca tctccaggac caaagggccg 1080

gcccgggagc cgcaggtgta tgtcctggcc ccaccccagg aagagctcag caaaagcacg 1140

gtcagcctca cctgcatggt caccagcttc tacccagact acatcgccgt ggagtggcag 1200

agaaacgggc agcctgagtc ggaggacaag tacggcacga ccccgcccca gctggacgcc 1260

gacagctcct acttcctgta cagcaagctc agggtggaca ggaacagctg gcaggaagga 1320

gacacctaca cgtgtgtggt gatgcacgag gccctgcaca atcactacac gcagaagtcc 1380

acctctaagt ctgcgggtaa ataa 1404

<210> 76
<211> 467
<212> PRT
<213> Artificial Sequence

<220>
<223> chimeric H chain

<400> 76

Met Ala Ile Leu Val Leu Leu Leu Cys Leu Val Thr Ile Pro His Ser
1               5                   10                  15

Val Leu Ser Gln Val Gln Leu Lys Glu Thr Gly Pro Gly Leu Val Gln
            20                  25                  30

Pro Thr Gln Thr Leu Ser Ile Thr Cys Thr Val Ser Gly Phe Ser Leu
        35                  40                  45

Thr Ser Tyr Tyr Ile Gln Trp Val Arg Gln Thr Pro Gly Lys Gly Leu
    50                  55                  60

Glu Trp Met Gly Phe Ile Arg Ser Gly Gly Ser Thr Glu Tyr Asn Ser
65                  70                  75                  80

Glu Phe Lys Ser Arg Leu Ser Ile Asn Arg Asp Thr Ser Lys Asn Gln
                85                  90                  95

Val Phe Leu Lys Met Asn Ser Leu Lys Thr Glu Asp Thr Gly Val Tyr
            100                 105                 110

Tyr Cys Ala Arg Thr Ser Ser Gly Tyr Glu Gly Gly Phe Asp Tyr Trp
        115                 120                 125

Gly Gln Gly Val Met Val Thr Val Ser Ser Ala Ser Thr Thr Ala Pro
    130                 135                 140

Lys Val Tyr Pro Leu Ser Ser Cys Cys Gly Asp Lys Ser Ser Ser Thr
145                 150                 155                 160

Val Thr Leu Gly Cys Leu Val Ser Ser Tyr Met Pro Glu Pro Val Thr
                165                 170                 175

Val Thr Trp Asn Ser Gly Ala Leu Lys Ser Gly Val His Thr Phe Pro
            180                 185                 190

Ala Val Leu Gln Ser Ser Gly Leu Tyr Ser Leu Ser Ser Met Val Thr
        195                 200                 205

Val Pro Gly Ser Thr Ser Gly Gln Thr Phe Thr Cys Asn Val Ala His
    210                 215                 220

Pro Ala Ser Ser Thr Lys Val Asp Lys Ala Val Asp Pro Thr Cys Lys
225                 230                 235                 240

Pro Ser Pro Cys Asp Cys Cys Pro Pro Pro Glu Leu Pro Gly Gly Pro
                245                 250                 255

Ser Val Phe Ile Phe Pro Pro Lys Pro Lys Asp Thr Leu Thr Ile Ser
            260                 265                 270

Gly Thr Pro Glu Val Thr Cys Val Val Val Asp Val Gly His Asp Asp
        275                 280                 285

Pro Glu Val Lys Phe Ser Trp Phe Val Asp Asp Val Glu Val Asn Thr
    290                 295                 300

Ala Thr Thr Lys Pro Arg Glu Glu Gln Phe Asn Ser Thr Tyr Arg Val
305                 310                 315                 320

Val Ser Ala Leu Arg Ile Gln His Gln Asp Trp Thr Gly Gly Lys Glu
                325                 330                 335

Phe Lys Cys Lys Val His Asn Glu Gly Leu Pro Ala Pro Ile Val Arg
            340                 345                 350

Thr Ile Ser Arg Thr Lys Gly Pro Ala Arg Glu Pro Gln Val Tyr Val
        355                 360                 365

Leu Ala Pro Pro Gln Glu Glu Leu Ser Lys Ser Thr Val Ser Leu Thr
    370                 375                 380

Cys Met Val Thr Ser Phe Tyr Pro Asp Tyr Ile Ala Val Glu Trp Gln
385                 390                 395                 400

Arg Asn Gly Gln Pro Glu Ser Glu Asp Lys Tyr Gly Thr Thr Pro Pro
                405                 410                 415

Gln Leu Asp Ala Asp Ser Ser Tyr Phe Leu Tyr Ser Lys Leu Arg Val
            420                 425                 430

Asp Arg Asn Ser Trp Gln Glu Gly Asp Thr Tyr Thr Cys Val Val Met
        435                 440                 445

His Glu Ala Leu His Asn His Tyr Thr Gln Lys Ser Thr Ser Lys Ser
    450                 455                 460

Ala Gly Lys
465

<210>  77
<211>  28
<212>  DNA
<213>  Artificial Sequence

<220>
<223>  primer

<400>  77
ctagctagca ccacagcccc gaaagtct                                        28

<210>  78
<211>  30
<212>  DNA
<213>  Artificial Sequence

<220>
<223>  primer

<400>  78

tgctctagat tatttacccg cagacttaga                                        30

<210>  79
<211>  40
<212>  DNA
<213>  Artificial Sequence

<220>
<223>  primer

<400>  79
ataagaatgc ggccgccacc atgtggctca taatagctct                             40

<210>  80
<211>  50
<212>  DNA
<213>  Artificial Sequence

<220>
<223>  primer

<400>  80
gccctcgagt taatggtgat ggtgatggtg aggagttgtt gactggaggc                  50

<210>  81
<211>  38
<212>  DNA
<213>  Artificial Sequence

<220>
<223>  primer

<400>  81
ataagaatgc tagccaccat ggggatcccc tcattcct                               38

<210>  82
<211>  50
<212>  DNA
<213>  Artificial Sequence

<220>
<223>  primer

<400>  82
gccgatatct taatggtgat ggtgatggtg cgatgagggg ccgctcgagc                  50

<210>  83
<211>  40
<212>  DNA
<213>  Artificial Sequence

<220>
<223>  primer

<400>  83
ataagaatgc ggccgccacc atgtggcaac tgctaccacc                             40

<210>  84
<211>  50
<212>  DNA
<213>  Artificial Sequence

<220>
<223>  primer

<400>  84
gccctcgagt taatggtgat ggtgatggtg gtgccaaggt agaaagaatg                50


<210>  85
<211>  48
<212>  DNA
<213>  Artificial Sequence

<220>
<223>  primer

<400>  85
ataagaatgc ggccgccacc atggccccca ccctccctgc cttgctct                  48


<210>  86
<211>  50
<212>  DNA
<213>  Artificial Sequence

<220>
<223>  primer

<400>  86
gccctcgagt taatggtgat ggtgatggtg attctgcatc gtgtagtctg                50

## Description

### TECHNICAL FIELD

The present invention relates to an anti-PD-1 antibody. More specifically, the present invention relates to an anti-PD-1 antibody comprising a variable region containing complementarity-determining regions (CDRs) of a rat anti-bovine PD-1 antibody and a constant region of an antibody of an animal other than rat.

### BACKGROUND ART

Programmed cell death 1 (PD-1), an immunoinhibitory receptor, and its ligand programmed cell death ligand 1 (PD-L1) are molecules identified by Prof. Tasuku Honjo et al., Kyoto University, as factors which inhibit excessive immune response and are deeply involved in immunotolerance (Non-Patent Document No. 1: Ishida Y, Agata Y, Shibahara K, Honjo T, The EMBO J., 11(11); Nov. 1992). Recently, it has been elucidated that these molecules are also involved in immunosuppression in tumors. In the field of human medical care, an antibody drug that inhibits the effect of PD-1 has been developed and put into practical use (Opdivo™, Ono Pharmaceutical Co., Ltd.).

To date, the present inventors have been developing an immunotherapy for animal refractory diseases targeting PD-1 or PD-L1, and have revealed that this novel immunotherapy is applicable to multiple-diseases and multile-animals. (Non-Patent Document No. 2: Ikebuchi R, Konnai S, Shirai T, Sunden Y, Murata S, Onuma M, Ohashi K. Vet. Res., 42:103; Sep. 2011; Non-Patent Document No. 3: Ikebuchi R, Konnai S, Okagawa T, Yokoyama K, Nakajima C, Suzuki Y, Murata S, Ohashi K. Vet. Res., 44:59; Jul. 22, 2013; Non-Patent Document No. 4: Ikebuchi R, Konnai S, Okagawa T, Yokoyama K, Nakajima C, Suzuki Y, Murata S, Ohashi K. Immunology, 142(4):551-61; Aug. 2014; Non-Patent Document No. 5: Maekawa N, Konnai S, Ikebuchi R, Okagawa T, Adachi M, Takagi S, Kagawa Y, Nakajima C, Suzuki Y, Murata S, Ohashi K. PLoS One, 9(6):e98415; Jun. 10, 2014; Non-Patent Document No. 6: Mingala CN, Konnai S, Ikebuchi R, Ohashi K. Comp. Immunol. Microbiol. Infect. Dis., 34(1):55-63; Jan. 2011).

However, the antibodies which the present inventors have prepared to date are rat antibodies, and therefore it is impossible to administer those antibodies repeatedly to animals other than rat.

### PRIOR ART LITERATURE

### Non-Patent Documents

Non-Patent Document No. 1: Ishida Y, Agata Y, Shibahara K, Honjo T. The EMBO Journal. 11(11):3887-3895; Nov. 1992
Non-Patent Document No. 2: Ikebuchi R, Konnai S, Shirai T, Sunden Y, Murata S, Onuma M, Ohashi K. Vet. Res., 42:103; Sep. 2011.
Non-Patent Document No. 3: Ikebuchi R, Konnai S, Okagawa T, Yokoyama K, Nakajima C, Suzuki Y, Murata S, Ohashi K. Vet. Res., 44:59; Jul. 22, 2013.
Non-Patent Document No. 4: Ikebuchi R, Konnai S, Okagawa T, Yokoyama K, Nakajima C, Suzuki Y, Murata S, Ohashi K. Immunology, 142(4):551-61; Aug. 2014.
Non-Patent Document No. 5: Maekawa N, Konnai S, Ikebuchi R, Okagawa T, Adachi M, Takagi S, Kagawa Y, Nakajima C, Suzuki Y, Murata S, Ohashi K. PLoS One, 9(6):e98415; Jun. 10, 2014.
Non-Patent Document No. 6: Mingala CN, Konnai S, Ikebuchi R, Ohashi K. Comp. Immunol. Microbiol. Infect. Dis., 34(1):55-63; Jan. 2011.

### DISCLOSURE OF THE INVENTION

### PROBLEM FOR SOLUTION BY THE INVENTION

It is an object of the present invention to provide an anti-PD-1 antibody capable of repeated administration even to animals other than rat.

### MEANS TO SOLVE THE PROBLEM

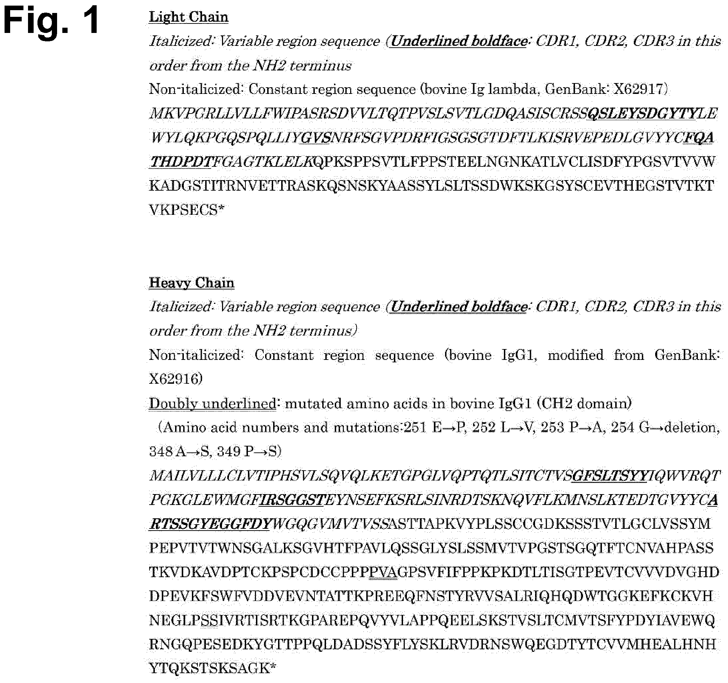

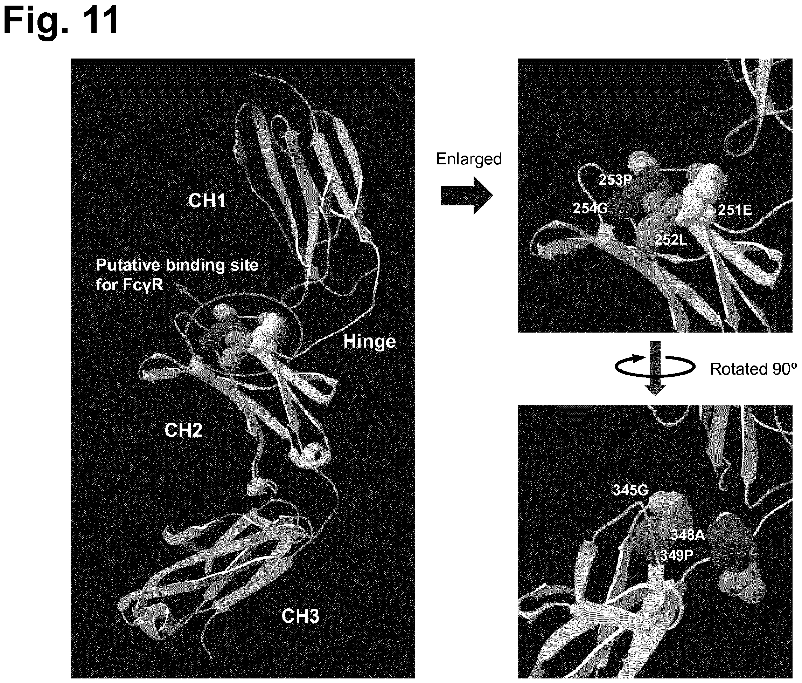

The present inventors have determined the variable regions of a rat anti-bovine PD-1 monoclonal antibody (5D2) capable of inhibiting the binding of bovine PD-1 to PD-L1, and then combined genes encoding the resultant variable regions with genes encoding the constant regions of a bovine immunoglobulin (bovine IgG1, with mutations having been introduced into the putative binding sites of Fcγ receptors in CH2 domain in order to inhibit ADCC activity; see Figs. 1 and 11 for amino acid numbers and mutations: 251 E→P, 252 L→V, 253 P→A, 254 G→deletion, 348 A→S, 349 P→S; Ikebuchi R, Konnai S, Okagawa T, Yokoyama K, Nakajima C, Suzuki Y, Murata S, Ohashi K. Immunology 2014 Aug; 142(4):551-561) to thereby obtain a chimeric antibody gene. This gene was introduced into Chinese hamster ovary cells (CHO cells). By culturing/proliferating the resultant cells, the present inventors have succeeded in preparing a rat-bovine chimeric anti-bovine PD-1 antibody. Further, the present inventors have determined the CDRs of the variable regions of rat anti-bovine PD-1 monoclonal antibody (5D2). The present invention has been achieved based on these findings.

A summary of the present invention is as described below.
(1) An anti-PD-1 antibody comprising (a) a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat; and (b) a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.
(2) The antibody of (1) above, wherein the light chain variable region and the heavy chain variable region are derived from rat.
(3) The antibody of (2) above, wherein the light chain variable region is the light chain variable region of a rat anti-bovine PD-1 antibody and the heavy chain variable region is the heavy chain variable region of a rat anti-bovine PD-1 antibody.
(4) The antibody of (3) above, wherein the light chain variable region has the amino acid sequence as shown in SEQ ID NO. 1 and the heavy chain variable region has the amino acid sequence as shown in SEQ ID NO: 2.
(5) The antibody of any one of (1) to (4) above, wherein the light chain constant region of an antibody of an animal other than rat has the amino acid sequence of the constant region of lambda chain or kappa chain.
(6) The antibody of any one of (1) to (5) above, wherein the heavy chain constant region of an antibody of an animal other than rat has the amino acid sequence of the constant region of an immunoglobulin equivalent to human IgG4, or has mutations introduced thereinto that reduce ADCC activity and/or CDC activity.
(7) The antibody of (6) above, wherein the animal other than rat is bovine; the light chain constant region of the bovine antibody has the amino acid sequence of the constant region of lambda chain; and the heavy chain constant region of the bovine antibody has mutations introduced thereinto that reduce ADCC activity and/or CDC activity.
(8) The antibody of (7) above, wherein the light chain constant region of the bovine antibody has the amino acid sequence as shown in SEQ ID NO: 3 and the heavy chain constant region of the bovine antibody has the amino acid sequence as shown in SEQ ID NO: 4
(9) The antibody of any one of (1) to (8) above which has a four-chain structure comprising two light chains and two heavy chains.
(10) A pharmaceutical composition comprising the antibody of any one of (1) to (9) above as an active ingredient.
(11) The composition of (10) above for prevention and/or treatment of cancers and/or inflammations.
(12) The composition of (11) above, wherein the cancers and/or inflammations are selected from the group consisting of neoplastic diseases, leukemia, Johne's disease, anaplasmosis, bacterial mastitis, mycotic mastitis, mycoplasma infections (such as mycoplasma mastitis, mycoplasma pneumonia or the like), tuberculosis, *Theileria orientalis* infection, cryptosporidiosis, coccidiosis, trypanosomiasis and leishmaniasis.
(13) An artificial genetic DNA comprising (a') a DNA encoding a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat and (b') a DNA encoding a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.
(14) A vector comprising the artificial genetic DNA of (13) above.
(15) A host cell transformed with the vector of (14) above.
(16) A method of preparing an antibody, comprising culturing the host cell of (15) above and collecting an anti-PD-1 antibody from the resultant culture.
(17) A DNA encoding a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat.
(18) A DNA encoding a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.

### EFFECT OF THE INVENTION

According to the present invention, a novel anti-PD-1 antibody has been obtained. This antibody is applicable even to those animals other than rat.

The present specification encompasses the contents disclosed in the specifications and/or drawings of Japanese Patent Applications No. 2016-159090 and No. 2017-099615 based on which the present patent application claims priority.

### BRIEF DESCRIPTION OF THE DRAWINGS

[Fig. 1] The amino acid sequence of rat-bovine chimeric anti-bovine PD-1 antibody ch5D2. CDR1, CDR2 and CDR3 in the light chain and the heavy chain variable regions of rat anti-bovine PD-1 antibody 5D2 are shown. Further, amino acids introduced as mutations to bovine IgG1 (CH2 domain) are also shown (amino acid numbers and mutations: 251 E→P, 252 L→V, 253 P→A, 254 G→deletion, 348 A→S, 349 P→S).
[Fig. 2] Schematic drawings of pDN112 vector and rat-bovine chimeric anti-bovine PD-1 antibody ch5D2.
[Fig. 3] The amount of production and the purity after purification of rat-bovine chimeric anti-bovine PD-1 antibody ch5D2.
[Fig. 4] Binding property of rat-bovine chimeric anti-bovine PD-1 antibody ch5D2.
[Fig. 5] Inhibitory activity of rat-bovine chimeric anti-bovine PD-1 antibody ch5D2 against bovine PD-1/PD-L1 binding.
[Fig. 6] Transition in blood concentrations of rat-bovine chimeric anti-bovine PD-1 antibody ch5D2 after administration to the cattle experimentally infected with BLV.
[Fig. 7] Proliferative response of T cells against BLV antigen in the cattle experimentally infected with BLV through administration of rat-bovine chimeric anti-bovine PD-1 antibody ch5D2.
[Fig. 8] Changes in the BLV provirus load in the cattle experimentally infected with BLV through administration of rat-bovine chimeric anti-bovine PD-1 antibody ch5D2.
[Fig. 9] Cross-reactivity of rat anti-bovine PD-1 antibody 5D2 with ovine PD-1
[Fig. 10] Cross-reactivity of rat anti-bovine PD-1 antibody 5D2 with water buffalo T cells
[Fig. 11] 3D structure of bovine IgG1 constant region and putative binding site for Fcγ receptors
[Fig. 12] pDC6 vector
[Fig. 13] Purities after purification of rat-bovine chimeric anti-bovine PD-1 antibodies ch5D2 IgG1 WT and IgG1 ADCC-.
[Fig. 14] Binding of rat-bovine chimeric anti-bovine PD-1 antibodies ch5D2 IgG1 WT and IgG1 ADCC- to individual bovine Fcγ receptors.

### BEST MODES FOR CARRYING OUT THE INVENTION

Hereinbelow, the present invention will be described in detail.

The present invention provides an anti-PD-1 antibody comprising (a) a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat; and (b) a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.

CDR1, CDR2 and CDR3 in the light chain variable region (VL) of rat anti-bovine PD-1 antibody 5D2 (to be described later) are respectively a region consisting of the amino acid sequence of QSLEYSDGYTY (SEQ ID NO: 16), a region consisting of the amino acid sequence of GVS and a region consisting of the amino acid sequence of FQATHDPDT (SEQ ID NO: 17) (see Fig. 1).

Further, CDR1, CDR2 and CDR3 in the heavy chain variable region (VH) of rat anti-bovine PD-1 antibody 5D2 are respectively a region consisting of the amino acid sequence of GFSLTSYY (SEQ ID NO: 18), a region consisting of the amino acid sequence of IRSGGST (SEQ ID NO: 19) and a region consisting of the amino acid sequence of ARTSSGYEGGFDY (SEQ ID NO: 20) (see Fig.1).

In the amino acid sequences of QSLEYSDGYTY (SEQ ID NO: 16), GVS and FQATHDPDT (SEQ ID NO: 17), as well as the amino acid sequences of GFSLTSYY (SEQ ID NO: 18), IRSGGST (SEQ ID NO: 19) and ARTSSGYEGGFDY (SEQ ID NO: 20), one, two, three, four or five amino acids may be deleted, substituted or added. Even when such mutations are introduced, the resulting amino acid sequences may be capable of having the function of a CDR in the light chain or heavy chain variable region of the PD-1 antibody.

As used herein, the term "antibody" is a concept encompassing not only full-length antibodies but also antibodies of smaller molecular sizes such as Fab, F(ab)'₂, ScFv, Diabody, V_{H}, V_{L}, Sc(Fv)₂, Bispecific sc(Fv)₂, Minibody, scFv-Fc monomer or scFv-Fc dimer.

In the anti-PD-1 antibody of the present invention, the VL and VH may be derived from rat. For example, the VL may be the VL of a rat anti-bovine PD-1 antibody, and the VH may be the VH of the rat anti-bovine PD-1 antibody.

The amino acid sequence of the VL and the amino acid sequence of the VH of the rat anti-bovine PD-1 antibody are shown in SEQ ID NOS: 1 and 2, respectively. The amino acid sequences as shown in SEQ ID NOS: 1 and 2 may have deletion(s), substitution(s) or addition(s) of one or several (e.g., up to five, about 10 at the most) amino acids. Even when such mutations are introduced, the resulting amino acid sequences may be capable of having the function as VL or VH of the PD-1 antibody.

The VL and VH of an antibody of an animal other than rat may be derived from an animal which produces a PD-1 that cross-reacts with rat anti-bovine PD-1 antibody 5D2.

There are two types of immunoglobulin light chain, which are called Kappa chain (κ) and Lambda chain (λ). In the anti-PD-1 antibody of the present invention, the light chain constant region (CL) of an antibody of an animal other than rat may have the amino acid sequence of the constant region of either Kappa chain or Lambda chain. However, the relative abundance of Lambda chain is higher in bovine, ovine, feline, canine and equine, and that of Kappa chain is higher in mouse, rat, human and porcine. Since a chain with a higher relative abundance is considered to be preferable, a bovine, ovine, feline, canine or equine antibody preferably has the amino acid sequence of the constant region of Lambda chain whereas a mouse, rat, human or porcine antibody preferably has the amino acid sequence of the constant region of Kappa chain.

The heavy chain constant region (CH) of an antibody of an animal other than rat may have the amino acid sequence of the constant region of an immunoglobulin equivalent to human IgG4. Immunoglobulin heavy chain is classified into γ chain, µ chain, α chain, δ chain and ε chain depending on the difference in constant region. According to the type of heavy chain present, five classes (isotypes) of immunoglobulin are formed; they are IgG, IgM, IgA, IgD and IgE.

Immunoglobulin G (IgG) accounts for 70-75% of human immunoglobulins and is the most abundantly found monomeric antibody in plasma. IgG has a four-chain structure consisting of two light chains and two heavy chains. Human IgG1, IgG2 and IgG4 have a molecular weight of about 146,000, whereas human IgG3 has a long hinge region that connects Fab region and Fc region and has a larger molecular weight of 170,000. Human IgG1 accounts for about 65% of human IgG, human IgG2 about 25%, human IgG3 about 7%, and human IgG4 about 3%. They are uniformly distributed inside and outside of blood vessels. Having a strong affinity for Fc receptors and complement factors on effector cell surfaces, human IgG1 induces antibody-dependent cell cytotoxicity (ADCC) and also activates complements to induce complement-dependent cell cytotoxicity (CDC). Human IgG2 and IgG4 are low at ADCC and CDC activities because their affinity for Fc receptors and complement factors is low.

Immunoglobulin M (IgM), which accounts for about 10% of human immunoglobulins, is a pentameric antibody consisting of five basic four-chain structures joined together. It has a molecular weight of 970,000. Usually occurring only in blood, IgM is first produced against infectious microorganisms and takes charge of early stage immunity.

Immunoglobulin A (IgA) accounts for 10-15% of human immunoglobulins. It has a molecular weight of 160,000. Secreted IgA is a dimeric antibody consisting of two IgA molecules joined together. IgAl is found in serum, nasal discharge, saliva and breast milk. In intestinal juice, IgA2 is found abundantly.

Immunoglobulin D (IgD) is a monomeric antibody accounting for no more than 1% of human immunoglobulins. IgD is found on B cell surfaces and involved in induction of antibody production.

Immunoglobulin E (IgE) is a monomeric antibody that occurs in an extremely small amount, accounting for only 0.001% or less of human immunoglobulins. Immunoglobulin E is considered to be involved in immune response to parasites but in advanced countries where parasites are rare, IgE is largely involved in bronchial asthma and allergy among other things.

In canine, sequences of IgG-A (equivalent to human IgG2), IgG-B (equivalent to human IgG1), IgG-C (equivalent to human IgG3) and IgG-D (equivalent to human IgG4) have been identified as the heavy chain of IgG. In the antibody of the present invention, an IgG's heavy chain constant region with neither ADCC activity nor CDC activity is preferable (IgG4 in human). In the case where the constant region of an immunoglobulin equivalent to human IgG4 has not been identified, one may use a constant region that has lost both ADCC activity and CDC activity as a result of introducing mutations into the relevant region of an immunoglobulin equivalent to human IgG4.

In bovine, sequences of IgG1, IgG2 and IgG3 have been identified as the heavy chain of IgG. In the antibody of the present invention, an IgG's heavy chain constant region with neither ADCC activity nor CDC activity is preferable (IgG4 in human). Although the constant region of wild-type human IgG1 has ADCC activity and CDC activity, it is known that these activities can be reduced by introducing amino acid substitutions or deletions into specific sites. In bovine, the constant region of an immunoglobulin equivalent to human IgG4 has not been identified, so mutations may be added to the relevant region of an immunoglobulin equivalent to human IgG1 and the resultant constant region then used. As one example, the amino acid sequence of the CH of a bovine antibody (IgG1 chain, GenBank: X62916) having mutations introduced into CH2 domain and a nucleotide sequence for such amino acid sequence (after codon optimization) are shown in SEQ ID NOS: 4 and 8, respectively.

An anti-PD-1 antibody is more preferable in which (i) the CL of a bovine antibody has the amino acid sequence of the constant region of Lambda chain and (ii) the CH of the bovine antibody has mutations introduced thereinto that reduce ADCC activity and/or CDC activity.

The anti-PD-1 antibody of the present invention encompasses rat-bovine chimeric antibodies, bovinized antibodies and complete bovine-type antibodies. However, the animal is not limited to bovine and may be exemplified by human, canine, porcine, simian, mouse, feline, equine, goat, ovine, water buffalo, rabbit, hamster, guinea pig and the like.

For example, the anti-PD-1 antibody of the present invention may be an anti-PD-1 antibody in which the CL of a bovine antibody has the amino acid sequence as shown in SEQ ID NO: 3 and the CH of the bovine antibody has the amino acid sequence as shown in SEQ ID NO: 4.

The amino acid sequences as shown in SEQ ID NOS: 3 and 4 may have deletion(s), substitution(s) or addition(s) of one or several (e.g., up to five, about 10 at the most) amino acids. Even when such mutations are introduced, the resulting amino acid sequences may be capable of having the function as CL or CH of the PD-1 antibody.

The anti-PD-1 antibody of the present invention may have a four-chain structure comprising two light chains and two heavy chains.

The anti-PD-1 antibody of the present invention may be prepared as described below. Briefly, an artificial gene is synthesized which comprises (i) the identified variable region sequences of a rat anti-bovine PD-1 antibody and (ii) the constant region sequences of an antibody of an animal other than rat (e.g., bovine) (preferably, an immunoglobulin equivalent to human IgG1, in which mutations have been introduced into the relevant region to reduce ADCC activity and/or CDC activity). The resultant gene is inserted into a vector (e.g., plasmid), which is then introduced into a host cell (e.g., mammal cell such as CHO cell). The host cell is cultured, and the antibody of interest is collected from the resultant culture.

The amino acid sequence and the nucleotide sequence of the VL of the rat anti-bovine PD-1 antibody identified by the present inventors are shown in SEQ ID NOS: 1 and 5, respectively. Further, the nucleotide sequence after codon optimization is shown in SEQ ID NO: 11.

The amino acid sequence and the nucleotide sequence of the VH of the rat anti-bovine PD-1 antibody identified by the present inventors are shown in SEQ ID NOS: 2 and 6, respectively. Further, the nucleotide sequence after codon optimization is shown in SEQ ID NO: 12.

The amino acid sequence and the nucleotide sequence of the CL (Lambda chain, GenBank: X62917) of a bovine antibody are shown in SEQ ID NOS: 3 and 7, respectively. Further, the nucleotide sequence after codon optimization is shown in SEQ ID NO: 13.

The amino acid sequence and the nucleotide sequence (after codon optimization) of the CH (IgG1 chain, modified from GenBank: X62916) of a bovine antibody are shown in SEQ ID NOS: 4 and 8, respectively.

Further, SEQ ID NO: 9 shows the amino acid sequence of a chimeric light chain consisting of the VL of the rat anti-bovine PD-1 antibody and the CL (Lambda chain, GenBank: X62917) of the bovine antibody. The nucleotide sequence (after codon optimization) of the chimeric light chain consisting of the VL of the rat anti-PD-1 antibody and the CL (Lambda chain, GenBank: X62917) of the bovine antibody is shown in SEQ ID NO: 14.

SEQ ID NO: 10 shows the amino acid sequence of a chimeric heavy chain consisting of the VH of the rat anti-bovine PD-1 antibody and the CH (IgG1 chain, modified from GenBank: X62916) of the bovine antibody. The nucleotide sequence (after codon optimization) of the chimeric heavy chain consisting of the VH of the rat anti-bovine PD-1 antibody and the CH (IgG1 chain, modified from GenBank: X62916) of the bovine antibody is shown in SEQ ID NO: 15.

Amino acid sequences and nucleotide sequences of CLs and CHs of various animals other than rat may be obtained from known databases for use in the present invention.

Amino acid sequences and nucleotide sequences of bovine CL and CH are summarized in the table below.

**(Table)**

| Species | Ig Domain | | Nucleotide Sequence | Amino Acid Sequence | GenBank Accession No. | IMGT Database | Reference |
|---|---|---|---|---|---|---|---|
| Bovine (Scientific Name: Bos Taurus) | Bovine Ig heavy chain constant region (CH1∼C H3) | IgG1 variant 1 | GCCTCCACCACAGCCCCGAAAGTCTACCCTCTGAGTTCTT GCTGCGGGGACAAGTCCAGCTCCACCGTGACCCTGGGCT GCCTGGTCTCCAGCTACATGCCCGAGCCGGTGACCGTGA CCTGGAACTCGGGTGCCCTGAAGAGCGGCGTGCACACCT TCCCGGCTGTCCTTCAGTCCTCCGGGCTGTACTCTCTCAG CAGCATGGTGACCGTGCCCGGCAGCACCTCAGGACAGAC CTTCACCTGCAACGTAGCCCACCCGGCCAGCAGCACCAA GGTGGACAAGGCTGTTGATCCCACATGCAAACCATCACC CTGTGACTGTTGCCCACCCCCTGAGCTCCCCGGAGGACC CTCTGTCTTCATCTTCCCACCGAAACCCAAGGACACCCTC ACAATCTCGGGAACGCCCGAGGTCACGTGTGTGGTGGTG GACGTGGGCCACGATGACCCCGAGGTGAAGTTCTCCTGG TTCGTGGACGACGTGGAGGTAAACACAGCCACGACGAA GCCGAGAGAGGAGCAGTTCAACAGCACCTACCGCGTGGT CAGCGCCCTGCGCATCCAGCACCAGGACTGGACTGGAGG AAAGGAGTTCAAGTGCAAGGTCCACAACGAAGGCCTCCC GGCCCCCATCGTGAGGACCATCTCCAGGACCAAAGGGCC GGCCCGGGAGCCGCAGGTGTATGTCCTGGCCCCACCCCA GGAAGAGCTCAGCAAAAGCACGGTCAGCCTCACCTGCAT GGTCACCAGCTTCTACCCAGACTACATCGCCGTGGAGTG GCAGAGAAACGGGCAGCCTGAGTCGGAGGACAAGTACG GCACGACCCCGCCCCAGCTGGACGCCGACAGCTCCTACT TCCTGTACAGCAAGCTCAGGGTGGACAGGAACAGCTGGC AGGAAGGAGACACCTACACGTGTGTGGTGATGCACGAG GCCCTGCACAATCACTACACGCAGAAGTCCACCTCTAAG TCTGCGGGTAAATGA (SEQ ID NO: 29) | ASTTAPKVYPLS SCCGDKSSSTVT LGCLVSSYMPEP VTVTWNSGALK SGVHTFPAVLQS SGLYSLSSMVTV PGSTSGQTFTCN VAHPASSTKVD KAVDPTCKPSPC DCCPPPELPGGP SVFIFPPKPKDTL TISGTPEVTCVV VDVGHDDPEVK FSWFVDDVEVN TATTKPREEQFN STYRVVSALRIQ HQDWTGGKEFK CKVHNEGLPAPI VRTISRTKGPAR EPQVYVLAPPQE ELSKSTVSLTCM VTSFYPDYIAVE WQRNGQPESED KYGTTPPQLDA DSSYFLYSKLRV DRNSWQEGDTY TCVVMHEALHN HYTQKSTSKSA GK* (SEQ ID NO: 21) | X62916 | http://www.imgt.org/IMGTrepertoire/index.php?section=LocusGenes&repertoire=genetable&species =bovine&group=IGHC | Symons D.B. et al., J. Immunogenet., 14, 273-283 (1987). PMID: 3141517 |
| | | | | | | | Symons D.B. et al., Mol. Immunol., 26, 841-850 (1989). PMID: 2513487 |
| | | | | | | | Kacskovics I. and Butler J.E., Mol. Immunol., 33, 189-195 (1996). PMID: 8649440 |
| | | | | | | | Rabbani H. et al., Immunogenetics, 46, 326-331 (1997). PMID: 9218535 |
| | | | | | | | Saini S.S. et al., Scand. J. Immunol. 65, 32-8 (2007). PMID: 17212764 |
| | | IgG1 variant 2 | GCCTCCACCACAGCCCCGAAAGTCTACCCTCTGAGTTCTT GCTGCGGGGACAAGTCCAGCTCCACCGTGACCCTGGGCT GCCTGGTCTCCAGCTACATGCCCGAGCCGGTGACCGTGA CCTGGAACTCGGGTGCCCTGAAGAGCGGCGTGCACACCT TCCCGGCCGTCCTTCAGTCCTCCGGGCTGTACTCTCTCAG CAGCATGGTGACCGTGCCCGGCAGCACCTCAGGACAGAC CTTCACCTGCAACGTAGCCCACCCGGCCAGCAGCACCAA GGTGGACAAGGCTGTTGATCCCACATGCAAACCATCACC CTGTGACTGTTGCCCACCCCCTGAGCTCCCCGGAGGACC CTCTGTCTTCATCTTCCCACCGAAACCCAAGGACACCCTC ACAATCTCGGGAACGCCCGAGGTCACGTGTGTGGTGGTG GACGTGGGCCACGATGACCCCGAGGTGAAGTTCTCCTGG TTCGTGGACGACGTGGAGGTAAACACAGCCACGACGAA GCCGAGAGAGGAGCAGTTCAACAGCACCTACCGCGTGGT CAGCGCCCTGCGCATCCAGCACCAGGACTGGACTGGAGG AAAGGAGTTCAAGTGCAAGGTCCACAACGAAGGCCTCCC GGCCCCCATCGTGAGGACCATCTCCAGGACCAAAGGGCC GGCCCGGGAGCCGCAGGTGTATGTCCTGGCCCCACCCCA GGAAGAGCTCAGCAAAAGCACGGTCAGCCTCACCTGCAT GGTCACCAGCTTCTACCCAGACTACATCGCCGTGGAGTG GCAGAGAAACGGGCAGCCTGAGTCGGAGGACAAGTACG GCACGACCCCGCCCCAGCTGGACGCCGACAGCTCCTACT TCCTGTACAGCAAGCTCAGGGTGGACAGGAACAGCTGGC AGGAAGGAGACACCTACACGTGTGTGGTGATGCACGAG GCCCTGCACAATCACTACACGCAGAAGTCCACCTCTAAG TCTGCGGGTAAATGA (SEQ ID NO: 30) | ASTTAPKVYPLS SCCGDKSSSTVT LGCLVSSYMPEP VTVTWNSGALK SGVHTFPAVLQS SGLYSLSSMVTV PGSTSGQTFTCN VAHPASSTKVD KAVDPTCKPSPC DCCPPPELPGGP SVFIFPPKPKDTL TISGTPEVTCVV VDVGHDDPEVK FSWFVDDVEVN TATTKPREEQFN STYRVVSALRIQ HQDWTGGKEFK CKVHNEGLPAPI VRTISRTKGPAR EPQVYVLAPPQE ELSKSTVSLTCM VTSFYPDYIAVE WQRNGQPESED KYGTTPPQLDA DSSYFLYSKLRV DRNSWQEGDTY TCVVMHEALHN HYTQKSTSKSA GK* (SEQ ID NO: 22) | X16701 (M25278) | | |
| | | IgG1 variant 3 | GCCTCCACCACAGCCCCGAAAGTCTACCCTCTGAGTTCTT GCTGCGGGGACAAGTCCAGCTCCACCGTGACCCTGGGCT GCCTGGTCTCCAGCTACATGCCCGAGCCGGTGACCGTGA CCTGGAACTCGGGTGCCCTGAAGAGCGGCGTGCACACCT TCCCGGCCGTCCTTCAGTCCTCCGGGCTCTACTCTCTCAG CAGCATGGTGACCGTGCCCGGCAGCACCTCAGGAACCCA GACCTTCACCTGCAACGTAGCCCACCCGGCCAGCAGCAC CAAGGTGGACAAGGCTGTTGATCCCAGATGCAAAACAAC CTGTGACTGTTGCCCACCGCCTGAGCTCCCTGGAGGACC CTCTGTCTTCATCTTCCCACCGAAACCCAAGGACACCCTC ACAATCTCGGGAACGCCCGAGGTCACGTGTGTGGTGGTG GACGTGGGCCACGATGACCCCGAGGTGAAGTTCTCCTGG TTCGTGGACGACGTGGAGGTAAACACAGCCACGACGAA GCCGAGAGAGGAGCAGTTCAACAGCACCTACCGCGTGGT CAGCGCCCTGCGCATCCAGCACCAGGACTGGACTGGAGG AAAGGAGTTCAAGTGCAAGGTCCACAACGAAGGCCTCCC AGCCCCCATCGTGAGGACCATCTCCAGGACCAAAGGGCC GGCCCGGGAGCCGCAGGTGTATGTCCTGGCCCCACCCCA GGAAGAGCTCAGCAAAAGCACGGTCAGCCTCACCTGCAT GGTCACCAGCTTCTACCCAGACTACATCGCCGTGGAGTG GCAGAGAAATGGGCAGCCTGAGTCAGAGGACAAGTACG GCACGACCCCTCCCCAGCTGGACGCCGACGGCTCCTACT TCCTGTACAGCAGGCTCAGGGTGGACAGGAACAGCTGGC AGGAAGGAGACACCTACACGTGTGTGGTGATGCACGAG GCCCTGCACAATCACTACACGCAGAAGTCCACCTCTAAG TCTGCGGGTAAATGA (SEQ ID NO: 31) | ASSTTAPKVYPLS SCCGDKSSSTVT LGCLVSSYMPEP VTVTWNSGALK SGVHTFPAVLQS SGLYSLSSMVTV PGSTSGTQTFTC NVAHPASSTKV DKAVDPRCKTT CDCCPPPELPGG PSVFIFPPKPKDT LTISGTPEVTCV VVDVGHDDPEV KFSWFVDDVEV NTATTKPREEQF NSTYRVVSALRI QHQDWTGGKEF KCKVHNEGLPA PIVRTISRTKGPA REPQVYVLAPPQ EELSKSTVSLTC MVTSFYPDYIAV EWQRNGQPESE DKYGTTPPQLD ADGSYFLYSRLR VDRNSWQEGDT YTCVVMHEALH NHYTQKSTSKS AGK* (SEQ ID NO: 23) | S82409 | | |
| | | IgG2 variant 1 | GCCTCCACCACAGCCCCGAAAGTCTACCCTCTGGCATCC AGCTGCGGAGACACATCCAGCTCCACCGTGACCCTGGGC TGCCTGGTGTCCAGCTACATGCCCGAGCCGGTGACCGTG ACCTGGAACTCGGGTGCCCTGAAGAGCGGCGTGCACACC TTCCCGGCTGTCCTTCAGTCCTCCGGGCTCTACTCTCTCA GCAGCATGGTGACCGTGCCCGCCAGCAGCTCAGGACAGA CCTTCACCTGCAACGTAGCCCACCCGGCCAGCAGCACCA AGGTGGACAAGGCTGTTGGGGTCTCCATTGACTGCTCCA AGTGTCATAACCAGCCTTGCGTGAGGGAACCATCTGTCT TCATCTTCCCACCGAAACCCAAAGACACCCTGATGATCA CAGGAACGCCCGAGGTCACGTGTGTGGTGGTGAACGTGG GCCACGATAACCCCGAGGTGCAGTTCTCCTGGTTCGTGG ATGACGTGGAGGTGCACACGGCCAGGTCGAAGCCAAGA GAGGAGCAGTTCAACAGCACGTACCGCGTGGTCAGCGCC CTGCCCATCCAGCACCAGGACTGGACTGGAGGAAAGGA GTTCAAGTGCAAGGTCAACAACAAAGGCCTCTCGGCCCC CATCGTGAGGATCATCTCCAGGAGCAAAGGGCCGGCCCG GGAGCCGCAGGTGTATGTCCTGGACCCACCCAAGGAAGA GCTCAGCAAAAGCACGCTCAGCGTCACCTGCATGGTCAC CGGCTTCTACCCAGAAGATGTAGCCGTGGAGTGGCAGAG AAACCGGCAGACTGAGTCGGAGGACAAGTACCGCACGA CCCCGCCCCAGCTGGACACCGACCGCTCCTACTTCCTGTA CAGCAAGCTCAGGGTGGACAGGAACAGCTGGCAGGAAG GAGACGCCTACACGTGTGTGGTGATGCACGAGGCCCTGC ACAATCACTACATGCAGAAGTCCACCTCTAAGTCTGCGG GTAAATGA (SEQ ID NO: 32) | ASTTAPKVYPLA SSCGDTSSSTVT LGCLVSSYMPEP VTVTWNSGALK SGVHTFPAVLQS SGLYSLSSMVTV PASSSGQTFTCN VAHPASSTKVD KAVGVSIDCSKC HNQPCVREPSVF IFPPKPKDTLMIT GTPEVTCVVVN VGHDNPEVQFS WFVDDVEVHTA RSKPREEQFNST YRVVSALPIQHQ DWTGGKEFKCK VNNKGLSAPIVR IISRSKGPAREPQ VYVLDPPKEELS KSTLSVTCMVT GFYPEDVAVEW QRNRQTESEDK YRTTPPQLDTDR SYFLYSKLRVDR NSWQEGDAYTC VVMHEALHNHY MQKSTSKSAGK * (SEQ ID NO: 24) | S82407 | | |
| | | IgG2 variant 2 | GCCTCCACCACAGCCCCGAAAGTCTACCCTCTGAGTTCTT GCTGCGGGGACAAGTCCAGCTCCACCGTGACCCTGGGCT GCCTGGTGTCCAGCTACATGCCCGAGCCGGTGACCGTGA CCTGGAACTCGGGTGCCCTGAAGAGCGGCGTGCACACCT TCCCGGCCGTCCTTCAGTCCTCCGGGCTCTACTCTCTCAG CAGCATGGTGACCGTGCCCGGCAGCACCTCAGGACAGAC CTTCACCTGCAACGTAGCCCACCCGGCCAGCAGCACCAA GGTGGACAAGGCTGTTGGGGTCTCCAGTGACTGCTCCAA GCCTAATAACCAGCATTGCGTGAGGGAACCATCTGTCTT CATCTTCCCACCGAAACCCAAAGACACCCTGATGATCAC AGGAACGCCCGAGGTCACGTGTGTGGTGGTGAACGTGGG CCACGATAACCCCGAGGTGCAGTTCTCCTGGTTCGTGGA CGACGTGGAGGTGCACACGGCCAGGACGAAGCCGAGAG AGGAGCAGTTCAACAGCACGTACCGCGTGGTCAGCGCCC TGCCCATCCAGCACCAGGACTGGACTGGAGGAAAGGAGT TCAAGTGCAAGGTCAACATCAAAGGCCTCTCGGCCTCCA TCGTGAGGATCATCTCCAGGAGCAAAGGGCCGGCCCGGG AGCCGCAGGTGTATGTCCTGGACCCACCCAAGGAAGAGC TCAGCAAAAGCACGGTCAGCGTCACCTGCATGGTCATCG GCTTCTACCCAGAAGATGTAGACGTGGAGTGGCAGAGAG ACCGGCAGACTGAGTCGGAGGACAAGTACCGCACGACC CCGCCCCAGCTGGACGCCGACCGCTCCTACTTCCTGTACA GCAAGCTCAGGGTGGACAGGAACAGCTGGCAGAGAGGA GACACCTACACGTGTGTGGTGATGCACGAGGCCCTGCAC AATCACTACATGCAGAAGTCCACCTCTAAGTCTGCGGGT AAATGA (SEQ ID NO: 33) | ASTTAPKVYPLS SCCGDKSSSTVT LGCLVSSYMPEP VTVTWNSGALK SGVHTFPAVLQS SGLYSLSSMVTV PGSTSGQTFTCN VAHPASSTKVD KAVGVSSDCSK PNNQHCVREPS VFIFPPKPKDTL MITGTPEVTCVV VNVGHDNPEVQ FSWFVDDVEVH TARTKPREEQFN STYRVVSALPIQ HQDWTGGKEFK CKVNIKGLSASI VRIISRSKGPARE PQVYVLDPPKEE LSKSTVSVTCM VIGFYPEDVDVE WQRDRQTESED KYRTTPPQLDA DRSYFLYSKLRV DRNSWQRGDTY TCVVMHEALHN HYMQKSTSKSA GK* (SEQ ID NO: 25) | M36946 (X06703) | | |
| | | IgG2 variant 3 | GCCTCCACCACAGCCCCGAAAGTCTACCCTCTGAGTTCTT GCTGCGGGGACAAGTCCAGCTCGGGGGTGACCCTGGGCT GCCTGGTCTCCAGCTACATGCCCGAGCCGGTGACCGTGA CCTGGAACTCGGGTGCCCTGAAGAGCGGCGTGCACACCT TCCCGGCCGTCCTTCAGTCCTCCGGGCTCTACTCTCTCAG CAGCATGGTGACCGTGCCCGCCAGCAGCTCAGGAACCCA GACCTTCACCTGCAACGTAGCCCACCCGGCCAGCAGCAC CAAGGTGGACAAGGCTGTTGGGGTCTCCAGTGACTGCTC CAAGCCTAATAACCAGCATTGCGTGAGGGAACCATCTGT CTTCATCTTCCCACCGAAACCCAAAGACACCCTGATGAT CACAGGAACGCCCGAGGTCACGTGTGTGGTGGTGAACGT GGGCCACGATAACCCCGAGGTGCAGTTCTCCTGGTTCGT GGACGACGTGGAGGTGCACACGGCCAGGACGAAGCCGA | ASSTTAPKVYPLS SCCGDKSSSGVT LGCLVSSYMPEP VTVTWNSGALK SGVHTFPAVLQS SGLYSLSSMVTV PASSSGTQTFTC NVAHPASSTKV DKAVGVSSDCS KPNNQHCVREP SVFIFPPKPKDTL MITGTPEVTCVV VNVGHDNPEVQ | X16702 (M25279) | | |
| | | | GAGAGGAGCAGTTCAACAGCACGTACCGCGTGGTCAGCG CCCTGCCCATCCAGCACCAGGACTGGACTGGAGGAAAGG AGTTCAAGTGCAAGGTCAACATCAAAGGCCTCTCGGCCT CCATCGTGAGGATCATCTCCAGGAGCAAAGGGCCGGCCC GGGAGCCGCAGGTGTATGTCCTGGACCCACCCAAGGAAG AGCTCAGCAAAAGCACGGTCAGCCTCACCTGCATGGTCA TCGGCTTCTACCCAGAAGATGTAGACGTGGAGTGGCAGA GAGACCGGCAGACTGAGTCGGAGGACAAGTACCGCACG ACCCCGCCCCAGCTGGACGCCGACCGCTCCTACTTCCTGT ACAGCAAGCTCAGGGTGGACAGGAACAGCTGGCAGAGA GGAGACACCTACACGTGTGTGGTGATGCACGAGGCCCTG CACAATCACTACATGCAGAAGTCCACCTCTAAGTCTGCG GGTAAATGA (SEQ ID NO: 34) | FSWFVDDVEVH TARTKPREEQFN STYRVVSALPIQ HQDWTGGKEFK CKVNIKGLSASI VRIISRSKGPARE PQVYVLDPPKEE LSKSTVSLTCMV IGFYPEDVDVE WQRDRQTESED KYRTTPPQLDA DRSYFLYSKLRV DRNSWQRGDTY TCVVMHEALHN HYMQKSTSKSA GK* (SEQ ID NO: 26) | | | |
| | | IgG3 variant 1 | GCCTCCACCACAGCCCCGAAAGTCTACCCTCTGGCATCC AGCTGCGGAGACACATCCAGCTCCACCGTGACCCTGGGC TGCCTGGTCTCCAGCTACATGCCCGAGCCGGTGACCGTG ACCTGGAACTCGGGTGCCCTGAAGAGCGGCGTGCACACC TTCCCGGCCGTCCGGCAGTCCTCTGGGCTGTACTCTCTCA GCAGCATGGTGACTGTGCCCGCCAGCAGCTCAGAAACCC AGACCTTCACCTGCAACGTAGCCCACCCGGCCAGCAGCA CCAAGGTGGACAAGGCTGTCACTGCAAGGCGTCCAGTCC CGACGACGCCAAAGACAACTATCCCTCCTGGAAAACCCA CAACCCCAAAGTCTGAAGTTGAAAAGACACCCTGCCAGT GTTCCAAATGCCCAGAACCTCTGGGAGGACTGTCTGTCTT CATCTTCCCACCGAAACCCAAGGACACCCTCACAATCTC GGGAACGCCCGAGGTCACGTGTGTGGTGGTGGACGTGGG CCAGGATGACCCCGAGGTGCAGTTCTCCTGGTTCGTGGA CGACGTGGAGGTGCACACGGCCAGGACGAAGCCGAGAG AGGAGCAGTTCAACAGCACCTACCGCGTGGTCAGCGCCC TGCGCATCCAGCACCAGGACTGGCTGCAGGGAAAGGAGT TCAAGTGCAAGGTCAACAACAAAGGCCTCCCGGCCCCCA TTGTGAGGACCATCTCCAGGACCAAAGGGCAGGCCCGGG AGCCGCAGGTGTATGTCCTGGCCCCACCCCGGGAAGAGC TCAGCAAAAGCACGCTCAGCCTCACCTGCCTGATCACCG GTTTCTACCCAGAAGAGATAGACGTGGAGTGGCAGAGAA ATGGGCAGCCTGAGTCGGAGGACAAGTACCACACGACC GCACCCCAGCTGGATGCTGACGGCTCCTACTTCCTGTACA GCAAGCTCAGGGTGAACAAGAGCAGCTGGCAGGAAGGA GACCACTACACGTGTGCAGTGATGCACGAAGCTTTACGG AATCACTACAAAGAGAAGTCCATCTCGAGGTCTCCGGGT AAATGA (SEQ ID NO: 35) | ASTTAPKVYPLA SSCGDTSSSTVT LGCLVSSYMPEP VTVTWNSGALK SGVHTFPAVRQS SGLYSLSSMVTV PASSSETQTFTC NVAHPASSTKV DKAVTARRPVP TTPKTTIPPGKPT TPKSEVEKTPCQ CSKCPEPLGGLS VFIFPPKPKDTLT ISGTPEVTCVVV DVGQDDPEVQF SWFVDDVEVHT ARTKPREEQFNS TYRVVSALRIQH QDWLQGKEFKC KVNNKGLPAPIV RTISRTKGQARE PQVYVLAPPREE LSKSTLSLTCLIT GFYPEEIDVEWQ RNGQPESEDKY HTTAPQLDADG SYFLYSKLRVNK SSWQEGDHYTC AVMHEALRNHY KEKSISRSPGK* (SEQ ID NO: 27) | U63638 | | |
| | | IgG3 variant 2 | GCCTCCACCACAGCCCCGAAAGTCTACCCTCTGGCATCC CGCTGCGGAGACACATCCAGCTCCACCGTGACCCTGGGC TGCCTGGTCTCCAGCTACATGCCCGAGCCGGTGACCGTG ACCTGGAACTCGGGTGCCCTGAAGAGTGGCGTGCACACC TTCCCGGCCGTCCTTCAGTCCTCCGGGCTGTACTCTCTCA GCAGCATGGTGACCGTGCCCGCCAGCACCTCAGAAACCC AGACCTTCACCTGCAACGTAGCCCACCCGGCCAGCAGCA CCAAGGTGGACAAGGCTGTCACTGCAAGGCGTCCAGTCC CGACGACGCCAAAGACAACCATCCCTCCTGGAAAACCCA CAACCCAGGAGTCTGAAGTTGAAAAGACACCCTGCCAGT GTTCCAAATGCCCAGAACCTCTGGGAGGACTGTCTGTCTT | ASTTAPKVYPLA SRCGDTSSSTVT LGCLVSSYMPEP VTVTWNSGALK SGVHTFPAVLQS SGLYSLSSMVTV PASTSETQTFTC NVAHPASSTKV DKAVTARRPVP TTPKTTIPPGKPT TQESEVEKTPCQ | U63639 | | |
| | | | CATCTTCCCACCGAAACCCAAGGACACCCTCACAATCTC GGGAACGCCCGAGGTCACGTGTGTGGTGGTGGACGTGGG CCAGGATGACCCCGAGGTGCAGTTCTCCTGGTTCGTGGA CGACGTGGAGGTGCACACGGCCAGGACGAAGCCGAGAG AGGAGCAGTTCAACAGCACCTACCGCGTGGTCAGCGCCC TGCGCATCCAGCACCAGGACTGGCTGCAGGGAAAGGAGT TCAAGTGCAAGGTCAACAACAAAGGCCTCCCGGCCCCCA TTGTGAGGACCATCTCCAGGACCAAAGGGCAGGCCCGGG AGCCGCAGGTGTATGTCCTGGCCCCACCCCGGGAAGAGC TCAGCAAAAGCACGCTCAGCCTCACCTGCCTGATCACCG GTTTCTACCCAGAAGAGATAGACGTGGAGTGGCAGAGAA ATGGGCAGCCTGAGTCGGAGGACAAGTACCACACGACC GCACCCCAGCTGGATGCTGACGGCTCCTACTTCCTGTACA GCAGGCTCAGGGTGAACAAGAGCAGCTGGCAGGAAGGA GACCACTACACGTGTGCAGTGATGCATGAAGCTTTACGG AATCACTACAAAGAGAAGTCCATCTCGAGGTCTCCGGGT AAATGA (SEQ ID NO: 36) | CSKCPEPLGGLS VFIFPPKPKDTLT ISGTPEVTCVVV DVGQDDPEVQF SWFVDDVEVHT ARTKPREEQFNS TYRVVSALRIQH QDWLQGKEFKC KVNNKGLPAPIV RTISRTKGQARE PQVYVLAPPREE LSKSTLSLTCLIT GFYPEEIDVEWQ RNGQPESEDKY HTTAPQLDADG SYFLYSRLRVNK SSWQEGDHYTC AVMHEALRNHY KEKSISRSPGK* (SEQ ID NO: 28) | | | |
| | Bovine Ig light chain constant region (CL) | Ig lambda | CAGCCCAAGTCCCCACCCTCGGTCACCCTGTTCCCGCCCT CCACGGAGGAGCTCAACGGCAACAAGGCCACCCTGGTGT GTCTCATCAGCGACTTCTACCCGGGTAGCGTGACCGTGG TCTGGAAGGCAGACGGCAGCACCATCACCCGCAACGTGG AGACCACCCGGGCCTCCAAACAGAGCAACAGCAAGTAC GCGGCCAGCAGCTACCTGAGCCTGACGAGCAGCGACTGG AAATCGAAAGGCAGTTACAGCTGCGAGGTCACGCACGA GGGGAGCACCGTGACGAAGACAGTGAAGCCCTCAGAGT GTTCTTAG (SEQ ID NO: 7) | QPKSPPSVTLFPP STEELNGNKATL VCLISDFYPGSV TVVWKADGSTI TRNVETTRASK QSNSKYAASSYL SLTSSDWKSKGS YSCEVTHEGSTV TKTVKPSECS* (SEQ ID NO: 3) | X62917 | Not registered | Chen L. et al., Vet. Immunol. Immunopathol., 124, 284-294 (2008). PMID: 18538861 |

Amino acid sequences and nucleotide sequences of ovine, water buffalo and human CL and CH are summarized in the table below.

**(Table)**

| Species | Ig Domain | | Nucleotide Sequence | Amino Acid Sequence | GenBank Accession No. | IMGT Database | Reference |
|---|---|---|---|---|---|---|---|
| Ovine (Scientific Name: Ovis aries) | Ovine heavy chain constant region (CH1∼C H3) | IgG1 | GCCTCAACAACACCCCCGAAAGTCTACCCTCTGA CTTCTTGCTGCGGGGACACGTCCAGCTCCATCGTG ACCCTGGGCTGCCTGGTCTCCAGCTATATGCCCGA GCCGGTGACCGTGACCTGGAACTCTGGTGCCCTG ACCAGCGGCGTGCACACCTTCCCGGCCATCCTGC AGTCCTCCGGGCTCTACTCTCTCAGCAGCGTGGTG ACCGTGCCGGCCAGCACCTCAGGAGCCCAGACCT TCATCTGCAACGTAGCCCACCCGGCCAGCAGCAC CAAGGTGGACAAGCGTGTTGAGCCCGGATGCCCG GACCCATGCAAACATTGCCGATGCCCACCCCCTG AGCTCCCCGGAGGACCGTCTGTCTTCATCTTCCCA CCGAAACCCAAGGACACCCTTACAATCTCTGGAA CGCCCGAGGTCACGTGTGTGGTGGTGGACGTGGG CCAGGATGACCCCGAGGTGCAGTTCTCCTGGTTC GTGGACAACGTGGAGGTGCGCACGGCCAGGACA AAGCCGAGAGAGGAGCAGTTCAACAGCACCTTCC GCGTGGTCAGCGCCCTGCCCATCCAGCACCAAGA CTGGACTGGAGGAAAGGAGTTCAAGTGCAAGGTC CACAACGAAGCCCTCCCGGCCCCCATCGTGAGGA CCATCTCCAGGACCAAAGGGCAGGCCCGGGAGCC GCAGGTGTACGTCCTGGCCCCACCCCAGGAAGAG CTCAGCAAAAGCACGCTCAGCGTCACCTGCCTGG TCACCGGCTTCTACCCAGACTACATCGCCGTGGA GTGGCAGAAAAATGGGCAGCCTGAGTCGGAGGA CAAGTACGGCACGACCACATCCCAGCTGGACGCC GACGGCTCCTACTTCCTGTACAGCAGGCTCAGGG TGGACAAGAACAGCTGGCAAGAAGGAGACACCT ACGCGTGTGTGGTGATGCACGAGGCTCTGCACAA CCACTACACACAGAAGTCGATCTCTAAGCCTCCG GGTAAATGA (SEQ ID NO: 38) | ASTTPPKVYPLTSCCGDTSSSIVTLGCLVS SYMPEPVTVTWNSGALTSGVHTFPAILQ SSGLYSLSSVVTVPASTSGAQTFICNVAH PASSTKVDKRVEPGCPDPCKHCRCPPPEL PGGPSVFIFPPKPKDTLTISGTPEVTCVVV DVGQDDPEVQFSWFVDNVEVRTARTKP REEQFNSTFRVVSALPIQHQDWTGGKEF KCKVHNEALPAPIVRTISRTKGQAREPQV YVLAPPQEELSKSTLSVTCLVTGFYPDYI AVEWQKNGQPESEDKYGTTTSQLDADG SYFLYSRLRVDKNSWQEGDTYACVVMH EALHNHYTQKSISKPPGK* (SEQ ID NO: 37) | X69797 | http://www.imgt.org/IMGTrepertoire/index.php?section=LocusGenes&repertoire=genetable&species=sheep&aroup=IGHC | Dufour V. et al., J. Immunol., 156, 2163-2170 (1996). PMID: 8690905 |
| | | IgG2 | GCCTCCACCACAGCCCCGAAAGTCTACCCTCTGA CTTCTTGCTGCGGGGACACGTCCAGCTCCAGCTCC ATCGTGACCCTGGGCTGCCTGGTCTCCAGCTATAT GCCCGAGCCGGTGACCGTGACCTGGAACTCTGGT GCCCTGACCAGCGGCGTGCACACCTTCCCGGCCA TCCTGCAGTCCTCCGGGCTCTACTCTCTCAGCAGC GTGGTGACCGTGCCGGCCAGCACCTCAGGAGCCC AGACCTTCATCTGCAACGTAGCCCACCCGGCCAG CAGCGCCAAGGTGGACAAGCGTGTTGGGATCTCC AGTGACTACTCCAAGTGTTCTAAACCGCCTTGCGT GAGCCGACCGTCTGTCTTCATCTTCCCCCCGAAAC CCAAGGACAGCCTCATGATCACAGGAACGCCCGA GGTCACGTGTGTGGTGGTGGACGTGGGCCAGGGT GACCCCGAGGTGCAGTTCTCCTGGTTCGTGGACA ACGTGGAGGTGCGCACGGCCAGGACAAAGCCGA GAGAGGAGCAGTTCAACAGCACCTTCCGCGTGGT CAGCGCCCTGCCCATCCAGCACGACCACTGGACT GGAGGAAAGGAGTTCAAGTGCAAGGTCCACAGC AAAGGCCTCCCGGCCCCCATCGTGAGGACCATCT CCAGGGCCAAAGGGCAGGCCCGGGAGCCGCAGG TGTACGTCCTGGCCCCACCCCAGGAAGAGCTCAG CAAAAGCACGCTCAGCGTCACCTGCCTGGTCACC GGCTTCTACCCAGACTACATCGCCGTGGAGTGGC AGAGAGCGCGGCAGCCTGAGTCGGAGGACAAGT ACGGCACGACCACATCCCAGCTGGACGCCGACGG CTCCTACTTCCTGTACAGCAGGCTCAGGGTGGAC AAGAGCAGCTGGCAAAGAGGAGACACCTACGCG TGTGTGGTGATGCACGAGGCTCTGCACAACCACT ACACACAGAAGTCGATCTCTAAGCCTCCGGGTAA ATGA (SEQ ID NO: 40) | ASTTAPKVYPLTSCCGDTSSSSSIVTLGCL VSSYMPEPVTVTWNSGALTSGVHTFPAI LQSSGLYSLSSVVTVPASTSGAQTFICNV AHPASSAKVDKRVGISSDYSKCSKPPCVS RPSVFIFPPKPKDSLMITGTPEVTCVVVD VGQGDPEVQFSWFVDNVEVRTARTKPR EEQFNSTFRVVSALPIQHDHWTGGKEFK CKVHSKGLPAPIVRTISRAKGQAREPQV YVLAPPQEELSKSTLSVTCLVTGFYPDYI AVEWQRARQPESEDKYGTTTSQLDADG SYFLYSRLRVDKSSWQRGDTYACVVMH EALHNHYTQKSISKPPGK* (SEQ ID NO: 39) | X70983 | | Clarkson C.A. et al., Mol. Immunol., 30, 1195-1204 (1993). PMID: 8413324 |
| | Ovine light chain constant region | Ig kappa (CK) | CCATCCGTCTTCCTCTTCAAACCATCTGAGGAACA GCTGAGGACCGGAACTGTCTCTGTCGTGTGCTTGG TGAATGATTTCTACCCCAAAGATATCAATGTCAA GGTGAAAGTGGATGGGGTTACCCAGAACAGCAAC TTCCAGAACAGCTTCACAGACCAGGACAGCAAGA AAAGCACCTACAGCCTCAGCAGCACCCTGACACT GTCCAGCTCAGAGTACCAGAGCCATAACGCCTAT GCGTGTGAGGTCAGCCACAAGAGCCTGCCCACCG CCCTCGTCAAGAGCTTCAATAAGAATGAATGTTA G (SEQ ID NO: 42) | PSVFLFKPSEEQLRTGTVSVVCLVNDFYP KDINVKVKVDGVTQNSNFQNSFTDQDS KKSTYSLSSTLTLSSSEYQSHNAYACEVS HKSLPTALVKSFNKNEC* (SEQ ID NO: 41) | X54110 | Not registered | Jenne C.N. et al., Dev. Comp. Immunol. 30 (1-2), 165-174 (2006). PMID: 16083958 |
| | | Ig lambda (CL) | GGTCAGCCCAAGTCCGCACCCTCGGTCACCCTGTT CCCGCCTTCCACGGAGGAGCTCAGTACCAACAAG GCCACCGTGGTGTGTCTCATCAACGACTTCTACCC GGGTAGCGTGAACGTGGTCTGGAAGGCAGATGGC AGCACCATCAATCAGAACGTGAAGACCACCCAGG CCTCCAAACAGAGCAACAGCAAGTACGCGGCCAG CAGCTACCTGACCCTGACGGGCAGCGAGTGGAAG TCTAAGAGCAGTTACACCTGCGAGGTCACGCACG AGGGGAGCACCGTGACGAAGACAGTGAAGCCCTC AGAGTGTTCTTAG (SEQ ID NO: 44) | GQPKSAPSVTLFPPSTEELSTNKATVVCLI NDFYPGSVNVVWKADGSTINQNVKTTQ ASKQSNSKYAASSYLTLTGSEWKSKSSY TCEVTHEGSTVTKTVKPSECS* (SEQ ID NO: 43) | AY734681 | | |
| Water buffalo (Scientific Name: Bubalus bubalis) | Water buffalo Ig heavy chain constant region (CH1∼C H3) | IgG1? | GAGCGGCGTGCACACCTTCCCGGCCGTCCTTCA GTCCTCCGGGCTCTACTCTCTCAGCAGCACGGT GACCGCGCCCGCCAGCGCCACAAAAAGCCAGA CCTTCACCTGCAACGTAGCCCACCCGGCCAGCA GCACCAAGGTGGACAAGGCTGTTGTTCCCCCAT GCAGACCGAAACCCTGTGATTGCTGCCCACCCC CTGAGCTCCCCGGAGGACCCTCTGTCTTCATCTT CCCACCAAAACCCAAGGACACCCTCACAATCTC TGGAACTCCTGAGGTCACGTGTGTGGTGGTGGA CGTGGGCCACGATGACCCCGAGGTGAAGTTCTC CTGGTTCGTGGACGATGTGGAGGTAAACACAGC CAGGACGAAGCCAAGAGAGGAGCAGTTCAACA GCACCTACCGCGTGGTCAGCGCCCTGCCCATCC AGCACAACGACTGGACTGGAGGAAAGGAGTTC AAGTGCAAGGTCTACAATGAAGGCCTCCCAGCC CCCATCGTGAGGACCATCTCCAGGACCAAAGGG CAGGCCCGGGAGCCGCAGGTGTACGTCCTGGCC CCACCCCAGGACGAGCTCAGCAAAAGCACGGTC AGCATCACTTGCATGGTCACTGGCTTCTACCCA GACTACATCGCCGTAGAGTGGCAGAAAGATGG GCAGCCTGAGTCAGAGGACAAATATGGCACGA CCCCGCCCCAGCTGGACAGCGATGGCTCCTACT TCCTGTACAGCAGGCTCAGGGTGAACAAGAACA GCTGGCAAGAAGGAGGCGCCTACACGTGTGTAG TGATGCATGAGGC (SEQ ID NO: 46) | SGVHTFPAVLQSSGLYSLSSTVTAP ASATKSQTFTCNVAHPASSTKVDK AVVPPCRPKPCDCCPPPELPGGPSV FIFPPKPKDTLTISGTPEVTCVVVDV GHDDPEVKFSWFVDDVEVNTART KPREEQFNSTYRVVSALPIQHNDW TGGKEFKCKVYNEGLPAPIVRTISR TKGQAREPQVYVLAPPQDELSKST VSITCMVTGFYPDYIAVEWQKDGQ PESEDKYGTTPPQLDSDGSYFLYSR LRVNKNSWQEGGAYTCVVMHE (SEQ ID NO: 45) | NW_ 005690903 | Not registered | None |
| | | IgG2? | GCCTCCATCACAGCCCCGAAAGTCTACCCTCTG ACTTCTTGCCGCGGGGAAACGTCCAGCTCCACC GTGACCCTGGGCTGCCTGGTCTCCAGCTACATG CCCGAGCCGGTGACCGTGACCTGGAACTCGGGT GCCCTGAAGAGCGGCGTGCACACCTTCCCGGCC GTCCTTCAGTCCTCTGGGCTCTACTCTCTCAGCA GCACGGTGACCGCGCCCGCCAGCGCCACAAAA | ASITAPKVYPLTSCRGETSSSTVTLG CLVSSYMPEPVTVTWNSGALKSGV HTFPAVLQSSGLYSLSSTVTAPASA TKSQTFTCNVAHPASSTKVDTAVG FSSDCCKFPKPCVRGPSVFIFPPKPK DTLMITGNPEVTCVVVDVGRDNPE VQFSWFVGDVEVHTGRSKPREEQF | NW_ 005766143 | | |
| | | | AGCCAGACCTTCACCTGCAACGTAGCCCACCCG GCCAGCAGCACCAAGGTGGACACGGCTGTTGGG TTCTCCAGTGACTGCTGCAAGTTTCCTAAGCCTT GTGTGAGGGGACCATCTGTCTTCATCTTCCCGCC GAAACCCAAAGACACCCTGATGATCACAGGAA ATCCCGAGGTCACATGTGTGGTGGTGGACGTGG GCCGGGATAACCCCGAGGTGCAGTTCTCCTGGT TCGTGGGTGATGTGGAGGTGCACACGGGCAGGT CGAAGCCGAGAGAGGAGCAGTTCAACAGCACC TACCGCGTGGTCAGCACCCTGCCCATCCAGCAC AATGACTGGACTGGAGGAAAGGAGTTCAAGTG CAAGGTCAACAACAAAGGCCTCCCAGCCCCCAT CGTGAGGACCATCTCCAGGACCAAAGGGCAGG CCCGGGAGCCGCAGGTGTACGTCCTGGCCCCAC CCCAGGAAGAGCTCAGCAAAAGCACGGTCAGC GTCACTTGCATGGTCACTGGCTTCTACCCAGACT ACATCGCCGTAGAGTGGCATAGAGACCGGCAG GCTGAGTCGGAGGACAAGTACCGCACGACCCCG CCCCAGCTGGACAGCGATGGCTCCTACTTCCTG TACAGCAGGCTCAAGGTGAACAAGAACAGCTG GCAAGAAGGAGGCGCCTACACGTGTGTAGTGAT GCATGAGGC (SEQ ID NO: 48) | NSTYRVVSTLPIQHNDWTGGKEFK CKVNNKGLPAPIVRTISRTKGQARE PQVYVLAPPQEELSKSTVSVTCMV TGFYPDYIAVEWHRDRQAESEDKY RTTPPQLDSDGSYFLYSRLKVNKNS WQEGGAYTCVVMHE (SEQ ID NO: 47) | | | |
| | | IgG3? | GCCTCCACCACAGCCCCGAAAGTCTACCCTCTG GCATCCAGCTGCGGGGACACGTCCAGCTCCACC GTGACCCTGGGCTGCCTGGTCTCCAGCTACATG CCCGAGCCGGTGACCGTGACCTGGAACTCGGGT GCCCTGAAGAACGGCGTGCACACCTTCCCGGCC GTCCGGCAGTCCTCCGGGCTCTACTCTCTCAGCA GCATGGTGACCATGCCCACCAGCACCGCAGGAA CCCAGACCTTCACCTGCAACGTAGCCCACCCGG CCAGCAGCACCAAGGTGGACACGGCTGTCACTG CAAGGCATCCGGTCCCGAAGACACCAGAGACA CCTATCCATCCTGTAAAACCCCCAACCCAGGAG CCCAGAGATGAAAAGACACCCTGCCAGTGTCCC AAATGCCCAGAACCTCTGGGAGGACTGTCTGTC TTCATCTTCCCACCGAAACCCAAGGACACCCTC ACAATCTCTGGAACGCCCGAGGTCACGTGTGTG GTGGTGGACGTGGGCCAGGATGACCCCGAAGTG CAGTTCTCCTGGTTCGTGGATGACGTGGAGGTG CACACAGCCAGGATGAAGCCAAGAGAGGAGCA GTTCAACAGCACCTACCGCGTGGTCAGCGCCCT GCCCATCCAGCACCAGGACTGGCTGCGGGAAAA GGAGTTCAAGTGCAAGGTCAACAACAAAGGCCT CCCGGCCCCCATCGTGAGGACCATCTCCAGGAC CAAAGGGCAGGCCCGGGAGCCACAGGTGTATG TCCTGGCCCCACCCCGGGAAGAGCTCAGCAAAA GCACGCTCAGCCTCACCTGCCTAATCACCGGCT TCTACCCAGAAGAGGTAGACGTGGAGTGGCAG AGAAATGGGCAGCCTGAGTCAGAGGACAAGTA CCACACGACCCCACCCCAGCTGGACGCTGACGG CTCCTACTTCCTGTACAGCAGGCTCAGGGTGAA CAGGAGCAGCTGGCAGGAAGGAGACCACTACA CGTGTGCAGTGATGCATGAAGCTTTACGGAATC ACTACAAAGAGAAGCCCATCTCGAGGTCTCCGG GTAAATGA (SEQ ID NO: 50) | ASTTAPKVYPLASSCGDTSSSTVTL GCLVSSYMPEPVTVTWNSGALKN GVHTFPAVRQSSGLYSLSSMVTMP TSTAGTQTFTCNVAHPASSTKVDT AVTARHPVPKTPETPIHPVKPPTQE PRDEKTPCQCPKCPEPLGGLSVFIFP PKPKDTLTISGTPEVTCVVVDVGQ DDPEVQFSWFVDDVEVHTARMKP REEQFNSTYRVVSALPIQHQDWLR EKEFKCKVNNKGLPAPIVRTISRTK GQAREPQVYVLAPPREELSKSTLSL TCLITGFYPEEVDVEWQRNGQPESE DKYHTTPPQLDADGSYFLYSRLRV NRSSWQEGDHYTCAVMHEALRNH YKEKPISRSPGK* (SEQ ID NO: 49) | NW_005784206 | | |
| | Water buffalo Ig light chain constant region (CL) | Ig lambda? | CAGCCCAAGTCCGCACCCTCAGTCACCCTGTTC CCACCCTCCACGGAGGAGCTCAGCGCCAACAAG GCCACCCTGGTGTGTCTCATCAGCGACTTCTACC CGGGTAGCATGACCGTGGCCAGGAAGGCAGAC GGCAGCACCATCACCCGGAACGTGGAGACCACC CGGGCCTCCAAACAGAGCAACAGCAAGTACGC GGCCAGCAGCTACCTGAGCCTGACGGGCAGCGA GTGGAAATCGAAAGGCAGTTACAGCTGCGAGGT CACGCACGAGGGGAGCACCGTGACAAAGACAG TGAAGCCCTCAGAGTGTTCTTAG (SEQ ID NO: 52) | QPKSAPSVTLFPPSTEELSANKATL VCLISDFYPGSMTVARKADGSTITR NVETTRASKQSNSKYAASSYLSLT GSEWKSKGSYSCEVTHEGSTVTKT VKPSECS* (SEQ ID NO: 51) | NW_ 005690786 | Not registered | None |
| Human (Scientific Name: Homo sapiens) | Human Ig heavy chain constant region (CH1∼C H3) | IgG4 variant 1 | GAGTCCAAATATGGTCCCCCATGCCCAT CATGCCCAGCACCTGAGTTCCTGGGGGG ACCATCAGTCTTCCTGTTCCCCCCAAAAC CCAAGGACACTCTCATGATCTCCCGGAC CCCTGAGGTCACGTGCGTGGTGGTGGAC GTGAGCCAGGAAGACCCCGAGGTCCAGT TCAACTGGTACGTGGATGGCGTGGAGGT GCATAATGCCAAGACAAAGCCGCGGGA GGAGCAGTTCAACAGCACGTACCGTGTG GTCAGCGTCCTCACCGTCCTGCACCAGG ACTGGCTGAACGGCAAGGAGTACAAGTG CAAGGTCTCCAACAAAGGCCTCCCGTCC TCCATCGAGAAAACCATCTCCAAAGCCA AAGGGCAGCCCCGAGAGCCACAGGTGTA CACCCTGCCCCCATCCCAGGAGGAGATG ACCAAGAACCAGGTCAGCCTGACCTGCC TGGTCAAAGGCTTCTACCCCAGCGACAT CGCCGTGGAGTGGGAGAGCAATGGGCA GCCGGAGAACAACTACAAGACCACGCCT CCCGTGCTGGACTCCGACGGCTCCTTCTT CCTCTACAGCAGGCTAACCGTGGACAAG AGCAGGTGGCAGGAGGGGAATGTCTTCT CATGCTCCGTGATGCATGAGGCTCTGCA CAACCACTACACACAGAAGAGCCTCTCC CTGTCTCTGGGTAAATGA (SEQ ID NO: 54) | ESKYGPPCPSCPAPEFLGGPSVFLFPP KPKDTLMISRTPEVTCVVVDVSQEDP EVQFNWYVDGVEVHNAKTKPREEQ FNSTYRVVSVLTVLHQDWLNGKEYK CKVSNKGLPSSIEKTISKAKGQPREPQ VYTLPPSQEEMTKNQVSLTCLVKGF YPSDIAVEWESNGQPENNYKTTPPVL DSDGSFFLYSRLTVDKSRWQEGNVF SCSVMHEALHNHYTQKSLSLSLGK* (SEQ ID NO: 53) | K01316 | http://www.imgt.org/IMGTrepertoire/index.php?sectioir LocusGenes&repertoire=aenetable&species- hiiman&group =IGHC | Ellison J. et al., DNA, 1, 11-18 (1981). PMID: 6299662 |
| | | IgG4 variant 2 | GAGTCCAAATATGGTCCCCCGTGCCCAT CATGCCCAGCACCTGAGTTCCTGGGGGG ACCATCAGTCTTCCTGTTCCCCCCAAAAC CCAAGGACACTCTCATGATCTCCCGGAC CCCTGAGGTCACGTGCGTGGTGGTGGAC GTGAGCCAGGAAGACCCCGAGGTCCAGT TCAACTGGTACGTGGATGGCGTGGAGGT GCATAATGCCAAGACAAAGCCGCGGGA GGAGCAGTTCAACAGCACGTACCGTGTG GTCAGCGTCCTCACCGTCGTGCACCAGG ACTGGCTGAACGGCAAGGAGTACAAGTG CAAGGTCTCCAACAAAGGCCTCCCGTCC TCCATCGAGAAAACCATCTCCAAAGCCA AAGGGCAGCCCCGAGAGCCACAGGTGTA CACCCTGCCCCCATCCCAGGAGGAGATG ACCAAGAACCAGGTCAGCCTGACCTGCC TGGTCAAAGGCTTCTACCCCAGCGACAT CGCCGTGGAGTGGGAGAGCAATGGGCA GCCGGAGAACAACTACAAGACCACGCCT CCCGTGCTGGACTCCGACGGCTCCTTCTT CCTCTACAGCAGGCTAACCGTGGACAAG AGCAGGTGGCAGGAGGGGAATGTCTTCT CATGCTCCGTGATGCATGAGGCTCTGCA CAACCACTACACGCAGAAGAGCCTCTCC CTGTCTCTGGGTAAATGA (SEQ ID NO: 56) | ESKYGPPCPSCPAPEFLGGPSVFLFPP KPKDTLMISRTPEVTCVVVDVSQEDP EVQFNWYVDGVEVHNAKTKPREEQ FNSTYRVVSVLTVVHQDWLNGKEY KCKVSNKGLPSSIEKTISKAKGQPREP QVYTLPPSQEEMTKNQVSLTCLVKG FYPSDIAVEWESNGQPENNYKTTPPV LDSDGSFFLYSRLTVDKSRWQEGNV FSCSVMHEALHNHYTQKSLSLSLGK* （SEQ ID NO: 55） | AJ001563 | | Brusco A. et al., Eur. J. Immunogenet., 25, 349-355 (1998). PMID: 9805657 |
| | | IgG4 variant 3 | GCACCTGAGTTCCTGGGGGGACCATCAG TCTTCCTGTTCCCCCCAAAACCCAAGGAC ACTCTCATGATCTCCCGGACCCCTGAGGT CACGTGCGTGGTGGTGGACGTGAGCCAG GAAGACCCCGAGGTCCAGTTCAACTGGT ACGTGGATGGCGTGGAGGTGCATAATGC CAAGACAAAGCCGCGGGAGGAGCAGTT CAACAGCACGTACCGTGTGGTCAGCGTC CTCACCGTCCTGCACCAGGACTGGCTGA ACGGCAAGGAGTACAAGTGCAAGGTCTC CAACAAAGGCCTCCCGTCCTCCATCGAG AAAACCATCTCCAAAGCCAAAGGGCAGC CCCGAGAGCCACAGGTGTACACCCTGCC CCCATCCCAGGAGGAGATGACCAAGAAC CAGGTCAGCCTGACCTGCCTGGTCAAAG GCTTCTACCCCAGCGACATCGCCGTGGA GTGGGAGAGCAATGGGCAGCCGGAGAA CAACTACAAGACCACGCCTCCCGTGCTG GACTCCGACGGCTCCTTCTTCCTCTACAG CAAGCTCACCGTGGACAAGAGCAGGTGG CAGGAGGGGAACGTCTTCTCATGCTCCG TGATGCATGAGGCTCTGCACAACCACTA CACGCAGAAGAGCCTCTCCCTGTCTCTG GGTAAATGA (SEQ ID NO: 58) | APEFLGGPSVFLFPPKPKDTLMISRTP EVTCVVVDVSQEDPEVQFNWYVDG VEVHNAKTKPREEQFNSTYRVVSVL TVLHQDWLNGKEYKCKVSNKGLPSS IEKTISKAKGQPREPQVYTLPPSQEEM TKNQVSLTCLVKGFYPSDIAVEWESN GQPENNYKTTPPVLDSDGSFFLYSKL TVDKSRWQEGNVFSCSVMHEALHN HYTQKSLSLSLGK* (SEQ ID NO: 57) | AJ001564 | | |
| | Human Ig light chain constant region | Ig kappa (CK) | ACTGTGGCTGCACCATCTGTCTTCATCTT CCCGCCATCTGATGAGCAGTTGAAATCT GGAACTGCCTCTGTTGTGTGCCTGCTGAA TAACTTCTATCCCAGAGAGGCCAAAGTA CAGTGGAAGGTGGATAACGCCCTCCAAT CGGGTAACTCCCAGGAGAGTGTCACAGA GCAGGACAGCAAGGACAGCACCTACAG CCTCAGCAGCACCCTGACGCTGAGCAAA GCAGACTACGAGAAACACAAAGTCTACG CCTGCGAAGTCACCCATCAGGGCCTGAG CTCGCCCGTCACAAAGAGCTTCAACAGG GGAGAGTGTTAG (SEQ ID NO: 60) | TVAAPSVFIFPPSDEQLKSGTASVVCL LNNFYPREAKVQWKVDNALQSGNS QESVTEQDSKDSTYSLSSTLTLSKAD YEKHKVYACEVTHQGLSSPVTKSFN RGEC* (SEQ ID NO: 59) | X96754 | http://www.imgt.org/IMGTrepertoire/index.php?section-LocusGenes&repertoire-genetable&species-human&group=IGKC | None |

The amino acid sequences as shown in SEQ ID NOS: 3, 21-28, 37, 39, 41, 43, 45, 47, 49, 51, 53, 55, 57 and 59 may have deletion(s), substitution(s) or addition(s) of one or several (e.g., up to five, about 10 at the most) amino acids. Even when such mutations have been introduced, the resulting amino acid sequences are capable of having the function as the constant region of Ig heavy chain or light chain.

Although the constant region of wild-type human IgG1 has ADCC activity and CDC activity, it is known that these activities can be reduced by introducing amino acid substitutions and deletions into specific sites. In the case of animals other than human where the constant region of an immunoglobulin equivalent to human IgG4 has not been identified, mutations may be introduced into the relevant region of an immunoglobulin equivalent to human IgG1 so that the resultant constant region with reduced ADCC activity and CDC activity can be used.

The present invention provides an artificial genetic DNA comprising (a') a DNA encoding a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat and (b') a DNA encoding a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat. The present invention also provides a DNA encoding a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat. Further, the present invention also provides a DNA encoding a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.

For (a) a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat; and (b) a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat, reference should be had to the foregoing description. The DNA of (a') is a DNA (gene) encoding the light chain of (a); and the DNA of (b') is a DNA (gene) encoding the heavy chain of (b). An artificial genetic DNA comprising the DNA of (a') and the DNA of ('b) may be synthesized on commercial synthesizer. Restriction enzyme recognition sites, KOZAK sequences, poly-A addition signal sequences, promoter sequences, intron sequences or the like may be added to the artificial genetic DNA.

The present invention also provides a vector comprising the above-mentioned artificial genetic DNA.

As the vector, *Escherichia* coli-derived plasmids (e.g., pBR322, pBR325, pUC12 or pUC13); *Bacillus subtilis*-derived plasmids (e.g., pUB110, pTP5 or pC194), yeast-derived plasmids (e.g., pSH19 or pSH15); bacteriophages such as λ phage; animal viruses such as retrovirus or vaccinia virus; or insect pathogen viruses such as baculovirus may be used. In the Examples described later, pDN112 (Marzi A, Yoshida R, Miyamoto H, Ishijima M, Suzuki Y, Higuchi M, Matsuyama Y, Igarashi M, Nakayama E, Kuroda M, Saijo M, Feldmann F, Brining D, Feldmann H, Takada A. PLoS One, 7:e36192, Apr. 27, 2012; Ikebuchi R, Konnai S, Okagawa T, Yokoyama K, Nakajima C, Suzuki Y, Murata S, Ohashi K. Immunology, 142(4):551-561, Aug. 2014) was used.

The vector may also comprise promoters, enhancers, splicing signals, poly-A addition signals, intron sequences, selection markers, SV40 replication origins, and so forth.

The present invention also provides a host cell transformed by the above vector. It is possible to prepare the anti-PD-1 antibody of the invention by culturing the host cell and collecting the antibody of interest from the resultant culture. Therefore, the present invention also provides a method of preparing an antibody, comprising culturing the above-described host cell and collecting the anti-PD-1 antibody of the invention from the culture. In the method of the present invention for preparing an antibody, a vector incorporating an artificial genetic DNA comprising a DNA encoding the light chain and a DNA encoding the heavy chain may be transfected into a host cell. Alternatively, a vector incorporating a DNA encoding the light chain and a vector incorporating a DNA encoding the heavy chain may be co-transfected into a host cell.

Examples of the host cell include, but are not limited to, bacterial cells (such as *Escherichia* bacteria, *Bacillus* bacteria or *Bacillus subtilis*), fungal cells (such as yeast or Aspergillus), insect cells (such as S2 cells or Sf cells), animal cells (such as CHO cells, COS cells, HeLa cells, C127 cells, 3T3 cells, BHK cells or HEK 293 cells) and plant cells. Among these, CHO-DG44 cell (CHO-DG44(dfhr⁻/⁻)) which is a dihydrofolate reductase deficient cell is preferable.

Introduction of a recombinant vector into a host cell may be performed by the methods disclosed in Molecular Cloning 2nd Edition, J. Sambrook et al., Cold Spring Harbor Lab. Press, 1989 (e.g., the calcium phosphate method, the DEAE-dextran method, transfection, microinjection, lipofection, electroporation, transduction, scrape loading, the shotgun method, etc.) or by infection.

The resultant transformant may be cultured in a medium, followed by collection of the anti-PD-1 antibody of the present invention from the culture. When the antibody is secreted into the medium, the medium may be recovered, followed by isolation and purification of the antibody from the medium. When the antibody is produced within the transformed cells, the cells may be lysed, followed by isolation and purification of the antibody from the cell lysate.

Examples of the medium include, but are not limited to, OptiCHO medium, Dynamis medium, CD CHO medium, ActiCHO medium, FortiCHO medium, Ex-Cell CD CHO medium, BalanCD CHO medium, ProCHO 5 medium and Cellvento CHO-100 medium.

The pH of the medium varies depending on the cell to be cultured. Generally, a pH range from 6.8 to 7.6 is used; mostly, a pH range from 7.0 to 7.4 is appropriate.

When the cell to be cultured is CHO cells, culture may be performed by methods known to those skilled in the art. For example, it is usually possible to perform culturing in a gas-phase atmosphere having a CO₂ concentration of 0-40%, preferably 2-10%, at 30-39°C, preferably around 37°C.

The appropriate period of culture is usually from one day to three months, preferably from one day to three weeks.

Isolation and purification of the antibody may be performed by known methods. Known isolation/purification methods which may be used in the present invention include, but are not limited to, methods using difference in solubility (such as salting-out or solvent precipitation); methods using difference in molecular weight (such as dialysis, ultrafiltration, gel filtration or SDS-polyacrylamide gel electrophoresis); methods using difference in electric charge (such as ion exchange chromatography); methods using specific affinity (such as affinity chromatography); methods using difference in hydrophobicity (such as reversed phase high performance liquid chromatography); and methods using difference in isoelectric point (such as isoelectric focusing).

The anti-PD-1 antibody of the present invention may be used as an antibody drug for animals or human. Therefore, the present invention provides a pharmaceutical composition comprising the above-described anti-PD-1 antibody as an active ingredient.

The pharmaceutical composition of the present invention may be used for prevention and/or treatment of cancers and/or infections. Examples of cancers and/or infections include, but are not limited to, neoplastic diseases (e.g., malignant melanoma, lung cancer, gastric cancer, renal cancer, breast cancer, bladder cancer, esophageal cancer, ovarian cancer and the like), leukemia, Johne's disease, anaplasmosis, bacterial mastitis, mycotic mastitis, mycoplasma infections (such as mycoplasma mastitis, mycoplasma pneumonia or the like), tuberculosis, *Theileria orientalis* infection, cryptosporidiosis, coccidiosis, trypanosomiasis and leishmaniasis.

The anti-PD-1 antibody of the present invention may be dissolved in buffers such as PBS, physiological saline or sterile water, optionally filter-sterilized with a filter or the like and then administered to animal subjects (including human) by injection. To the solution of this antibody, additives (such as coloring agents, emulsifiers, suspending agents, surfactants, solubilizers, stabilizers, preservatives, antioxidants, buffers, isotonizing agents, pH adjusters and the like) may be added. As routes of administration, intravenous, intramuscular, intraperitoneal, subcutaneous or intradermal administration and the like may be selected. Transnasal or oral administration may also be used.

The dose and the number of times and frequency of administration of the anti-PD-1 antibody of the present invention may vary depending on the symptoms, age and body weight of the animal subject, the method of administration, the dosage form and so on. For example, 0.1-100 mg/kg body weight, preferably 1-10 mg/kg body weight, per adult animal may usually be administered at least once at such a frequency that enables confirmation of desired effect.

While the pharmaceutical composition of the present invention may be used alone, it may be used in combination with surgical operations, radiation therapies, other immunotherapies such as cancer vaccine, or molecular target drugs. Synergistic effect can be expected from such combinations.

### EXAMPLES

Hereinbelow, the present invention will be described in more detail with reference to the following Examples. However, the present invention is not limited to these Examples.

### [Example 1] Establishment of Rat-Bovine Chimeric Anti-Bovine PD-1 Antibody Introduction

Programmed cell death 1 (PD-1), an immunoinhibitory receptor, and its ligand programmed cell death ligand 1 (PD-L1) are molecules identified by Prof. Tasuku Honjo et al., Kyoto University, as factors which inhibit excessive immune response and are deeply involved in immunotolerance. Recently, it has been elucidated that these molecules are also involved in immunosuppression in tumors. In the subject Example, for the purpose of establishing a novel therapy for bovine infections, a chimeric antibody gene was prepared in which variable region genes of rat anti-bovine PD-1 monoclonal antibody 5D2 capable of inhibiting the binding of bovine PD-1 to PD-L1 were linked to constant region genes of bovine immunoglobulins (bovine IgG1 and Igλ, with mutations having been introduced into the putative binding sites of Fcγ receptors in bovine IgGl's CH2 domain to inhibit ADCC activity; see Figs. 1 and 11 for amino acid numbers and mutations: 250 E→P, 251 L→V, 252 P→A, 253 G→deletion, 347 A→S, 348 P→S; Ikebuchi R, Konnai S, Okagawa T, Yokoyama K, Nakajima C, Suzuki Y, Murata S, Ohashi K. Immunology, 142(4):551-561, Aug. 2014). The resultant chimeric antibody gene was introduced into Chinese hamster ovary cells (DHO cells), which were cultured and proliferated to produce a rat-bovine chimeric anti-bovine PD-1 antibody ch5D2. The effect of this chimeric antibody was confirmed *in vitro* and *in vivo.*

### Materials, Methods and Experimental Results

### 2.1. Construction of Bovine PD-1 and PD-L1 Expressing Cells

The nucleotide sequences of the full length cDNAs of bovine PD-1 gene (GenBank accession number AB510901; Ikebuchi R, Konnai S, Sunden Y, Onuma M, Ohashi K. Microbiol. Immunol., 54(5):291-298; May 2010) and bovine PD-L1 gene (GenBank accession number AB510902; Ikebuchi R, Konnai S, Shirai T, Sunden Y, Murata S, Onuma M, Ohashi K. Vet. Res., 42:103, Sep. 26, 2011) were determined. Based on the resultant genetic information, bovine PD-1 and bovine PD-L1 expressing cells were prepared. First, for preparing bovine PD-1 or PD-L1 expressing plasmid, PCR was performed using a synthesized bovine peripheral blood mononuclear cell (PBMC)-derived cDNA as a template and designed primers having *Not*I and *Hind*III (bovine PD-1) recognition sites or *NheI* and *Xho*I (bovine PD-L1) recognition sites on the 5' side (boPD-1-myc F and R; or boPD-L1-EGFP F and R). The PCR products were digested with *Not*I (Takara) and *Hind*III (Takara; bovine PD-1) or *Nhe*I (Takara) and *Xho*I (Takara; bovine PD-L1), purified with FastGene Gel/PCR Extraction Kit (NIPPON Genetics) and cloned into pCMV-Tag1 vector (Agilent Technologies; bovine PD-1) or pEGFP-N2 vector (Clontech; bovine PD-L1) treated with the restriction enzymes in the same manner. The resultant expression plasmid of interest was extracted with QIAGEN Plasmid Midi kit (Qiagen) and stored at -30°C until use in experiments. Hereinafter, the thus prepared expression plasmid is designated as pCMV-Tag1-boPD-1 or pEGFP-N2-boPD-Ll.
Primer (boPD-1-myc F): ATATGCGGCCGCATGGGGACCCCGCGGGCGCT (SEQ ID NO: 61)
Primer (boPD-1-myc R): GCGCAAGCTTTCAGAGGGGCCAGGAGCAGT (SEQ ID NO: 62)
Primer (boPD-L1-EGFP F): CTAGCTAGCACCATGAGGATATATAGTGTCTTAAC (SEQ ID NO: 63)
Primer (boPD-L1-EGFP R): CAATCTCGAGTTACAGACAGAAGATGACTGC (SEQ ID NO: 64)

Bovine PD-1 expressing cells were prepared by the procedures described below. First, 2.5 µg of pCMV-Tag1-boPD-1 was introduced into 4x10⁶ CHO-DG44 cells using Lipofectamine LTX (Invitrogen). Forty-eight hours later, the medium was exchanged with CD DG44 medium (Life Technologies) containing 800 µg/ml G418 (Enzo Life Science), 20 ml/L GlutaMAX supplement (Life Technologies), and 18 ml/L 10% Pluronic F-68 (Life Technologies), followed by selection. The resultant expression cells were reacted with rat anti-bovine PD-1 antibody 5D2 at room temperature. After washing, the cells were further reacted with anti-rat IgG microbead-labeled antibody (Miltenyi Biotec) at room temperature. Cells expressing bovine PD-1 at high levels were isolated with Auto MACS (Miltenyi Biotec). Subsequently, re-isolation was performed in the same manner to obtain still higher purity. The resultant expression cells were subjected to cloning by limiting dilution to thereby obtain a CHO DG44 cell clone expressing bovine PD-1 at high level (bovine PD-1 expressing cells).

Bovine PD-L1 membrane expressing cells were prepared by the procedures described below. First, 2.5 µg of pEGFP-N2-boPD-L1 or pEGFP-N2 (negative control) was introduced into 4x10⁶ CHO-DG44 cells using Lipofectamine LTX (Invitrogen). Forty-eight hours later, the medium was exchanged with CD DG44 medium (Life Technologies) containing 800 µg/ml G418 (Enzo Life Science), 20 ml/L GlutaMAX supplement (Life Technologies) and 18 ml/L 10% Pluronic F-68 (Life Technologies), followed by selection and cloning by limiting dilution (bovine PD-L1 expressing cell clone). In order to confirm the expression of bovine PD-L1 in the thus prepared cell clone, intracellular localization of EGFP was visualized with an inverted confocal laser microscope LSM700 (ZEISS).

### 2.2 Construction of Soluble Bovine PD-1

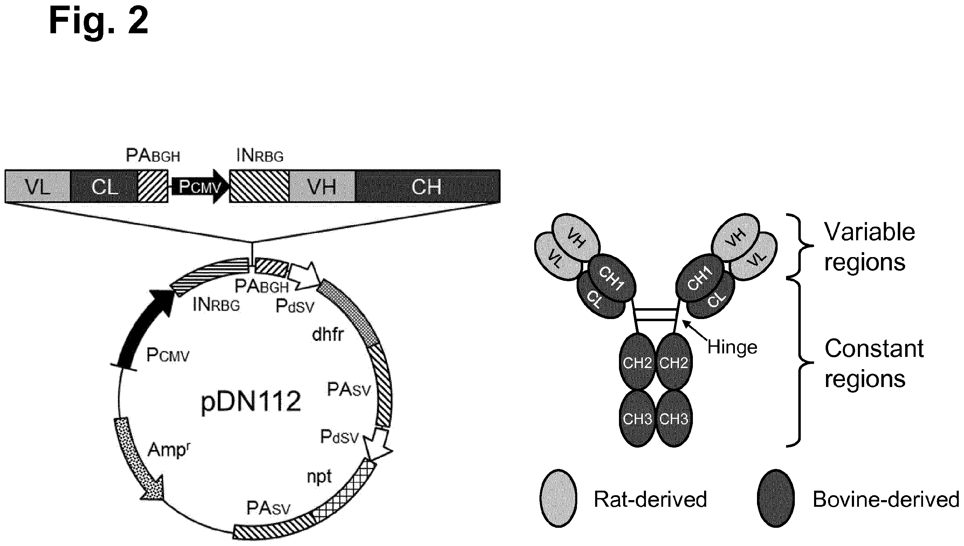

Bovine PD-1-Ig expressing plasmid was constructed by the procedures described below. Briefly, the signal peptide and the extracellular region of bovine PD-1 (GenBank accession number AB510901) were linked to the constant region of a known bovine IgG1 (GenBank accession number X62916) to prepare a gene sequence. After codons were optimized for CHO cells, gene synthesis was performed in such a manner that *Not*I recognition sequence, KOZAK sequence, bovine PD-1 signal peptide sequence, bovine PD-1 gene extracellular region sequence, bovine IgG1 Fc region sequence, and *Xba*I recognition sequence would be located in the gene in this order. It should be noted here that bovine IgG1 was mutated to inhibit ADCC activity; more specifically, mutations were introduced into the putative binding sites for Fcγ receptors of CH2 domain (sites of mutation: 185 E→P, 186 L→ V, 187 P→A, 189 G→deletion, 281 A→S, 282 P→S; Ikebuchi R, Konnai S, Okagawa T, Yokoyama K, Nakajima C, Suzuki Y, Murata S, Ohashi K. Immunology, 142(4):551-561, Aug. 204; the amino acid sequence of PD-1-Ig and the sites of mutation are disclosed in Figure 2 of this article). The synthesized gene strand was digested with *Not*I (Takara) and *Xba*I (Takara), purified with FastGene Gel/PCR Extraction Kit (NIPPON Genetics), and incorporated into the cloning site (*Not*I and *Xba*I restriction enzyme recognition sequences downstream of PCMV and between INRBG and PABGH) of expression vector pDN11 (kindly provided by Prof. S. Suzuki, Hokkaido University Research Center for Zoonosis Control) treated with the restriction enzymes in the same manner, whereby bovine PD-1-Ig expressing vector was constructed. The expression plasmid was purified with QIAGEN Plasmid Midi kit (Qiagen) and stored at -30°C until use in experiments. Hereinafter, the thus prepared expression plasmid is designated as pDN11-boPD-1-Ig.

Bovine PD-1-His expressing plasmid was prepared by the procedures described below. Briefly, for the purpose of amplifying the signal peptide and the extracellular region of bovine PD-1 (GenBank accession number AB510901), primers were designed in which *Not*I and *Xho*I recognition sites were added on the 5' side (boPD-1-His F and R). A genetic sequence encoding a 6xHis tag was added to the reverse primer. PCR was performed using a synthesized bovine PBMC-derived cDNA as a template. The respective PCR products were digested with *Not*I (Takara) and *Xho*I (Takara), purified with FastGene Gel/PCR Extraction Kit (NIPPON Genetics) and cloned into pCXN2.1(+) vector (Niwa H, Yamamura K, Miyazaki J. Gene, 108(2):193-199; Dec. 15, 1991; kindly provided by Dr. T. Yokomizo, Juntendo University Graduate School of Medicine) treated with the restriction enzymes in the same manner. The resultant expression plasmid was purified with FastGene Xpress Plasmid PLUS Kit (NIPPON Genetics) and stored at -30°C until use in experiments. Hereinafter, the thus prepared expression plasmid is designated as pCXN2.1-boPD-1-His.
Primer (boPD-1-His F): ATAAGAATGCGGCCGCCACCATGGGGACCCCGCGGGCGCT (SEQ ID NO: 65)
Primer (boPD-1-His R): GCCCTCGAGTTAATGGTGATGGTGATGGTGGATGACCAGGCTCTGCATCT (SEQ ID NO: 66)

Soluble bovine PD-1-Ig expressing cells were prepared by the procedures described below. Briefly, 2.5 µg of pDN11-boPD-1-Ig was introduced into 4x10⁶ CHO-DG44 cells using Lipofectamine LTX (Invitrogen). Forty-eight hours later, the medium was exchanged with CD OptiCHO medium (Life Technologies) containing 800 µg/ml G418 (Enzo Life Science) and 20 ml/L GlutaMAX supplement (Life Technologies). After cultured for 3 weeks, the cells were subjected to selection. Briefly, the concentrations of the Fc fusion recombinant protein in the culture supernatants of the resultant cell clones were measured by ELISA using anti-bovine IgG F(c) rabbit polyclonal antibody (Rockland) to thereby select those cell clones that express the Fc fusion recombinant protein at high levels. The resultant highly expressing cell clone was transferred to a G418-free medium and cultured under shaking for 14 days, followed by collection of a culture supernatant. The culture supernatant containing the Fc fusion recombinants protein was ultrafiltered with Centricon Plus-70 (Millipore). Then, the Fc fusion recombinant protein was purified with Ab-Capcher Extra (ProteNova). After purification, the buffer was exchanged with phosphate-buffered physiological saline (PBS; pH 7.4) using PD-10 Desalting Column (GE Healthcare). The resultant protein was stored at -30°C until use in experiments (bovine PD-1-Ig). The concentration of the purified bovine PD-1-Ig was measured by ELISA using IgG F(c) rabbit polyclonal antibody (Rockland). For each washing operation in ELISA, Auto Plate Washer BIO WASHER 50 (DS Pharma Biomedical) was used. Absorbance was measured with Microplate Reader MTP-650FA (Corona Electric).

Soluble bovine PD-1-His expressing cells were prepared by the procedures described below. Briefly, 30 µg of pCXN2.1-boPD-1-His was introduced into 7.5x10⁷ Expi293F cells (Life Technologies) using Expifectamine (Life Technologies). After a 7-day culture under shaking, the culture supernatant was collected. The recombinant protein of interest was purified from the culture supernatant using TALON Metal Affinity Resin (Clontech; bovine PD-1-His). After purification, the buffer was exchanged with PBS (pH 7.4) using PD MiniTrap G-25 (GE Healthcare). The resultant protein was stored at -30°C until use in experiments (bovine PD-1-His). The concentration of purified bovine PD-1-His was quantitatively determined in terms of the absorbance (280 nm) measured with Nanodrop8000 Spectrophotometer (Thermo Fisher Scientific).

### 2.3. Preparation of Rat Anti-Bovine PD-1 Monoclonal Antibody Producing Cells

Rat was immunized in the footpad with bovine PD-1-Ig (described above). Hybridomas were established by the iliac lymph node method to thereby obtain rat anti-bovine PD-1 monoclonal antibody producing hybridoma 5D2. With respect to the method of establishment of rat anti-bovine PD-1 monoclonal antibody, details are disclosed in the following non-patent document (Ikebuchi R, Konnai S, Okagawa T, Yokoyama K, Nakajima C, Suzuki Y, Murata S, Ohashi K. Vet. Res. 44:59; Jul. 22, 2013).

### 2.4. Preparation of Rat-Bovine Chimeric Anti-Bovine PD-1 Antibody Expressing Vector

Rat-bovine chimeric anti-bovine PD-1 antibody ch5D2 was established by fusing the antibody constant regions of bovine IgG1 and Igλ, with rat anti-bovine PD-1 antibody 5D2 being used as antibody variable regions.

First, the genes of heavy chain and light chain variable regions were identified by the RACE method from a hybridoma that would produce rat anti-bovine PD-1 antibody 5D2. Subsequently, a gene sequence was prepared in which the heavy chain and the light chain variable regions of the rat anti-bovine PD-1 antibody 5D2 were linked to known constant regions of bovine IgG1 (heavy chain, modified from GenBank Accession number X62916) and bovine Igλ (light chain; GenBank Accession number X62917), respectively. Then, codon optimization was carried out (SEQ ID NOS: 9 and 10 (amino acid sequences); SEQ ID NOS: 14 and 15 (nucleotide sequences after codon optimization)). It should be noted that bovine IgG1 had mutations added to the putative binding sites of Fcγ receptors in CH2 domain in order to suppress ADCC activity (See Figs. 1 and 11 for amino acid numbers and mutations: 251 E→P, 252 L→V, 253 P→A, 254 G→deletion, 348 A→S, 349 P→S; Ikebuchi R, Konnai S, Okagawa T, Yokoyama K, Nakajima C, Suzuki Y, Murata S, Ohashi K. Immunology, 142(4):551-561; Aug. 2014). Then, the gene was artificially synthesized in such a manner that *Not*I recognition sequence, KOZAK sequence, chimeric antibody light chain sequence, poly-A addition signal sequence (PABGH), promoter sequence (PCMV), *Sac*I recognition sequence, intron sequence (INRBG), KOZAK sequence, chimeric antibody heavy chain sequence and *Xba*I recognition sequence would be located in this order. The synthesized gene strand was digested with *Not*I (Takara) and *Xba*I (Takara), purified with FastGene Gel/PCR Extraction Kit (NIPPON Genetics) and cloned into the cloning site (*Not*I and *Xba*I restriction enzyme recognition sequences downstream of PCMV and between INRBG and PABGH) of expression plasmid pDN112 (kindly provided by Prof. S. Suzuki, Hokkaido University Research Center for Zoonosis Control) treated with the restriction enzymes in the same manner (Fig. 2). The resultant plasmid of interest was extracted with QIAGEN Plasmid Midi kit (Qiagen) and stored at -30°C until use in experiments. Hereinafter, the thus prepared expression plasmid is designated as pDN112-boPD-1ch5D2.

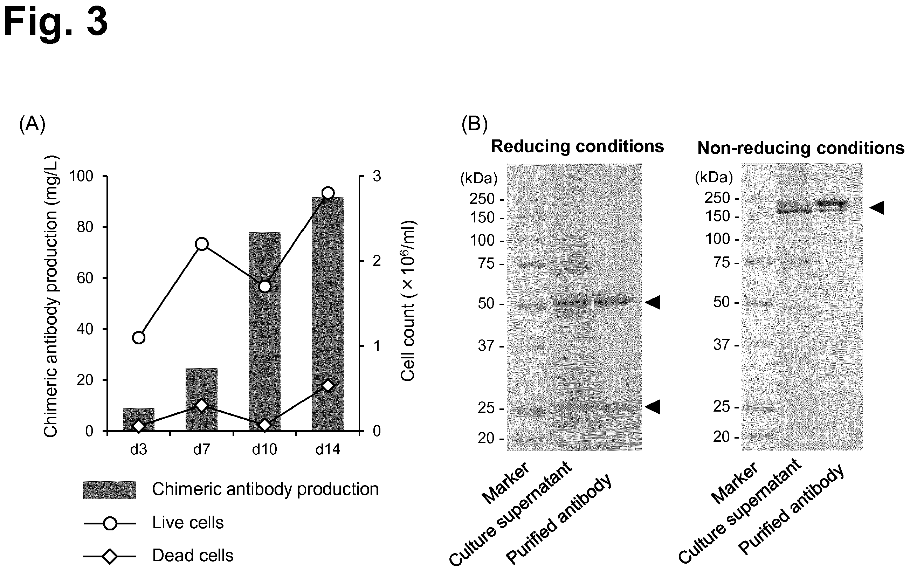

### 2.5. Expression of Rat-Bovine Chimeric Anti-Bovine PD-1 Antibody (Fig. 3)

The pDN112-boPD-1ch5D2 prepared above was transfected into CHO-DG44 cells (CHO-DG44(dfhr⁻/⁻)) which were a dihydrofolate reductase deficient cell. Forty-eight hours later, the medium was exchanged with CD OptiCHO medium (Life Technologies) containing 2 mM GlutaMAX supplement (Life Technologies) and 800 µg/ml G418 sulfate (Enzo Life Science). After cultured for 3 weeks, the expression cells were subjected to selection and cloning by limiting dilution. Subsequently, the concentrations of the chimeric antibody in the culture supernatants were measured by dot blotting and ELISA using anti-bovine IgG F(c) rabbit polyclonal antibody (Rockland) to thereby select high expression clones. Further, to the selected clones expressing rat-bovine chimeric anti-bovine PD-1 antibody at high levels were subjected to gene amplification treatment by adding a load with 60 nM methotrexate (Mtx; Wako)-containing medium. The thus established cell clone stably expressing rat-bovine chimeric anti-bovine PD-1 antibody was transferred into Mtx-free CD Opti-CHO medium and cultured under shaking for 14 days (125 rpm, 37 °C, 5% CO₂). Chimeric antibody production in the culture supernatant was measured by ELISA using anti-bovine IgG F(c) rabbit polyclonal antibody (Rockland). For each washing operation in ELISA, Auto Plate Washer BIO WASHER 50 (DS Pharma Biomedical) was used. Absorbance was measured with Microplate Reader MTP-650FA (Corona Electric). The culture supernatant at day 14 was centrifuged at 10,000 g for 10 min to remove cells, and the centrifugal supernatant was passed through a Steritop-GP 0.22 µm filter (Millipore) for sterilization and then stored at 4°C until it was subjected to purification.

The results are shown in Fig. 3A. Among the rat-bovine chimeric anti-bovine PD-1 antibody expressing cell clones, the most productive clone secreted 91.7 mg/l of the chimeric antibody into the culture supernatant during the 14-day culture under shaking.

### 2.6. Purification of Rat-Bovine Chimeric Anti-Bovine PD-1 Antibody

From the culture supernatant prepared as described above, each chimeric antibody was purified using Ab Capcher Extra (ProteNova). An open column method was used for binding to resin; 1.5 M Glycine/3 M NaCl (pH 8.0) was used as equilibration buffer and wash buffer. As elution buffer, 0.1 M Glycine-HCl (pH 2.8) was used. As neutralization buffer, 1M Tris (pH 9.0) was used. The purified antibody was subjected to buffer exchange with PBS (pH 7.4) using PD-10 Desalting Column (GE Healthcare) and concentrated using Amicon Ultra-15 (50 kDa, Millipore). The thus purified chimeric antibody was passed through a 0.22 µm syringe filter (Pall Life Sciences) for sterilization and stored at 4°C until use in experiments.

### 2.7. Confirmation of the Purity of Purified Rat-Bovine Chimeric Anti-Bovine PD-1 Antibody (Fig. 3)

In order to confirm the purity of purified rat-bovine chimeric anti-bovine PD-1 antibody, antibody proteins were detected by SDS-PAGE and CBB staining. Purified rat-bovine chimeric anti-bovine PD-1 antibody ch5D2 was suspended in Laemmli Sample Buffer (Bio-Rad) and denatured at 95°C for 5 min under reducing conditions (reduced with 2-mercaptoethaanol; Sigma-Aldrich) or under non-reducing conditions. The thus prepared samples were electrophoresed using 10% polyacrylamide gel. As molecular weight markers, Precision Plus Protein All Blue Standards (Bio-Rad) were used. After electrophoresis, the gel was stained with Quick-CBB kit (Wako) and subsequently decolored in distilled water.

The results are shown in Fig. 3B. Bands of rat-bovine chimeric anti-bovine PD-1 antibody were observed at predicted positions, that is, at 25 kDa (light chain) and 50 kDa (heavy chain) under reducing conditions and at 150 kDa under non-reducing conditions.

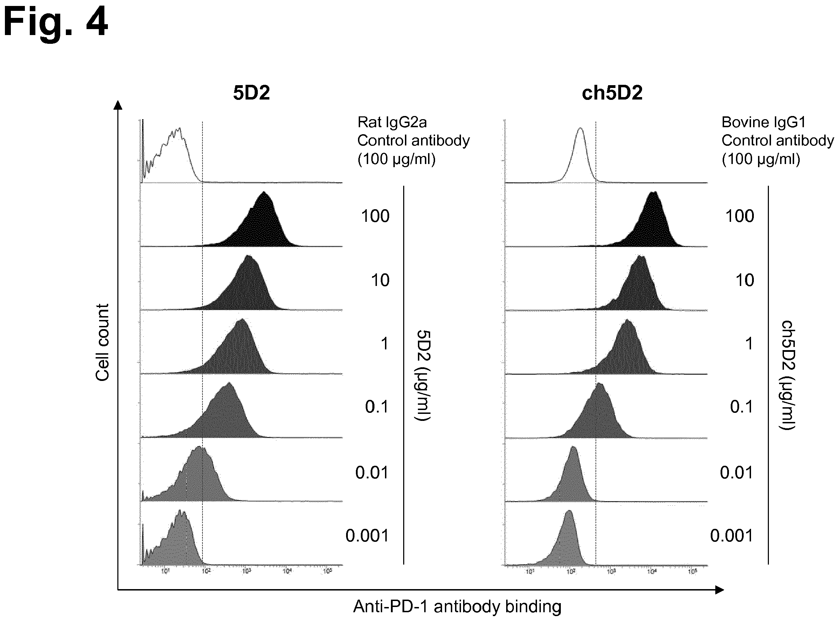

### 2.8. Binding Specificity of Rat-Bovine Chimeric Anti-Bovine PD-1 Antibody (Fig. 4)

It was confirmed by flow cytometry that rat-bovine chimeric anti-bovine PD-1 antibody specifically binds to bovine PD-1 expressing cells (described above). First, rat anti-bovine PD-1 antibody 5D2 or rat-bovine chimeric anti-bovine PD-1 antibody ch5D2 was reacted with bovine PD-1 expressing cells at room temperature for 30 min. After washing, Allophycocyanine (APC)-labeled anti-rat Ig goat antibody (Southern Biotech) or Alexa Fluor 647-labeled anti-bovine IgG (H+L) goat F(ab')2 (Jackson ImmunoResearch) was reacted at room temperature for 30 min. As negative control antibody, rat IgG2a (κ) isotype control (BD Biosciences) or bovine IgG1 antibody (Bethyl) was used. After washing, each rat antibody or rat-bovine chimeric antibody bound to cell surfaces was detected by FACS Verse (BD Biosciences). For every washing operation and dilution of antibodies, PBS supplemented with 1% bovine serum albumin (Sigma-Aldrich) was used.

The experimental results are shown in Fig. 4. It was revealed that rat-bovine chimeric anti-bovine PD-1 antibody ch5D2 binds to bovine PD-1 expressing cells in the same manner as rat anti-bovine PD-1 antibody 5D2.

### 2.9. PD-1 Binding Avidity of Rat-Bovine Chimeric Anti-Bovine PD-1 Antibody

The binding avidities to bovine PD-1 of rat anti-bovine PD-1 antibody 5D2 and rat-bovine chimeric anti-bovine PD-1 antibody ch5D2 were measured by surface plasmon resonance using a biomolecular interaction analyzer (Biacore X100). Briefly, bovine PD-1-His (described above) was immobilized on a CM5 sensor chip (GE Healthcare) as a ligand. Subsequently, rat anti-bovine PD-1 antibody 5D2 or rat-bovine chimeric anti-bovine PD-1 antibody ch5D2 was reacted as an analyte, followed by single kinetics analysis. The experiment was repeated 3 times under the same conditions. Binding constant (kd value) and dissociation constant (ka value) were determined in each experiment, and binding avidity (KD value) was obtained.

The experimental results are shown in the table below. The binding avidity of rat-bovine chimeric anti-bovine PD-1 antibody for PD-1 protein was similar to that of rat anti-bovine PD-1 antibody 5D2, with no statistical difference observed (p>0.05; Welch's t-test).

| **Anti-Bovine PD-1 Antibody** | **ka (1/Ms)** | **kd (1/s)** | **KD (M)** |
|---|---|---|---|
| **5D2** | 1.84×10⁴ ± 0.27 | 2.15×10⁻⁴ ± 0.44 | **1.22x10⁻⁸ ± 0.39** |
| **ch5D2** | 2.07×10⁴ ± 0.06 | 2.16×10⁻⁴ ± 1.12 | **1.05×10⁻⁸ ± 0.58** |

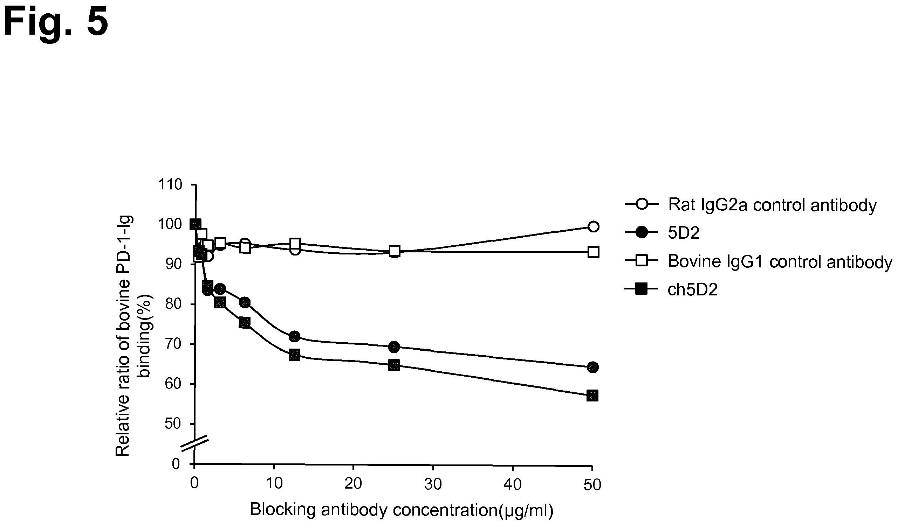

### 2.10. Blockade of Bovine PD-1/PD-L1 Binding by Rat-Bovine Chimeric Anti-Bovine PD-1 Antibody (Fig. 5)

Using bovine PD-L1 expressing cells (described above) and bovine PD-1-Ig (described above), bovine PD-1/PD-L1 binding inhibition by anti-PD-1 antibodies was tested. First, rat anti-bovine PD-1 antibody 5D2 or rat-bovine chimeric anti-bovine PD-1 antibody ch5D2 (final concentration: 0, 0.39, 0.78, 1.56, 3.12, 6.25, 12.5, 25 or 50 µg/ml) and bovine PD-1-Ig (final concentration: 5 µg/ml) labeled with biotin using Lightning-Link Type A Biotin Labeling Kit (Innova Biosciences) were added to 96-well plates, followed by reaction at 37°C for 30 min. The resultant mixture was reacted with 1x10⁵ bovine PD-L1 expressing cells at 37°C for 30 min. After washing, the reaction mixture was reacted with APC-labeled streptavidin (BioLegend) at room temperature for 30 min to thereby detect bovine PD-1-Ig bound to cell surfaces. For analysis, FACS Verse (BD Biosciences) was used. For every washing operation and dilution of antibodies, PBS supplemented with 1% bovine serum albumin (Sigma-Aldrich) was used. Taking the proportion of bovine PD-1-Ig-bound cells without addition of antibodies as 100%, the proportion of bovine PD-1-Ig-bound cells at each antibody concentration was shown as a relative value.

The experimental results are shown in Fig. 5. Rat-bovine chimeric anti-bovine PD-1 antibody ch5D2 inhibited the binding of PD-1-Ig to PD-L1 expressing cells by the same degree as rat anti-bovine PD-1 antibody 5D2 did.

### 2.11. CDR Analysis of Rat Anti-Bovine PD-1 Antibody

The complementarity-determining regions (CDRs) of rat anti-bovine PD-1 antibody 5D2 were determined using NCBI IGBLAST (http://www.ncbi.nlm.nih.gov/igblast/). The results are shown in Fig. 1.

### 2.12. Inoculation Test on Cattle with Rat-Bovine Chimeric Anti-Bovine PD-1 Antibody

Established rat-bovine chimeric anti-bovine PD-1 antibody ch5D2 (14 mg; 0.08 mg/kg) was intravenously adinistrated into an experimentally BLV-infected calf (Holstein, male, 4 months old, 173.5 kg). Blood samples were collected chronologically from the infected calf, followed by collection of blood (with heparin sodium (Ajinomoto) used as anticoagulant) and serum. Peripheral blood mononuclear cells (PBMCs) were isolated from the blood by density gradient centrifugation using Percoll (GE Healthcare).

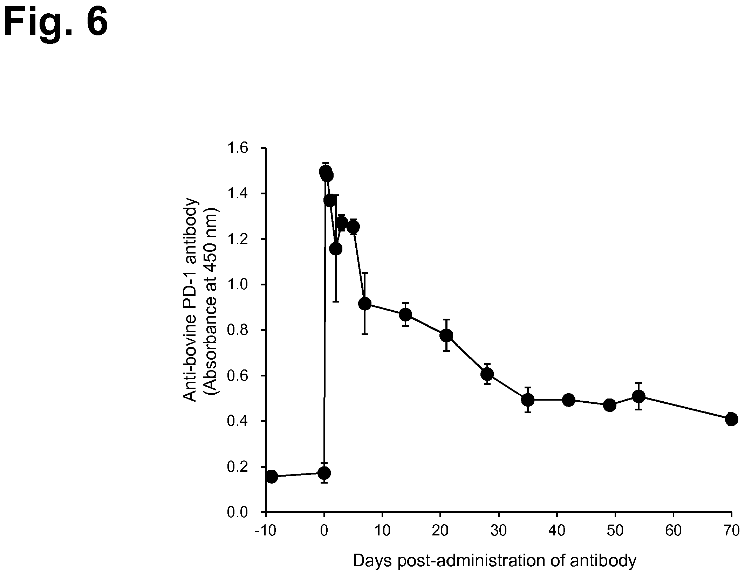

### 2.13. Kinetics of Administered Rat-Bovine Chimeric Anti-Bovine PD-1 Antibody in Blood (Fig. 6)

Bovine PD-1-His (described above) was immobilized on ELISA plates (H type, Sumitomo Bakelite) at a final concentration of 10 µg/ml at 4°C overnight. Subsequently, each well was washed with 200 µl of 0.05% Tween 20-supplemented Tris-buffered saline (TBS-T) five times, followed by blocking with 1% skim milk-supplemented TBS-T at room temperature for 1 hr. Another washing was carried out in the same manner. The serum collected from the test calf was added to each well and reacted at room temperature for 1 hr. After washing, horseradish peroxidase-labeled anti-bovine IgG F(c) rabbit polyclonal antibody (Rockland) was reacted at room temperature for 1 hr. Each well was washed again and then TMB One Component Substrate (Bethyl) was added for coloring. The enzyme reaction was terminated with 0.18 M dilute sulfuric acid. Absorbance (450 nm) was measured with Microplate Reader MTP-650FA (Corona Electric). For every plate washing operation, Auto Plate Washer BIO WASHER 50 (DS Pharma Biomedical) was used.

The experimental results are shown in Fig. 6. Rat-bovine chimeric anti-bovine PD-1 antibody was detected in the serum of the test calf until 70 days after administration (at the end of the clinical test). The antibody retained particularly high concentrations for one week after administration.

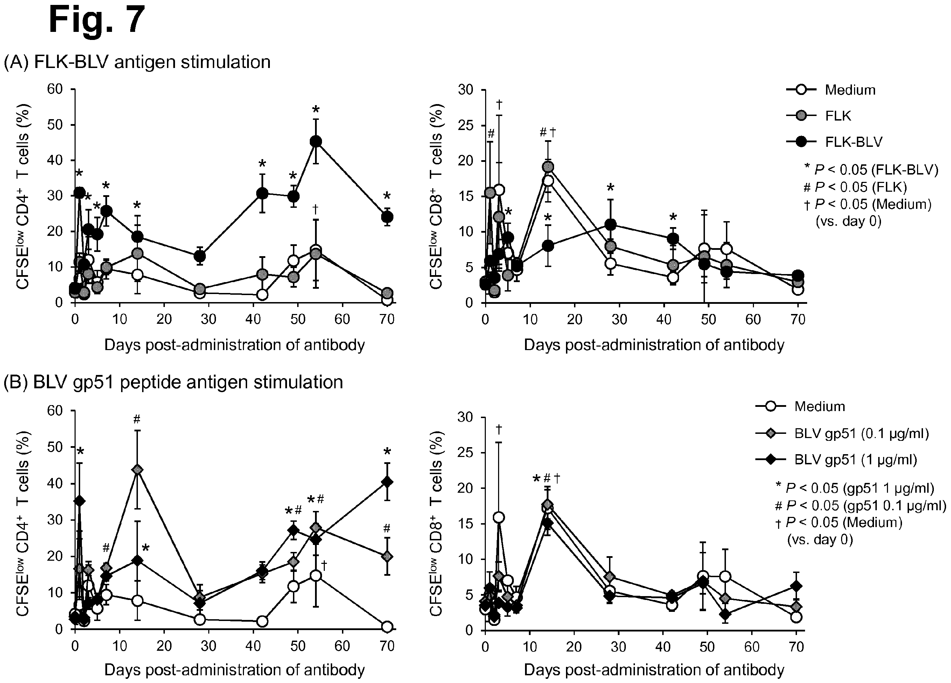

### 2.14. Cell Proliferation Response of T Cells to BLV Antigen (Fig. 7)

Bovine PBMCs were suspended in PBS and reacted with carboxyfluorescein succinimidyl ester (CFSE; Invitrogen) at room temperature for 20 min for labeling. After washing twice with RPMI 1640 medium (Sigma-Aldrich) containing 10% inactivated fetal bovine serum (Cell Culture Technologies), penicillin200 U/ml, streptomycin 200 µg/ml and 0.01% L-glutamine (Life Technologies), cell concentration was adjusted to 4x10⁶ cells/ml using the same medium. To the PBMCs, culture supernatant of 2% BLV-infected fetal lamb kidney cell (FLK-BLV), culture supernatant of fetal lamb kidney cell (FLK) not infected with 2% BLV, or BLV gp51 peptide mix 0.1 µg/ml or 1 µg/ml was added, followed by a 6-day culture at 37°C under 5% CO₂. After 6 days, PBMCs were recovered and reacted with Alexa Fluor 647-labeled mouse anti-bovine CD4 antibody (CC30, AbD Serotec), Peridinin-chlorophyll-protein complex/cyanin 5.5-labeled mouse anti-bovine CD8 antibody (CC63, AbD Serotec) and R-Phycoerythrin/cyanin 7 (PE/Cy7)-labeled anti-bovine IgM mouse antibody (IL-A30, AbD Serotec) at 4°C for 20 min. For labeling antibodies, Zenon Mouse IgG1 Labeling Kits (Life Technologies) or Lightning-Link Kits (Innova Biosciences) was used. For analysis, FACS Verse (BD Biosciences) was used. For every washing operation and dilution of antibodies, PBS supplemented with 1% bovine serum albumin (Sigma-Aldrich) was used. With respect to the proportion of proliferated T cells (CFSE^{low} cells), statistical test was performed using the method of Dunnett.

The experimental results are shown in Fig. 7. Upon administration of rat-bovine chimeric anti-bovine PD-1 antibody, BLV-specific cell proliferative response in CD4⁺ T cells showed a statistically significant increase immediately after the administration, compared to the response before administration.

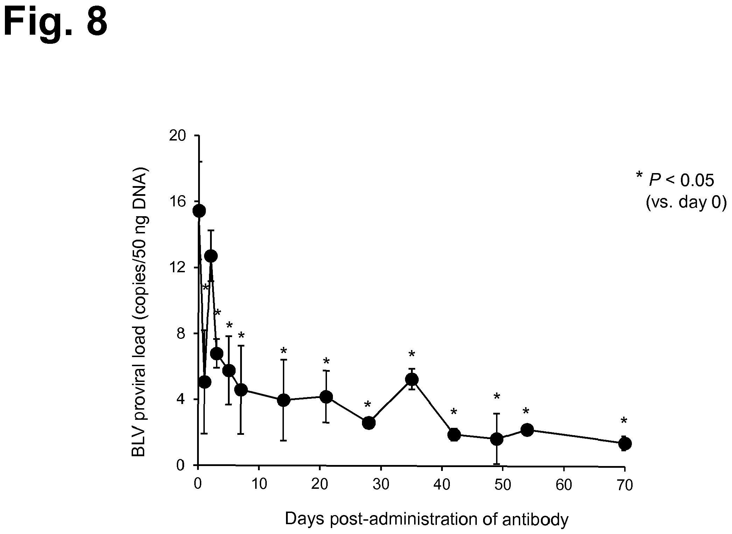

### 2.15. Transition in BLV Proviral Load (Fig. 8)

DNA was extracted from isolated bovine PBMCs using Wizard DNA Purification kit (Promega). The concentration of the extracted DNA was quantitatively determined based on the absorbance (260 nm) measured with Nanodrop 8000 Spectrophotometer (Thermo Fisher Scientific). For measuring BLV proviral load in PBMCs, real time PCR was performed using Cycleave PCR Reaction Mix SP (Takara) and Probe/Primer/Positive control (Takara) for bovine leukemia virus detection. LightCycler480 System II (Roche Diagnosis) was used for the measurement. With respect to the measured proviral load, statistical test was performed by the method of Dunnett.

The experimental results are shown in Fig. 8. Upon administration of rat-bovine chimeric anti-bovine PD-1 antibody, BLV proviral load in PBMCs showed a statistically significant decrease immediately after the administration, compared to the load before administration. The BLV proviral load remained at low levels until the end of the clinical test (day 70).

### [Example 2] Application of Anti-PD-1 Antibody to Other Animal Species 1. Materials, Methods and Experimental Results

### 1.1. Identification of Ovine and Water Buffalo PD-1 Genes

In order to determine the full-lengths of the coding sequences (CDSs) of ovine and water buffalo PD-1 cDNAs, primers for amplifying the full lengths of CDSs were first designed (ovPD-1 CDS F and R; buPD-1 CDS F1, R1, F2 and R2) based on the nucleotide sequences of ovine and water buffalo PD-1 genes (GenBank accession numbers BC123854 and XM_012176227), and then PCR was performed using a synthesized ovine or water buffalo PBMC-derived cDNA as a template. For the resultant amplified products, nucleotide sequences were determined with a capillary sequencer according to conventional methods (Mingala CN, Konnai S, Ikebuchi R, Ohashi K. Comp. Immunol. Microbiol. Infect. Dis., 34(1):55-63; Jan. 2011; Water buffalo PD-1 gene was identified in this article).
Primer (ovPD-1 CDS F): ATGGGGACCCCGCGGGCGCC (SEQ ID NO: 67)
Primer (ovPD-1 CDS R): TCAGAGGGGCCAGGAGCAGTGTCCA (SEQ ID NO: 68)
Primer (buPD-1 CDS F1): ATGGGGACCCCGCGGGCGCT (SEQ ID NO: 69)
Primer (buPD-1 CDS R1): GATGACCAGGCTCTGCATCT (SEQ ID NO: 70)
Primer (buPD-1 CDS F2): AATGACAGCGGCGTCTACTT (SEQ ID NO: 71)
Primer (buPD-1 CDS R2): TCAGAGGGGCCAGGAGCAGT (SEQ ID NO: 72)

### 1.2. Construction of Ovine PD-1 Expressing COS-7 Cells

In order to prepare ovine PD-1 expression plasmid, PCR was performed using a synthesized ovine PBMC-derived cDNA as a template and primers designed by adding *Bgl*II and *Sma*I recognition sites on the 5' side (ovPD-1-EGFP F and R). The resultant PCR products were digested with *Bgl*II (Takara) and *Sma*I (Takara), purified with FastGene Gel/PCR Extraction Kit (NIPPON Genetics) and cloned into pEGFP-N2 vector (Clontech) treated with the restriction enzymes in the same manner. The expression plasmid of interest was extracted using FastGene Xpress Plasmid PLUS Kit (NIPPON Genetics) and stored at -30°C until use in experiments. Hereinafter, the thus prepared plasmid is designated as pEGFP-N2-ovPD-1.
Primer (ovPD-1-EGFP F): GAAGATCTATGGGGACCCCGCGGGCGCCG (SEQ ID NO: 73)
Primer (ovPD-1-EGFP R): GACCCGGGGAGGGGCCAGGAGCAGTGTCC (SEQ ID NO: 74)

COS-7 cells were subcultured at a density of 5x10⁴ cells/cm² in 6-well plates, and then cultured overnight in RPMI 1640 medium containing 10% inactivated fetal bovine serum (Invitrogen) and 0.01% L-glutamine (Life Technologies) at 37°C in the presence of 5% CO₂. The pEGFP-N2-ovPD-1 or pEGFP-N2 (negative control) was introduced into COS-7 cells at 0.4 µg/cm² using Lipofectamine 2000 (Invitrogen). The cells were cultured for 48 hours (ovPD-1-EGFP expressing cells). In order to confirm the expression of ovine PD-1 in the thus prepared expressing cells, intracellular localization of EGFP was visualized with an all-in-one fluorescence microscope BZ-9000 (KEYENCE).

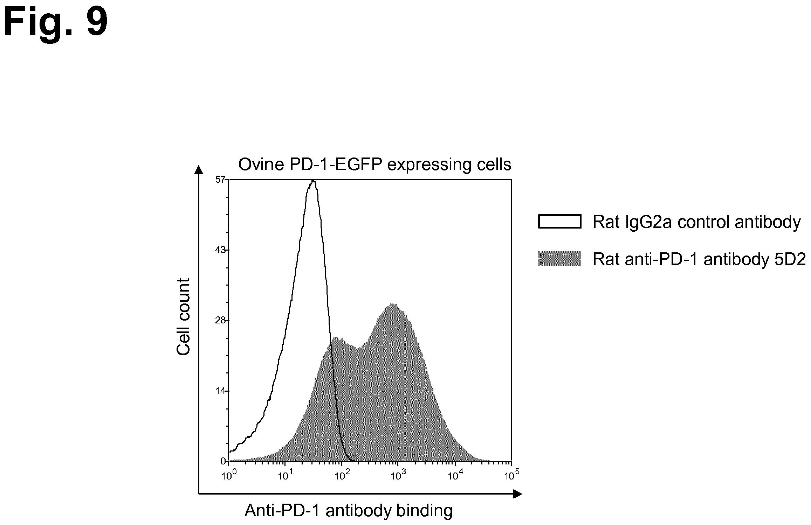

### 1.3. Reactivity of Rat Anti-Bovine PD-1 Antibody 5D2 with Ovine PD-1 (Fig. 9)

It was confirmed by flow cytometry that rat anti-bovine PD-1 monoclonal antibody cross-reacts with ovine PD-1. Ovine PD-1-EGFP expressing COS-7 cells were blocked with 10% inactivated goat serum (Invitrogen)-supplemented PBS at room temperature for 15 min and reacted with 10 µg/ml of rat anti-bovine PD-1 antibody 5D2 at room temperature for 30 min. After washing, the cells were reacted with APC-labeled anti-rat Ig goat antibody (Beckman Coulter) at room temperature for 30 min. As a negative control antibody, rat IgG2a (κ) isotype control (BD Bioscience) was used. For analysis, FACS Verse (BD Bioscience) was used. For every washing operation and dilution of antibodies, 1% bovine serum albumin (Sigma-Aldrich)-supplemented PBS was used.

The experimental results are shown in Fig. 9. It was confirmed that rat anti-bovine PD-1 antibody 5D2 binds to ovine PD-1 expressing cells.

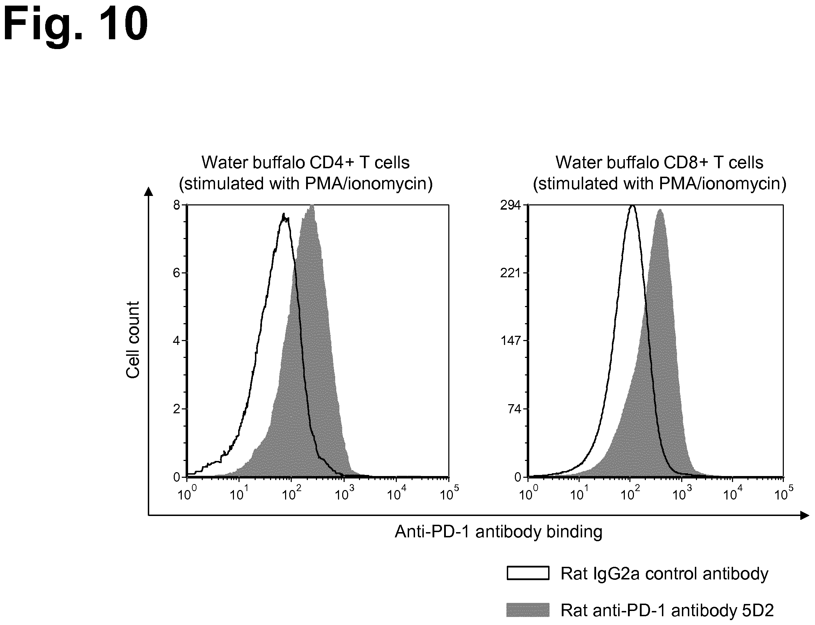

### 1.4. Reactivity of Rat Anti-Bovine PD-1 Antibody 5D2 with Water Buffalo Lymphocytes (Fig. 10)

Peripheral blood mononuclear cells (PBMCs) were isolated from peripheral blood of water buffalo (*Bubalus ubalis*; Asian water buffalo) by density gradient centrifugation using Percoll (GE Healthcare). The isolated water buffalo PBMCs were suspended in RPMI 1640 medium (Sigma-Aldrich) containing 10% inactivated fetal bovine serum (Cell Culture Technologies), penicillin 200 U/ml, streptomycin 200 µg/ml and 0.01% L-glutamine (Life Technologies). Cell density was adjusted to 2x10⁶ cells/ml. To these PBMCs, phorbol 12-myristate acetate (PMA) 20 ng/ml and ionomycin 1 µg/ml (Sigma-Aldrich) were added, followed by a 2-day culture at 37°C under 5% CO₂. Cultured PBMCs were harvested and blocked with 10% inactivated goat serum (Invitrogen)-supplemented PBS at room temperature for 15 min. Then, rat anti-bovine PD-1 antibody 5D2 and mouse anti-bovine CD8 antibody (38.65, AbD Serotec) were reacted at room temperature for 30 min. As a negative control, rat IgG2a (κ) isotype control (BD Bioscience) was used. After washing, APC-labeled goat anti-rat Ig antibody (Beckman Coulter) and PE-labeled goat anti mouse IgG antibody (Beckman Coulter) were reacted at room temperature for 30 min. After further washing, Alexa Flour488-labeled mouse anti-bovine CD4 antibody (CC30, AbD Serotec) and PE/Cy7-labeled anti-bovine IgM mouse antibody (IL-A30, AbD Serotec) were reacted at room temperature for 30 min. For antibody labeling, Zenon Mouse IgG1 Labeling Kits (Life Technologies) or Lightning-Link Kits (Innova Biosciences) was used. For analysis, FACS Verse (BD Biosciences) was used. For every washing operation and dilution of antibodies, 10% inactivated goat serum (Invitrogen)-supplemented PBS was used.

The experimental results are shown in Fig. 10. Rat anti-bovine PD-1 antibody 5D2 strongly bound to water buffalo CD4⁺ T cells (IgM⁻ CD4⁺) and CD8⁺ T cells (IgM⁻ CD8⁺) that had been activated by PMA/ionomycin stimulation.

### [Example 3] Binding to Bovine Fcγ Receptors of Rat-Bovine Chimeric Anti-Bovine PD-1 Antibody Having Wild-Type or Mutated Bovine IgG1

### Introduction

The present inventors have established a rat-bovine chimeric anti-bovine PD-1 antibody in Example 1 with a view to establishing a novel therapy for bovine infections. In the process, mutations were added to putative binding sites for Fcγ receptors in bovine IgG1 CH2 domain in order to suppress ADCC activity mediated by the chimeric antibody (Figs. 1 and 11). In the subject Example, in order to examine the effect of these amino acid mutations, the present inventors prepared rat-bovine chimeric anti-bovine PD-1 antibodies having mutated bovine IgG1 ("IgG1 ADCC-" described above) and wild-type bovine IgG1 ("IgG1 WT"), respectively, and confirmed their binding to known bovine Fey receptors.

### Materials, Methods and Experimental Results

### 2.1. Preparation of Rat-Bovine Chimeric Anti-Bovine PD-1 Antibody Expressing Vector

Rat-bovine chimeric anti-bovine PD-1 antibody ch5D2 having wild-type bovine IgG1 (IgG1 WT) or mutated bovine IgG1 (IgG1 ADCC- described above) was established.

An expression plasmid encoding rat-bovine chimeric anti-bovine PD-1 antibody ch5D2 having mutated bovine IgG1 (IgG1 ADCC-) was prepared according to the procedures described in Example 1 (SEQ ID NOS: 9 and 10 (amino acid sequences), SEQ ID NOS: 14 and 15 (nucleotide sequences after codon optimization)). It should be noted that in order to suppress ADCC activity, the bovine IgG1 used in ch5D2 IgG1 ADCC- had mutations added to the putative binding sites for Fcγ receptors in CH2 domain (see Figs. 1 and 11 for amino acid numbers and mutations: 251 E→P, 252 L→V, 253 P→A, 254 G→deletion, 348 A→S, 349 P→S; Ikebuchi R, Konnai S, Okagawa T, Yokoyama K, Nakajima C, Suzuki Y, Murata S, Ohashi K. Immunology, 142(4):551-561; Aug. 2014). Hereinafter, the thus prepared plasmid is designated as pDN112-boPD-1ch5D2 IgG1 ADCC-.

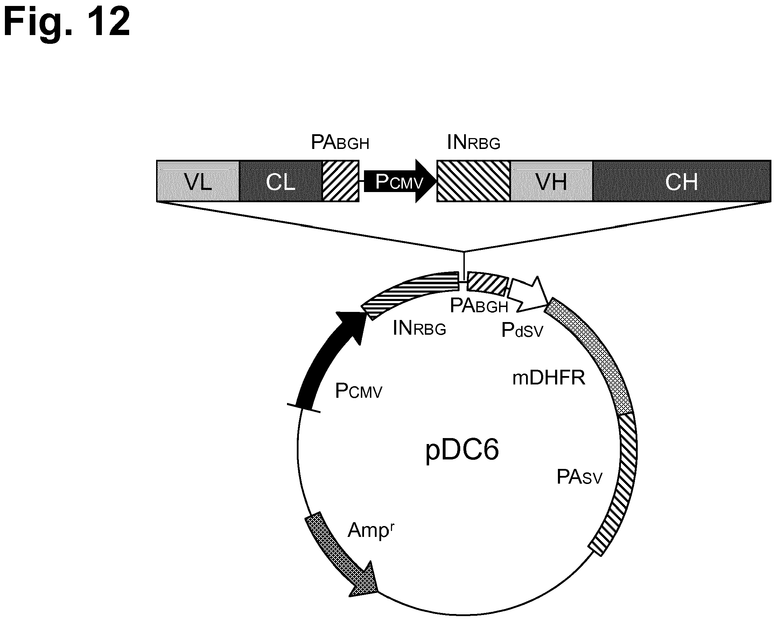

An expression plasmid encoding rat-bovine chimeric anti-bovine PD-1 antibody ch5D2 having wild-type IgG1 (IgG1 WT) was prepared according to the procedures described below. First, in order to amplify the gene encoding the constant region of wild-type bovine IgG1 (GenBank accession number X62916), PCR was performed using a synthesized bovine PBMC-derived cDNA as a template and designed primers that have *Nhe*I and *Xba*I recognition sites added on the 5' side (boIgG1 CH1 F and boIgG1 CH3 R). The amplified gene strand was digested with NheI (Takara) and XbaI (Takara), purified with FastGene Gel/PCR Extraction Kit (NIPPON Genetics), and cloned into pDN112-boPD-1ch5D2 IgG1 ADCC- that had been treated with the restriction enzymes in the same manner. Further, the resultant plasmid was purified with QIAGEN Plasmid Midi kit (Qiagen) and digested with *Not*I (Takara) and *Xba*I (Takara) to thereby obtain an expression cassette for ch5D2's light chain (SEQ ID NO: 9 (amino acid sequence), SEQ ID NO: 14 (nucleotide sequence)) and heavy chain (IgG1 WT) (SEQ ID NO: 75 (amino acid sequence), SEQ ID NO: 76 (nucleotide sequence)). This gene fragment was purified with FastGene Gel/PCR Extraction Kit (NIPPON Genetics) and cloned into the cloning site (*Not*I and *Xba*I restriction enzyme recognition sequences downstream of PCMV and between INRBG and PABGH) of expression vector pDC6 (kindly provided by Prof. S. Suzuki, Hokkaido University Research Center for Zoonosis Control) (Fig. 12). The resultant expression plasmid of interest was extracted with QIAGEN Plasmid Midi kit (Qiagen) and stored at -30°C until use in experiments. Hereinafter, the thus prepared expression plasmid is designated as pDC6-boPD-1ch5D2 IgG1 WT.
Primer (boIgG1 CH1 F): CTAGCTAGCACCACAGCCCCGAAAGTCT (SEQ ID NO: 77)
Primer (boIgG1 CH3 R): TGCTCTAGATTATTTACCCGCAGACTTAGA (SEQ ID NO: 78)

### 2.2. Expression and Purification of Rat-Bovine Chimeric Anti-Bovine PD-1 Antibody

Thirty micrograms of pDC6-boPD-lch5D2 IgG1 WT or pDN112-boPD-lch5D2 IgG1 ADCC- was introduced into 7.5x10⁷ Expi293F cells (Life Technologies) using Expifectamine (Life Technologies) and the transfected cells were then cultured under shaking for 5 to 7 days, followed by collection of a culture supernatant. Each chimeric antibody was purified from the culture supernatant using Ab Capcher Extra (ProteNova). An open column method was used for binding to resin; 1.5 M Glycine/3 M NaCl (pH 8.0) was used as equilibration buffer and wash buffer. As elution buffer, 0.1 M Glycine-HCl (pH 2.8) was used. As neutralization buffer, 1M Tris (pH 9.0) was used. The purified antibody was subjected to buffer exchange with PBS (pH 7.4) using PD-10 Desalting Column (GE Healthcare) and concentrated using Amicon Ultra-15 (50 kDa, Millipore). The thus purified chimeric antibody was passed through a 0.22 µm syringe filter (Pall Life Science) for sterilization and stored at 4°C until use in experiments. The concentration of each chimeric antibody as purified was quantitatively determined with the absorbance (280 nm) measured with Nanodrop8000 Spectrophotometer (Thermo Fisher Scientific).

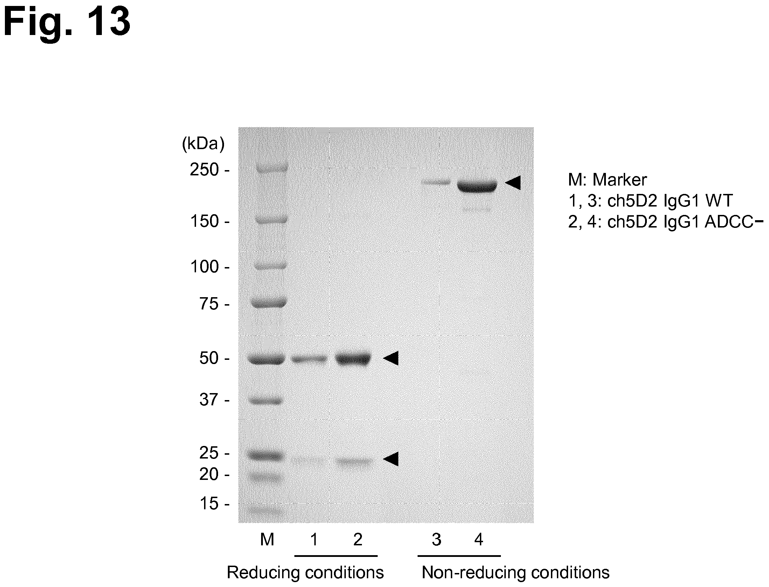

### 2.3. Confirmation of the Purity of Purified Rat-Bovine Chimeric Anti-Bovine PD-1 Antibodies (Fig. 13)

In order to confirm the purity of purified rat-bovine chimeric anti-bovine PD-1 antibodies (ch5D2 IgG1 WT and ch5D2 IgG1 ADCC-), antibody proteins were detected by SDS-PAGE and CBB staining. Each chimeric antibody purified was suspended in Laemmli Sample Buffer (Bio-Rad) and denatured at 95°C for 5 min under reducing conditions (reduced with 2-mercaptoethaanol; Sigma-Aldrich) or under non-reducing conditions. The thus prepared samples were electrophoresed using SuperSep Ace 5%-20% gradient polyacrylamide gel (Wako). As molecular weight markers, Precision Plus Protein All Blue Standards (Bio-Rad) were used. After electrophoresis, the gel was stained with Quick-CBB kit (Wako) and decolored in distilled water.

The results are shown in Fig. 13. Bands of ch5D2IgG1 WT and ch5D2 IgG1 ADCC-were observed at predicted positions, that is, at 25 kDa (light chain) and 50 kDa (heavy chain) under reducing conditions and at 150 kDa under non-reducing conditions.

### 2.4. Construction of Soluble Bovine Fcγ Receptors (FcγRs)

Bovine FcγRI-His, FcγRII-His, FcγRIII-His and Fcγ2R-His expressing plasmids were constructed according to the procedures described below. In order to amplify the signal peptide and the extracellular region of bovine FcγRI, FcγRII, FcγRIII and Fcγ2R (GenBank accession numbers NM_174538, NM_174539, NM_001077402 and NM_001001138), primers were designed which had NotI and *Xho*I recognition sites added on the 5' side (boFcγRI-His F and R; boFcγRIII-His F and R; or boFcγ2R-His F and R) or *Nhe*I and *Eco*RV recognition sites added on the 5' side (boFcyRIII-His F and R). A gene sequence encoding a 6xHis tag was added to reverse primers. PCR was performed using a synthesized bovine PBMC-derived cDNA as a template. The respective PCR products were digested with *Not*I (Takara) and *Xho*I (Takara) (FcγRI-His, FcγRIII-His and Fcγ2R-His) or *Nhe*I (Takara) and *Eco*RV (Takara) (FcγRII-His), purified with FastGene Gel/PCR Extraction Kit (NIPPON Genetics) and cloned into pCXN2.1(+) vector (Niwa H, Yamamura K, Miyazaki J. Gene, 108(2):193-199; Dec. 15, 1991; kindly provided by Dr. T. Yokomizo, Juntendo University Graduate School of Medicine). The resultant expression plasmids were purified with FastGene Xpress Plasmid PLUS Kit (NIPPON Genetics) and stored at -30°C until use in experiments. Hereinafter, the thus prepared expression plasmid is designated as pCXN2.1-boFcγRI-His, pCXN2.1-boFcγRII-His, pCXN2.1-boFcγRIII-His or pCXN2.1-boFcγ2R-His.
Primer (boFcyRI-His F): ATAAGAATGCGGCCGCCACCATGTGGCTCATAATAGCTCT (SEQ ID NO: 79)
Primer (boFcγRI-His R): GCCCTCGAGTTAATGGTGATGGTGATGGTGAGGAGTTGTTGACTGGAGGC (SEQ ID NO: 80)
Primer (boFcγRII-His F): ATAAGAATGCTAGCCACCATGGGGATCCCCTCATTCCT (SEQ ID NO: 81)
Primer (boFcγRII-His R): GCCGATATCTTAATGGTGATGGTGATGGTGCGATGAGGGGCCGCTCGAGC (SEQ ID NO: 82)
Primer (boFcγRIII-His F): ATAAGAATGCGGCCGCCACCATGTGGCAACTGCTACCACC (SEQ ID NO: 83)
Primer (boFcγRIII-His R): GCCCTCGAGTTAATGGTGATGGTGATGGTGGTGCCAAGGTAGAAAGAATG (SEQ ID NO: 84)
Primer (boFcγ2R-His F): ATAAGAATGCGGCCGCCACCATGGCCCCCACCCTCCCTGCCTTGCTCT (SEQ ID NO: 85)
Primer (boFcγ2R-His R): GCCCTCGAGTTAATGGTGATGGTGATGGTGATTCTGCATCGTGTAGTCTG (SEQ ID NO: 86)

Soluble bovine FcγRI-His, FcγRII-His, FcγRIII-His and Fcγ2R-His expressing cells were prepared according to the procedures described below. Briefly, 30 µg of pCXN2.1-boFcγRI-His, pCXN2.1-boFcγRII-His, pCXN2.1-boFcγRIII-His or pCXN2.1-boFcγ2R-His was introduced into 7.5x10⁷ Expi293F cells (Life Technologies) using Expifectamine (Life Technologies) and the transfected cells were then cultured under shaking for 5 to 7 days, followed by collection of a culture supernatant. Recombinant proteins were purified from the culture supernatant using TALON Metal Affinity Resin (Clontech). After purification, the buffer was exchanged with PBS (pH 7.4) using Amicon Ultra-15 Centrifugal Filter Unit (10 kDa, Millipore), and the recombinant proteins were stored at -30°C until use in experiments (bovine PD-1-His). The concentrations of purified bovine FcγRI-His, FcγRII-His, FcγRIII-His and Fcγ2R-His were quantitatively determined in terms of the absorbance (280 nm) measured with Nanodrop8000 Spectrophotometer (Thermo Fisher Scientific).

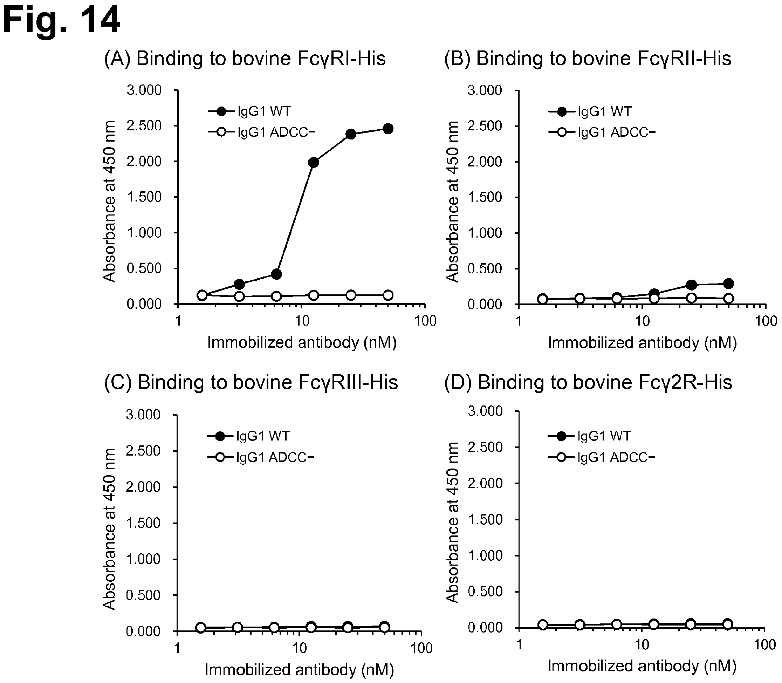

### 2.5. Binding to Bovine FcyRs of Rat-Bovine Chimeric Anti-Bovine PD-1 Antibody ch5D2 IgG1 WT and IgG1 ADCC- (Fig. 14)

Rat-bovine chimeric anti-bovine PD-1 antibody ch5D2 IgG1 WT or IgG1 ADCC- was immobilized on Nunc MaxiSorp ELISA plates (Nunc) at a final concentration of 50, 25, 12.5, 6.25, 3.12 or 1.5610 nM at 37°C for 2 hr. Subsequently, each well was washed with 200 µl of 0.05% Tween 20-supplemented PBS (PBS-T) five times, followed by blocking with SuperBlock (PBS) Blocking Buffer (Thermo Fisher Scientific) at 37°C for 30 min. Each well was washed again in the same manner. Then, bovine FcγRI-His, FcγRII-His, FcγRIII-His or Fcγ2R-His was added to each well at a final concentration of 10 µg/ml and reacted at 37°C for 1 hr. After washing, anti-polyhistidine tag mouse monoclonal antibody (Abcam) was reacted at 37°C for 30 min. Subsequently, each well was washed, and horseradish peroxidase-labeled anti-mouse IgG goat polyclonal antibody (MP Biomedicals) was reacted at 37°C for 30 min. Each well was washed again, and then TMB One Component Substrate (Bethyl) was added for coloring. Thereafter, the enzyme reaction was terminated with 0.18 M dilute sulfuric acid, and absorbance (450 nm) was measured with Microplate Reader MTP-900 (Corona Electric). For every plate washing operation, Auto Plate Washer BIO WASHER 50 (DS Pharma Biomedical) was used.

The experimental results are shown in Fig. 14. IgG1 WT strongly bound to bovine FcγRI-His and weakly bound to bovine FcγRII-His. On the other hand, IgG1 ADCC- did not bind to bovine FcγRI-His or FcγRII-His. Neither IgG1 WT nor IgG1 ADCC- bound to bovine FcγRIII-His or Fcγ2R-His.

All publications, patents and patent applications cited herein are incorporated herein by reference in their entirety.

### INDUSTRIAL APPLICABILITY

The anti-PD-1 antibody of the present invention is applicable to prevention and/or treatment of cancers and infections of animals.

### SEQUENCE LISTING FREE TEXT

<SEQ ID NO: 1>
   SEQ ID NO: 1 shows the amino acid sequence of the light chain variable region (VL) of a rat anti-bovine PD-1 antibody. Underlined parts: CDR1, CDR2 and CDR3 in this order from the NH2 terminus. 
MKVPGRLLVLLFWIPASRSDVVLTQTPVSLSVTLGDQASISCRSSQSLEYSDGYTYLE
WYLQKPGQSPQLLIYGVSNRFSGVPDRFIGSGSGTDFTLKISRVEPEDLGVYYCFQAT
HDPDTFGAGTKLELK
<SEQ ID NO: 2>
   SEQ ID NO: 2 shows the amino acid sequence of the heavy chain variable region (VH) of a rat anti-bovine PD-1 antibody. Underlined parts: CDR1, CDR2 and CDR3 in this order from the NH2 terminus. 
MAILVLLLCLVTIPHSVLSQVQLKETGPGLVQPTQTLSITCTVSGFSLTSYYIQWVRQT
PGKGLEWMGFIRSGGSTEYNSEFKSRLSINRDTSKNQVFLKMNSLKTEDTGVYYCAR
TSSGYEGGFDYWGQGVMVTVSS
<SEQ ID NO: 3>
   SEQ ID NO: 3 shows the amino acid sequence of the light chain constant region (CL) of a bovine antibody (bovine Ig lambda, GenBank: X62917). 
QPKSPPSVTLFPPSTEELNGNKATLVCLISDFYPGSVTVVWKADGSTITRNVETTRASK
QSNSKYAASSYLSLTSSDWKSKGSYSCEVTHEGSTVTKTVKPSECS
<SEQ ID NO: 4>
   SEQ ID NO: 4 shows the amino acid sequence of the heavy chain constant region (CH) of a bovine antibody (bovine IgG1, modified from GenBank: X62916). Mutated parts are underlined. Amino acid numbers and mutations: 123 E→P, 124 L→V, 125 P→A, 126 G→ deletion, 218 A→S, 219 P→S 
ASTTAPKVYPLSSCCGDKSSSTVTLGCLVSSYMPEPVTVTWNSGALKSGVHTFPAVL
QSSGLYSLSSMVTVPGSTSGQTFTCNVAHPASSTKVDKAVDPTCKPSPCDCCPPPPVA
GPSVFIFPPKPKDTLTISGTPEVTCVVVDVGHDDPEVKFSWFVDDVEVNTATTKPREE
QFNSTYRVVSALRIQHQDWTGGKEFKCKVHNEGLPSSIVRTISRTKGPAREPQVYVLA
PPQEELSKSTVSLTCMVTSFYPDYIAVEWQRNGQPESEDKYGTTPPQLDADSSYFLYS
KLRVDRNSWQEGDTYTCVVMHEALHNHYTQKSTSKSAGK
<SEQ ID NO: 5>
   SEQ ID NO: 5 shows the nucleotide sequence of the VL of a rat anti-bovine PD-1 antibody. 
ATGAAAGTGCCTGGTAGGCTGCTGGTGCTGTTGTTTGGATTCCAGCTTCCAGGAG
TGATGTTGTGTTGACACAAACTCCAGTTTCCCTGTCTGTCACACTTGGAGATCAAG
CTTCTATATCTTGCAGGTCTAGTCAGAGCCTGGAATATAGTGATGGATACACTTATT
TGGAATGGTACCTACAGAAGCCAGGCCAGTCTCCACAGCTCCTCATCTATGGAGTT
TCCAACCGATTTTCTGGGGTCCCAGACAGGTTCATTGGCAGTGGGTCAGGGACAG
ATTTCACCCTCAAGATCAGCAGAGTAGAGCCTGAGGACTTGGGAGTTTATTACTGC
TTCCAAGCTACACATGATCCGGACACGTTTGGAGCTGGGACCAAGCTGGAACTGA
AA
The nucleotide sequence of SEQ ID NO: 5 after codon optimization is shown in <SEQ ID NO: 11>. 
ATGAAGGTCCCTGGTAGGCTGCTGGTTCTCTTGTTCTGGATCCCTGCTTCCAGAAG
TGACGTGGTGCTGACTCAAACACCAGTGAGTCTCAGTGTGACCCTCGGCGACCA
GGCCTCCATTTCTTGCCGTAGCAGCCAGTCCTTGGAATACTCTGATGGTTATACTTA
TCTGGAGTGGTACCTCCAGAAGCCCGGGCAGTCACCCCAGCTTCTTATCTACGGT
GTGAGCAACAGATTTTCTGGGGTTCCTGATCGGTTTATTGGATCTGGATCCGGTAC
CGACTTCACATTGAAAATTTCACGCGTCGAACCCGAGGATCTGGGGGTCTACTATT
GCTTCCAAGCCACCCACGATCCCGACACCTTCGGCGCTGGCACTAAGCTGGAGCT
GAAA
<SEQ ID NO: 6>
   SEQ ID NO: 6 shows the nucleotide sequence of the VH of a rat anti-bovine PD-1 antibody. 
ATGGCTATCCTGGTGCTGCTTCTCTGCCTGGTGACCATTCCACACTCTGTCTTGTCC
CAGGTGCAGCTGAAGGAGACAGGACCTGGCCTGGTGCAACCAACACAGACCCTG
TCCATCACATGTACTGTTTCTGGGTTCTCATTAACCAGCTATTATATACAGTGGGTTC
GCCAGACTCCAGGAAAGGGACTAGAATGGATGGGATTTATACGGAGTGGTGGAAG
CACAGAGTATAATTCAGAGTTCAAATCCCGACTTAGCATCAACAGGGACACCTCC
AAGAACCAAGTTTTCTTAAAAATGAACAGTCTGAAAACAGAGGACACAGGCGTG
TACTACTGTGCCAGAACCTCTTCGGGGTACGAAGGGGGTTTTGATTACTGGGGCC
AAGGAGTCATGGTCACAGTCTCCTCA
The nucleotide sequence of SEQ ID NO: 6 after codon optimization is shown in <SEQ ID NO: 12>. ATGGCAATCCTCGTGTTGCTTCTGTGCTTGGTGACCATTCCACACTCTGTGCTTTCC 
CAGGTGCAGCTCAAGGAAACAGGGCCAGGACTCGTCCAACCTACACAAACCCTG
TCAATCACCTGTACCGTATCCGGTTTAGCCTCACCAGCTATTATATACAATGGGTG
AGGCAGACCCCCGGGAAAGGACTGGAATGGATGGGCTTCATTCGCAGCGGTGGG
AGTACCGAGTACAATAGCGAGTTTAAAAGTCGCTTGAGTATCAATAGAGATACTTC
CAAGAATCAGGTGTTCTTGAAGATGAACTCCCTCAAGACCGAAGATACAGGGGTC
TATTACTGCGCCAGGACCTCCAGTGGATATGAAGGAGGCTTTGATTATTGGGGGCA
GGGCGTCATGGTAACTGTGAGCTCA
<SEQ ID NO: 7>
   SEQ ID NO: 7 shows the nucleotide sequence of the CL of a bovine antibody (bovine Ig lambda, GenBank: X62917). 
CAGCCCAAGTCCCCACCCTCGGTCACCCTGTTCCCGCCCTCCACGGAGGAGCTCA
ACGGCAACAAGGCCACCCTGGTGTGTCTCATCAGCGACTTCTACCCGGGTAGCGT
GACCGTGGTCTGGAAGGCAGACGGCAGCACCATCACCCGCAACGTGGAGACCAC
CCGGGCCTCCAAACAGAGCAACAGCAAGTACGCGGCCAGCAGCTACCTGAGCCT
GACGAGCAGCGACTGGAAATCGAAAGGCAGTTACAGCTGCGAGGTCACGCACGA
GGGGAGCACCGTGACGAAGACAGTGAAGCCCTCAGAGTGTTCTTAG
The nucleotide sequence of SEQ ID NO: 7 after codon optimization is shown in <SEQ ID NO: 13>. 
CAACCCAAGTCACCACCATCCGTGACTCTGTTTCCTCCATCTACAGAGGAGCTGA
ACGGAAACAAAGCTACCTTGGTGTGTCTCATCTCTGACTTTTACCCCGGATCTGTC
ACTGTGGTATGGAAAGCAGATGGCAGCACAATAACCAGGAATGTTGAAACCACAC
GAGCCTCCAAGCAGTCCAATAGTAAGTATGCCGCATCTTCATATCTGTCCCTTACAA
GCTCAGACTGGAAATCCAAAGGCAGCTACAGTTGCGAGGTCACACATGAAGGCA
GCACCGTGACAAAGACCGTAAAGCCATCTGAGTGTAGCTAG
<SEQ ID NO: 8>
   SEQ ID NO: 8 shows the nucleotide sequence (after codon optimization) of the CH of a bovine antibody (bovine IgG1, modified from GenBank: X62916). 
GCTAGCACCACAGCACCTAAAGTTTACCCTCTGTCTTCCTGCTGCGGCGACAAGT
CTTCATCAACTGTTACTCTTGGATGCCTGGTCTCAAGTTACATGCCCGAGCCCGTG
ACAGTGACCTGGAACTCAGGCGCTCTGAAGTCTGGAGTGCACACATTTCCAGCTG
TGCTTCAGTCTAGCGGCCTGTATTCCCTCAGCTCTATGGTTACTGTACCTGGTAGCA
 
CCAGCGGACAGACTTTCACCTGTAATGTTGCCCATCCCGCATCTTCTACCAAGGTC
GATAAAGCCGTTGACCCCACTTGCAAACCATCCCCTTGTGATTGTTGTCCACCCCC
TCCAGTGGCTGGCCCTTCCGTCTTCATTTTCCCTCCTAAACCTAAGGATACTCTGAC
CATCTCAGGGACACCCGAGGTCACCTGTGTCGTCGTGGACGTGGGACATGACGAC
CCAGAAGTCAAGTTCTCATGGTTCGTGGACGATGTGGAGGTGAACACAGCAACA
ACAAAGCCCAGAGAAGAACAGTTTAACAGCACATATCGGGTGGTCAGCGCCTTGC
GTATTCAGCACCAGGACTGGACTGGTGGCAAGGAGTTTAAGTGCAAGGTGCATAA
CGAAGGTCTGCCCTCTTCTATAGTGAGAACTATCTCCCGAACTAAGGGCCCCGCTC
GGGAGCCCCAGGTTTACGTCCTTGCTCCCCCTCAGGAGGAACTGAGTAAATCAAC
CGTGAGTCTCACCTGTATGGTTACCTCATTTACCCAGACTACATCGCCGTAGAGT
GGCAGAGGAATGGACAGCCAGAGTCTGAGGACAAATACGGCACTACTCCTCCCC
AACTGGATGCCGACTCTTCCTACTTCCTCTACTCCAAATTGCGAGTTGACCGGAAC
TCATGGCAGGAGGGGGACACATACACATGCGTCGTTATGCACGAGGCCCTGCACA
ACCATTACACCCAGAAGTCCACATCTAAAAGTGCAGGTAAGTAA
<SEQ ID NO: 9>
   SEQ ID NO: 9 shows the amino acid sequence of a chimeric light chain consisting of the VL of a rat anti-bovine PD-1 antibody and the CL of a bovine antibody. 
MKVPGRLLVLLFWIPASRSDVVLTQTPVSLSVTLGDQASISCRSSQSLEYSDGYTYLE
WYLQKPGQSPQLLIYGVSNRFSGVPDRFIGSGSGTDFTLKISRVEPEDLGVYYCFQAT
HDPDTFGAGTKLELKQPKSPPSVTLFPPSTEELNGNKATLVCLISDFYPGSVTVVWKA
DGSTITRNVETTRASKQSNSKYAASSYLSLTSSDWKSKGSYSCEVTHEGSTVTKTVKP
SECS
<SEQ ID NO: 10>
   SEQ ID NO: 10 shows the amino acid sequence of a chimeric heavy chain consisting of the VH of a rat anti-bovine PD-1 antibody and the CH of a bovine antibody (bovine IgG1, modified from GenBank: X62916). 
MAILVLLLCLVTIPHSVLSQVQLKETGPGLVQPTQTLSITCTVSGFSLTSYYIQWVRQT
PGKGLEWMGFIRSGGSTEYNSEFKSRLSINRDTSKNQVFLKMNSLKTEDTGVYYCAR
TSSGYEGGFDYWGQGVMVTVSSASTTAPKVYPLSSCCGDKSSSTVTLGCLVSSYMPE
PVTVTWNSGALKSGVHTFPAVLQSSGLYSLSSMVTVPGSTSGQTFTCNVAHPASSTKV
DKAVDPTCKPSPCDCCPPPPVAGPSVFIFPPKPKDTLTISGTPEVTCVVVDVGHDDPEV
KFSWFVDDVEVNTATTKPREEQFNSTYRVVSALRIQHQDWTGGKEFKCKVHNEGLP
 
SSIVRTISRTKGPAREPQVYVLAPPQEELSKSTVSLTCMVTSFYPDYIAVEWQRNGQPE
SEDKYGTTPPQLDADSSYFLYSKLRVDRNSWQEGDTYTCVVMHEALHNHYTQKSTS
KSAGK
<SEQ ID NO: 14>
   SEQ ID NO: 14 shows the nucleotide sequence (after codon optimization) of a chimeric light chain consisting of the VL of a rat anti-bovine PD-1 antibody and the CL of a bovine antibody. 
ATGAAGGTCCCTGGTAGGCTGCTGGTTCTCTTGTTCTGGATCCCTGCTTCCAGAAG
TGACGTGGTGCTGACTCAAACACCAGTGAGTCTCAGTGTGACCCTCGGCGACCA
GGCCTCCATTTCTTGCCGTAGCAGCCAGTCCTTGGAATACTCTGATGGTTATACTTA
TCTGGAGTGGTACCTCCAGAAGCCCGGGCAGTCACCCCAGCTTCTTATCTACGGT
GTGAGCAACAGATTTTCTGGGGTTCCTGATCGGTTTATTGGATCTGGATCCGGTAC
CGACTTCACATTGAAAATTTCACGCGTCGAACCCGAGGATCTGGGGGTCTACTATT
GCTTCCAAGCCACCCACGATCCCGACACCTTCGGCGCTGGCACTAAGCTGGAGCT
GAAACAACCCAAGTCACCACCATCCGTGACTCTGTTTCCTCCATCTACAGAGGAG
CTGAACGGAAACAAAGCTACCTTGGTGTGTCTCATCTCTGACTTTTACCCCGGATC
TGTCACTGTGGTATGGAAAGCAGATGGCAGCACAATAACCAGGAATGTTGAAACC
ACACGAGCCTCCAAGCAGTCCAATAGTAAGTATGCCGCATCTTCATATCTGTCCCTT
ACAAGCTCAGACTGGAAATCCAAAGGCAGCTACAGTTGCGAGGTCACACATGAA
GGCAGCACCGTGACAAAGACCGTAAAGCCATCTGAGTGTAGCTAG
<SEQ ID NO: 15>
   SEQ ID NO: 15 shows the nucleotide sequence (after codon optimization) of a chimeric heavy chain consisting of the VH of a rat anti-bovine PD-1 antibody and the CH of a bovine antibody (bovine IgG1, modified from GenBank: X62916). 
ATGGCAATCCTCGTGTTGCTTCTGTGCTTGGTGACCATTCCACACTCTGTGCTTTCC
CAGGTGCAGCTCAAGGAAACAGGGCCAGGACTCGTCCAACCTACACAAACCCTG
TCAATCACCTGTACCGTATCCGGTTTTAGCCTCACCAGCTATTATATACAATGGGTG
AGGCAGACCCCCGGGAAAGGACTGGAATGGATGGGCTTCATTCGCAGCGGTGGG
AGTACCGAGTACAATAGCGAGTTTAAAAGTCGCTTGAGTATCAATAGAGATACTTC
CAAGAATCAGGTGTTCTTGAAGATGAACTCCCTCAAGACCGAAGATACAGGGGTC
TATTACTGCGCCAGGACCTCCAGTGGATATGAAGGAGGCTTTGATTATTGGGGGCA
GGGCGTCATGGTAACTGTGAGCTCAGCTAGCACCACAGCACCTAAAGTTTACCCT
 
CTGTCTTCCTGCTGCGGCGACAAGTCTTCATCAACTGTTACTCTTGGATGCCTGGT
CTCAAGTTACATGCCCGAGCCCGTGACAGTGACCTGGAACTCAGGCGCTCTGAAG
TCTGGAGTGCACACATTTCCAGCTGTGCTTCAGTCTAGCGGCCTGTATTCCCTCAG
CTCTATGGTTACTGTACCTGGTAGCACCAGCGGACAGACTTTCACCTGTAATGTTG
CCCATCCCGCATCTTCTACCAAGGTCGATAAAGCCGTTGACCCCACTTGCAAACCA
TCCCCTTGTGATTGTTGTCCACCCCCTCCAGTGGCTGGCCCTTCCGTCTTCATTTTC
CCTCCTAAACCTAAGGATACTCTGACCATCTCAGGGACACCCGAGGTCACCTGTGT
CGTCGTGGACGTGGGACATGACGACCCAGAAGTCAAGTTCTCATGGTTCGTGGAC
GATGTGGAGGTGAACACAGCAACAACAAAGCCCAGAGAAGAACAGTTTAACAGC
ACATATCGGGTGGTCAGCGCCTTGCGTATTCAGCACCAGGACTGGACTGGTGGCA
AGGAGTTTAAGTGCAAGGTGCATAACGAAGGTCTGCCCTCTTCTATAGTGAGAACT
ATCTCCCGAACTAAGGGCCCCGCTCGGGAGCCCCAGGTTTACGTCCTTGCTCCCC
CTCAGGAGGAACTGAGTAAATCAACCGTGAGTCTCACCTGTATGGTTACCTCATTT
TACCCAGACTACATCGCCGTAGAGTGGCAGAGGAATGGACAGCCAGAGTCTGAG
GACAAATACGGCACTACTCCTCCCCAACTGGATGCCGACTCTTCCTACTTCCTCTA
CTCCAAATTGCGAGTTGACCGGAACTCATGGCAGGAGGGGGACACATACACATGC
GTCGTTATGCACGAGGCCCTGCACAACCATTACACCCAGAAGTCCACATCTAAAA
GTGCAGGTAAGTAA
<SEQ ID NO: 16>
   SEQ ID NO: 16 shows the amino acid sequence (QSLEYSDGYTY) of CDR1 of the VL of rat anti-bovine PD-1 antibody 5D2.
<SEQ ID NO: 17>
   SEQ ID NO: 17 shows the amino acid sequence (FQATHDPDT) of CDR3 of the VL of rat anti-bovine PD-1 antibody 5D2.
<SEQ ID NO: 18>
   SEQ ID NO: 18 shows the amino acid sequence (GFSLTSYY) of CDR1 of the VH of rat anti-bovine PD-1 antibody 5D2.
<SEQ ID NO: 19>
   SEQ ID NO: 19 shows the amino acid sequence (IRSGGST) of CDR2 of the VH of rat anti-bovine PD-1 antibody 5D2.
<SEQ ID NO: 20>
   SEQ ID NO: 20 shows the amino acid sequence (ARTSSGYEGGFDY) of CDR3 of the VH of rat anti-bovine PD-1 antibody 5D2.
<SEQ ID NO: 21>
   SEQ ID NO: 21 shows the amino acid sequence of the CH (CH1-CH3) of a bovine antibody (IgG1 variant 1).
<SEQ ID NO: 22>
   SEQ ID NO: 22 shows the amino acid sequence of the CH (CH1-CH3) of a bovine antibody (IgG1 variant 2).
<SEQ ID NO: 23>
   SEQ ID NO: 23 shows the amino acid sequence of the CH (CH1-CH3) of a bovine antibody (IgG1 variant 3).
<SEQ ID NO: 24>
   SEQ ID NO: 24 shows the amino acid sequence of the CH (CH1-CH3) of a bovine antibody (IgG2 variant 1).
<SEQ ID NO: 25>
   SEQ ID NO: 25 shows the amino acid sequence of the CH (CH1-CH3) of a bovine antibody (IgG2 variant 2).
<SEQ ID NO: 26>
   SEQ ID NO: 26 shows the amino acid sequence of the CH (CH1-CH3) of a bovine antibody (IgG2 variant 3).
<SEQ ID NO: 27>
   SEQ ID NO: 27 shows the amino acid sequence of the CH (CH1-CH3) of a bovine antibody (IgG3 variant 1).
<SEQ ID NO: 28>
   SEQ ID NO: 28 shows the amino acid sequence of the CH (CH1-CH3) of a bovine antibody (IgG3 variant 2).
<SEQ ID NO: 29>
   SEQ ID NO: 29 shows the nucleotide sequence of the CH (CH1-CH3) of a bovine antibody (IgG1 variant 1).
<SEQ ID NO: 30>
   SEQ ID NO: 30 shows the nucleotide sequence of the CH (CH1-CH3) of a bovine antibody (IgG1 variant 2).
<SEQ ID NO: 31>
   SEQ ID NO: 31 shows the nucleotide sequence of the CH (CH1-CH3) of a bovine antibody (IgG1 variant 3).
<SEQ ID NO: 32>
   SEQ ID NO: 32 shows the nucleotide sequence of the CH (CH1-CH3) of a bovine antibody (IgG2 variant 1).
<SEQ ID NO: 33>
   SEQ ID NO: 33 shows the nucleotide sequence of the CH (CH1-CH3) of a bovine antibody (IgG2 variant 2).
<SEQ ID NO: 34>
   SEQ ID NO: 34 shows the nucleotide sequence of the CH (CH1-CH3) of a bovine antibody (IgG2 variant 3).
<SEQ ID NO: 35>
   SEQ ID NO: 35 shows the nucleotide sequence of the CH (CH1-CH3) of a bovine antibody (IgG3 variant 1).
<SEQ ID NO: 36>
   SEQ ID NO: 36 shows the nucleotide sequence of the CH (CH1-CH3) of a bovine antibody (IgG3 variant 2).
<SEQ ID NO: 37>
   SEQ ID NO: 37 shows the amino acid sequence of the CH (CH1-CH3) of an ovine antibody (IgG1).
<SEQ ID NO: 38>
   SEQ ID NO: 38 shows the nucleotide sequence of the CH (CH1-CH3) of an ovine antibody (IgG1).
<SEQ ID NO: 39>
   SEQ ID NO: 39 shows the amino acid sequence of the CH (CH1-CH3) of an ovine antibody (IgG2).
<SEQ ID NO: 40>
   SEQ ID NO: 40 shows the nucleotide sequence of the CH (CH1-CH3) of an ovine antibody (IgG2).
<SEQ ID NO: 41>
   SEQ ID NO: 41 shows the amino acid sequence of the light chain (Ig kappa (CK)) constant region of an ovine antibody.
<SEQ ID NO: 42>
   SEQ ID NO: 42 shows the nucleotide sequence of the light chain (Ig kappa (CK)) constant region of an ovine antibody.
<SEQ ID NO: 43>
   SEQ ID NO: 43 shows the amino acid sequence of the light chain (Ig lambda (CL)) constant region of an ovine antibody.
<SEQ ID NO: 44>
   SEQ ID NO: 44 shows the nucleotide sequence of the light chain (Ig lambda (CL)) constant region of an ovine antibody.
<SEQ ID NO: 45>
   SEQ ID NO: 45 shows the amino acid sequence of the CH (CH1-CH3) of a water buffalo antibody (presumed to be IgG1).
<SEQ ID NO: 46>
   SEQ ID NO: 46 shows the nucleotide sequence of the CH (CH1-CH3) of a water buffalo antibody (presumed to be IgG1).
<SEQ ID NO: 47>
   SEQ ID NO: 47 shows the amino acid sequence of the CH (CH1-CH3) of a water buffalo antibody (presumed to be IgG2).
<SEQ ID NO: 48>
   SEQ ID NO: 48 shows the nucleotide sequence of the CH (CH1-CH3) of a water buffalo antibody (presumed to be IgG2).
<SEQ ID NO: 49>
   SEQ ID NO: 49 shows the amino acid sequence of the CH (CH1-CH3) of a water buffalo antibody (presumed to be IgG3).
<SEQ ID NO: 50>
   SEQ ID NO: 50 shows the nucleotide sequence of the CH (CH1-CH3) of a water buffalo antibody (presumed to be IgG3).
<SEQ ID NO: 51>
   SEQ ID NO: 51 shows the amino acid sequence of the constant region of the light chain (presumed to be Ig lambda) of a water buffalo antibody.
<SEQ ID NO: 52>
   SEQ ID NO: 52 shows the nucleotide sequence of the constant region of the light chain (presumed to be Ig lambda) of a water buffalo antibody.
<SEQ ID NO: 53>
   SEQ ID NO: 53 shows the amino acid sequence of the CH (CH1-CH3) of a human antibody (IgG4 variant 1).
<SEQ ID NO: 54>
   SEQ ID NO: 54 shows the nucleotide sequence of the CH (CH1-CH3) of a human antibody (IgG4 variant 1).
<SEQ ID NO: 55>
   SEQ ID NO: 55 shows the amino acid sequence of the CH (CH1-CH3) of a human antibody (IgG4 variant 2).
<SEQ ID NO: 56>
   SEQ ID NO: 56 shows the nucleotide sequence of the CH (CH1-CH3) of a human antibody (IgG4 variant 2).
<SEQ ID NO: 57>
   SEQ ID NO: 57 shows the amino acid sequence of the CH (CH1-CH3) of a human antibody (IgG4 variant 3).
<SEQ ID NO: 58>
   SEQ ID NO: 58 shows the nucleotide sequence of the CH (CH1-CH3) of a human antibody (IgG4 variant 3).
<SEQ ID NO: 59>
   SEQ ID NO: 59 shows the amino acid sequence of the CL of a human antibody.
<SEQ ID NO: 60>
   SEQ ID NO: 60 shows the nucleotide sequence of the CL of a human antibody.
<SEQ ID NOS: 61-74>
   SEQ ID NOS: 61-74 show the nucleotide sequences of primers boPD-1-myc F, boPD-1-myc R, boPD-L1-EGFP F, boPD-L1-EGFP R, boPD-1-His F, boPD-1-His R, ovPD-1 CDS F, ovPD-1 CDS R, buPD-1 CDS F1, buPD-1 CDS R1, buPD-1 CDS F2, buPD-1 CDS R2, ovPD-1-EGFP F and ovPD-1-EGFP R in this order.
<SEQ ID NO: 75>
   SEQ ID NO: 75 shows the nucleotide sequence of a chimeric heavy chain consisting of the VH of a rat anti-bovine PD-1 antibody and the CH of a bovine antibody (bovine IgG1, GenBank: X62916). 
ATGGCAATCCTCGTGTTGCTTCTGTGCTTGGTGACCATTCCACACTCTGTGCTTTCC
CAGGTGCAGCTCAAGGAAACAGGGCCAGGACTCGTCCAACCTACACAAACCCTG
TCAATCACCTGTACCGTATCCGGTTTTAGCCTCACCAGCTATTATATACAATGGGTG
AGGCAGACCCCCGGGAAAGGACTGGAATGGATGGGCTTCATTCGCAGCGGTGGG
AGTACCGAGTACAATAGCGAGTTTAAAAGTCGCTTGAGTATCAATAGAGATACTTC
CAAGAATCAGGTGTTCTTGAAGATGAACTCCCTCAAGACCGAAGATACAGGGGTC
TATTACTGCGCCAGGACCTCCAGTGGATATGAAGGAGGCTTTGATTATTGGGGGCA
GGGCGTCATGGTAACTGTGAGCTCAGCCTCCACCACAGCCCCGAAAGTCTACCCT
CTGAGTTCTTGCTGCGGGGACAAGTCCAGCTCCACCGTGACCCTGGGCTGCCTGG
TCTCCAGCTACATGCCCGAGCCGGTGACCGTGACCTGGAACTCGGGTGCCCTGAA
GAGCGGCGTGCACACCTTCCCGGCTGTCCTTCAGTCCTCCGGGCTGTACTCTCTCA
GCAGCATGGTGACCGTGCCCGGCAGCACCTCAGGACAGACCTTCACCTGCAACGT
AGCCCACCCGGCCAGCAGCACCAAGGTGGACAAGGCTGTTGATCCCACATGCAA
ACCATCACCCTGTGACTGTTGCCCACCCCCTGAGCTCCCCGGAGGACCCTCTGTC
TTCATCTTCCCACCGAAACCCAAGGACACCCTCACAATCTCGGGAACGCCCGAGG
TCACGTGTGTGGTGGTGGACGTGGGCCACGATGACCCCGAGGTGAAGTTCTCCTG
GTTCGTGGACGACGTGGAGGTAAACACAGCCACGACGAAGCCGAGAGAGGAGC
AGTTCAACAGCACCTACCGCGTGGTCAGCGCCCTGCGCATCCAGCACCAGGACTG
GACTGGAGGAAAGGAGTTCAAGTGCAAGGTCCACAACGAAGGCCTCCCGGCCCC
CATCGTGAGGACCATCTCCAGGACCAAAGGGCCGGCCCGGGAGCCGCAGGTGTAT
GTCCTGGCCCCACCCCAGGAAGAGCTCAGCAAAAGCACGGTCAGCCTCACCTGC
ATGGTCACCAGCTTCTACCCAGACTACATCGCCGTGGAGTGGCAGAGAAACGGGC
AGCCTGAGTCGGAGGACAAGTACGGCACGACCCCGCCCCAGCTGGACGCCGACA
GCTCCTACTTCCTGTACAGCAAGCTCAGGGTGGACAGGAACAGCTGGCAGGAAG
GAGACACCTACACGTGTGTGGTGATGCACGAGGCCCTGCACAATCACTACACGCA
GAAGTCCACCTCTAAGTCTGCGGGTAAATAA
<SEQ ID NO: 76>
   SEQ ID NO: 76 shows the amino acid sequence of a chimeric heavy chain consisting of the VH of a rat anti-bovine PD-1 antibody and the CH of a bovine antibody (bovine IgG1, GenBank: X62916). 
MAILVLLLCLVTIPHSVLSQVQLKETGPGLVQPTQTLSITCTVSGFSLTSYYIQWVRQT
PGKGLEWMGFIRSGGSTEYNSEFKSRLSINRDTSKNQVFLKMNSLKTEDTGVYYCAR
TSSGYEGGFDYWGQGVMVTVSSASTTAPKVYPLSSCCGDKSSSTVTLGCLVSSYMPE
PVTVTWNSGALKSGVHTFPAVLQSSGLYSLSSMVTVPGSTSGQTFTCNVAHPASSTKV
DKAVDPTCKPSPCDCCPPPELPGGPSVFIFPPKPKDTLTISGTPEVTCVVVDVGHDDPE
VKFSWFVDDVEVNTATTKPREEQFNSTYRVVSALRIQHQDWTGGKEFKCKVHNEGL
PAPIVRTISRTKGPAREPQVYVLAPPQEELSKSTVSLTCMVTSFYPDYIAVEWQRNGQP
ESEDKYGTTPPQLDADSSYFLYSKLRVDRNSWQEGDTYTCVVMHEALHNHYTQKST
SKSAGK
<SEQ ID NOS: 77-86>
   SEQ ID NOS: 77-86 show the nucleotide sequences of primers boIgG1 CH1 F, boIgG1 CH3 R, boFcγRI-His F, boFcyRI-His R, boFcγRII-His F, boFcγRII-His R, boFcγRIII-His F, boFcγRIII-His R, boFcγ2R-His F and boFcγ2R-His R in this order.

## Claims

1. An anti-PD-1 antibody comprising (a) a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat; and (b) a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.

2. The antibody of claim 1, wherein the light chain variable region and the heavy chain variable region are derived from rat.

3. The antibody of claim 2, wherein the light chain variable region is the light chain variable region of a rat anti-bovine PD-1 antibody and the heavy chain variable region is the heavy chain variable region of a rat anti-bovine PD-1 antibody.

4. The antibody of claim 3, wherein the light chain variable region has the amino acid sequence as shown in SEQ ID NO. 1 and the heavy chain variable region has the amino acid sequence as shown in SEQ ID NO: 2.

5. The antibody of any one of claims 1 to 4, wherein the light chain constant region of an antibody of an animal other than rat has the amino acid sequence of the constant region of lambda chain or kappa chain.

6. The antibody of any one of claims 1 to 5, wherein the heavy chain constant region of an antibody of an animal other than rat has the amino acid sequence of the constant region of an immunoglobulin equivalent to human IgG4, or has mutations introduced thereinto that reduce ADCC activity and/or CDC activity.

7. The antibody of claim 6, wherein the animal other than rat is bovine; the light chain constant region of the bovine antibody has the amino acid sequence of the constant region of lambda chain; and the heavy chain constant region of the bovine antibody has mutations introduced thereinto that reduce ADCC activity and/or CDC activity.

8. The antibody of claim 7, wherein the light chain constant region of the bovine antibody has the amino acid sequence as shown in SEQ ID NO: 3 and the heavy chain constant region of the bovine antibody has the amino acid sequence as shown in SEQ ID NO: 4

9. The antibody of any one of claims 1 to 8 which has a four-chain structure comprising two light chains and two heavy chains.

10. A pharmaceutical composition comprising the antibody of any one of claims 1 to 9 as an active ingredient.

11. The composition of claim 10 for prevention and/or treatment of cancers and/or inflammations.

12. The composition of claim 11, wherein the cancers and/or inflammations are selected from the group consisting of neoplastic diseases, leukemia, Johne's disease, anaplasmosis, bacterial mastitis, mycotic mastitis, mycoplasma infections (such as mycoplasma mastitis, mycoplasma pneumonia or the like), tuberculosis, *Theileria orientalis* infection, cryptosporidiosis, coccidiosis, trypanosomiasis and leishmaniasis.

13. An artificial genetic DNA comprising (a') a DNA encoding a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat and (b') a DNA encoding a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.

14. A vector comprising the artificial genetic DNA of claim 13.

15. A host cell transformed with the vector of claim 14.

16. A method of preparing an antibody, comprising culturing the host cell of claim 15 and collecting an anti-PD-1 antibody from the resultant culture.

17. A DNA encoding a light chain comprising a light chain variable region containing CDR1 having the amino acid sequence of QSLEYSDGYTY (SEQ ID NO: 16), CDR2 having the amino acid sequence of GVS and CDR3 having the amino acid sequence of FQATHDPDT (SEQ ID NO: 17) and the light chain constant region of an antibody of an animal other than rat.

18. A DNA encoding a heavy chain comprising a heavy chain variable region containing CDR1 having the amino acid sequence of GFSLTSYY (SEQ ID NO: 18), CDR2 having the amino acid sequence of IRSGGST (SEQ ID NO: 19) and CDR3 having the amino acid sequence of ARTSSGYEGGFDY (SEQ ID NO: 20) and the heavy chain constant region of an antibody of an animal other than rat.
